(19) Europäisches Patentamt
European Patent Office
Office européen des brevets

(11) **EP 2 332 651 A2**

(12) **EUROPEAN PATENT APPLICATION**

(43) Date of publication:
**15.06.2011 Bulletin 2011/24**

(51) Int Cl.:
*B01L 3/00* (2006.01)   *C12M 1/34* (2006.01)
*G02B 21/34* (2006.01)   *G01N 15/14* (2006.01)

(21) Application number: **10183774.8**

(22) Date of filing: **25.10.2001**

(84) Designated Contracting States:
**AT BE CH CY DE DK ES FI FR GB GR IE IT LI LU MC NL PT SE TR**

(62) Document number(s) of the earlier application(s) in accordance with Art. 76 EPC:
**01982673.4 / 1 438 385**

(27) Previously filed application:
**25.10.2001 PCT/IL01/00992**

(71) Applicant: **BAR ILAN UNIVERSITY**
**52900 Ramat Gan (IL)**

(72) Inventor: **Deutsch, Mordechai**
**42855, Doar-Na Lev HaSharon (IL)**

(74) Representative: **Fichter, Robert Arno et al**
**Dennemeyer & Associates S.A.**
**Patent Department**
**55, rue des Bruyères**
**1274 Howald (LU)**

Remarks:
This application was filed on 30-09-2010 as a divisional application to the application mentioned under INID code 62.

(54) **Interactive transparent individual cells biochip processor**

(57)     The present invention concerns a device for holding a plurality of living cells, comprising:
a transparent element defining a multiplicity of optically transparent wells, wherein the size of said wells is configured to hold a single cell, any defined number of cells, or other defined particles; and characterized by a plurality of said wells including an integral biosensor component.

Coin With Built-In Spacers

Fig 43a

EP 2 332 651 A2

**Description**

## FIELD OF THE INVENTION

The present invention relates to a new Interactive Transparent Individual Cells Biochip Processor (ITICBP) device which suggests a new generation of cytometer, referred to as Lab on a Cell Chip device, applicable in determination of activity of an identified same, or different, single cell. More specifically, the new ITICBP device allows on-line measurement of a vast spectrum of physiological activities of an visually observable individual cell, or a group of cells, using a wide-range of methods such as, morphometry, fluorescence, chromometry, reflectance, electrochemical, and other chemical- and optical-based procedures. These new capabilities of the new individual cell processor, may expand for the first time, the use of morphometric, fluorometric, chromometric and biochemical (metabolic) parameters in measuring the same individual cell in population, and/or measuring groups of identifiable cells.

The ITICBP device of present invention opens new horizons in the area of cell biology. In the rapidly expanding field of analytical cytology in AIDS, cancer biology, immunology and prenatal diagnosis, the ITICBP device supposes to provide an answer to the need for quantitative measuring, manipulating and modulating controlled biological processes within a single living cell.

## BACKGROUND OF THE INVENTION

Combinatorial (bio)chemistry has evolved as an essential practical means permitting synthesis of many biologically-active and pharmaceutical structures, which must then be tested for their effects on animals and humans. The use of single, individual cell- based assays is an important tool in modern and advanced biomedical studies. Furthermore, cell functions are comprised of many interconnecting signaling and feedback pathways. Many times, a compound study based on isolated targets or cell preparations can not resolve this complexity. Thus, for a comprehensive understanding of a compound effect, testing of a single, whole living cell, is required. Such tests, in addition to their assistance in discovering and developing safer products, they provide a useful tool in detecting biological and toxic effects, suggesting an alternative method for present toxicological tests resulted in reducing the number of animals used for testing.

The advantages of using intact, individual living cells for compound screening includes:

- Efficacy of function of tested compounds can be best estimated by observing and measuring their biological effect on, or within, a single intact cell.
- Cell intracellular molecular interactions can be evaluated within the context of "working environment" inside the cell.
- Toxicity and nonspecific effects can be identified on a level of an individual cell.
- Drug effects on selective cell types can be distinguished.
- Drug penetration can be evaluated in studies applying single whole cell.

Orphan targets require cell based functional assays.

- Whole cell assays obviate protein purification & expression steps.

Many cell-based assay procedures have been developed over the years and are in use today, most often for lead optimization and predictive toxicology to qualify "hits" from primary screening. Examples include viral titer assays, second messenger assays like luciferase, and advanced fluorescent signal assays.

Still, these assays are limited in the minimal sample size they may measure and none of them allows individual cell-based assay procedure.

Static and dynamic properties of living cells are presently measured using two main methods: (a) bulk measurement in cuvettes (macroscopic well arrays), producing a signal characteristic of the population as a whole, and is therefore preferably used for the study of very homogeneous populations; (b) in flow cytometry, measurements are being performed on a moving single cells which are lost following their measurement. Therefore, it is impossible to perform a series of sequential static and dynamic measurements on the same individual cell. Many who have developed and used the apparatus and techniques of flow cytometry have come to the realization that some of the most critical questions in various areas of cell and developmental biology, immunology, oncology, and pharmacology cannot be answered using even the most sophisticated flow cytometers. The reason for that is the existing fact that these instruments measure single events only once during a few microseconds

In order to understand the cellular reaction induced by antigens, carcinogens, drugs, growth factors, and hormones, it is necessary to monitor the processes for minutes, hours, and even days. This requires a means of non-intrusive repetitive measurement on the same cell or group of cells. Thus, there is a need of capability to maintain cell viability and to define and retain the location of each individual cell in order to correlate its measured properties with subsequent behaviour in culture and/or with additional physical or biochemical analyses.

Thirty years of intensive work, prompted by the necessity to position each individual living cell in an exact location in order to overcome the shortcomings of existing cytometric methods, has borne but little fruit (Freed and Engle, 1962; Mansberg and Ohringer, 1969; Tomei et al., 1988; Shack et al., 1979; Hart et al., 1990; Burger and Gershman, 1988; Green, 1979; Kamentsky and Kamentsky, 1991, Read et al., 1979; Tanaka et al., 1979; Boddington et al., 1967; Dawson, et al., 1967; Shapiro, 1983; de Grooth et al., 1985.

The only system that quite successfully addressed the need for repeatable individual cell measurements is the Cellscan apparatus (Deutsch and Weinreb, 1994). The heart of the Cellscan static cytometer is the cell carrier which is made of conducting materials (copper, nickel, etc.) using a standard electroplating technique of the type commercially employed in microelectronic fabrication and for making transmission electron microscope grids. As is known in the art, that process, in its last stages, involves the deposition of metal by electrolysis on a conducting plane, usually made of copper, which has array of spots on top of it, made of a photo-resistant substance (dielectric). The deposited metal built up on the spot-free zones of the conducting plane symmetrically overlaps the photo-resistant spots. Spilling off the novo deposit metallic layer, about 10 $\mu$m depth, and desolving the regaining photo-resistant spots revile holes there-through the metallic layer. The cross-section of the holes is conical-like having circular upper (of about 7$\mu$m diameter) and lower (of about 3.5$\mu$m diameter) openings.

The Sellscan cell carrier provides capabilities for separating biological cells from one another by placing each separated cell within a precisely dimensioned hole at a known address, to which one can return, for repeated cell observation and/or repeated stimulation followed by subsequent analysis.

There are several US patents relating to the Cellscan device and its application in measuring the activity of an individual cell. More specifically, the patents deal with a method and system for individually analyzing a living cell placed at a defined location, on a cell by cell basis.

US Patent 4,729,949 provides a capability for separating biological cells from one another by placing each separated cell within a precisely dimensioned hole (referred to as an aperture) at a known address, to which one can return, for repeated determinations of cell activity and/or repeated stimulation followed by subsequent analysis. More specifically, the patent deal with a method and a system for individually analyzing a living cell placed at a defined location, on a cell by cell basis. The tests and the effects on each cell are performed automatically in order to reduce the testing time and to permit the task to be performed by relatively unskilled personnel.

US Patent 5,272,081 demonstrates a method for producing cells having at least one common optical property, electromagnetic property or biological property (cf. US Patent 4,729,949). The selected living cells are separated from all other cells on the carrier, or by removing undesired living cells from the carrier, or by killing undesired living cells on the carrier. The desired selected cells are growing either on the carrier or after having been removed therefrom.

US Patents 5,310,674 and 5,506,141 both refer to an apertured cell carrier which has the capability of containing individual living cells (one cell only per aperture or hole) at identifiable locations. These cell carriers enable the method of US Patent 4,729,949 to be carried out. In other words, they are utilized for trapping individual cells at known locations, thereby enabling at least one sub-population of cells to be selected from a more general cell population, using defined parameters common to the sub-population, and also enabling the simultaneous study of large groups of living cells (e.g., 10,000 or more living cells), on a cell-by-cell basis.

In general, these patents disclose a method and apparatus for placing individual cells at identifiable addresses within the holes of a carrier, and for performing, on a cell-by-cell basis, one or more of the following operations:

a) observing or measuring a property of a living cell;
b) moving a living cell (by moving the cell carrier and not as an individual or as a sub-group);
c) killing a living cell;

Generally speaking, in accordance with the above defined series of patents, a large number of cells, e.g., lymphocytes in the blood (representing a group or a defined population of cells), are first separated from all other cells, i.e., from different groups of populations of cells. After the separation process, the separated lymphocytes are subjected simultaneously to selected tests and thereafter each cell is separately investigated to determine whether or not, as a result of the test, or stimulation, it exhibits a particular property. The address of every cell exhibiting said property is recorded. Thus, after all the separated cells have been investigated the addresses of all the cells which exhibited the particular property are known. These cells represent a particular subgroup of lymphocytes within the larger entire group of lymphocytes.

Each cell in the subgroup is individually investigated by directing the investigative instrumentation to the cell's unique known location or address (other cells, not being part of the subgroup, are ignored). Consequently, once a subgroup has been identified only its cells are investigated, thereby limiting investigation time only to the subgroup cells which are of interest.

In the first step the lymphocytes are separated from the rest of the cells contained in the blood. The separation is performed by means of a perforated cell carrier (includes a base in which are formed apertures or holes having larger openings at the tops than at the bottom thereof). The shape of the apertures enable the cells to be effectively held to the carrier by applying means, such as a pressure difference between the upper and the bottom side of the carrier, or electromagnetic forces. The carrier is chosen to have holes of well defined sizes so that when the sample (e.g., blood) containing the various cell groups is placed on the carrier, effectively most, if not all, of the holes are occupied by cells of the group of interest, one cell per hole. In this way, the desired population of cells (e.g., 7 $\mu$m lymphocytes) can easily enter the aperture without suffering substantial damage and yet, once in the aperture, the cells are held therein and cannot pass out of the bottom of the carrier. The size of the aperture should be related to the size of the desired cells, so that when a desired cell enters an aperture, practically the entire cell is captured and retained within the aperture, thus preventing it from being washed out during a washing step.

Nevertheless, there are several major and crucial shortcomings of the above mentioned cell carrier:

(a) Living cells are tightly trapped in the cell carrier (CC) holes and cannot move in it, due to its characteristic conical cross section. This prevents any mechanical manipulation of the trapped cell, including their extraction for any further operations.

(b) The space starting beneath the highest tangential circumference, where the spherical, but flexible living cell, touches the internal sloped (curved) wall of a hole-trap, is prevented from any biochemical manipulations, since neither efflux nor influx of solutions to or from this space is possible. Thus, only about the surface of the upper half of the cell globe remains available for dye and/or biochemical delivery. For example, compared to processes occurring in cell suspension, it strongly affects (decreases) the cell staining rate as well as production rates of intracellular products out of penetrating substrates. Similarly, from the same reason measurements of product release and leakage rates are distorted.

(c) Since the CC is opaque and the diameter of the lower opening (second cross section) of each trap must be smaller than that of the trapped cell, than the image of a trapped cell can never be visually observed. This is a huge deficiency in many fields of research and application since there is no way to correlate between the measured data (i.e. fluorescence intensity (FI), fluorescence polarization or anisotropy (FP), fluorescence lifetime (FLT), fluorescence polarization decay (FPD) and electrode-based reactions) and visually observed data_(i.e. morphology, shape, intracellular compartments etc.).

(d) A hole-trap on the CC cannot be ever visually observed. As a consequence, when by chance more than one single cell is trapped in the same hole-trap, the measured data obtained from that trap cannot be distinguished and associated with any of the trapped cells specifically.

(e) Due to strong reflections, even when a blackened CC is used, scattered light measurements of a single trapped cell in the CC are unrealisable.

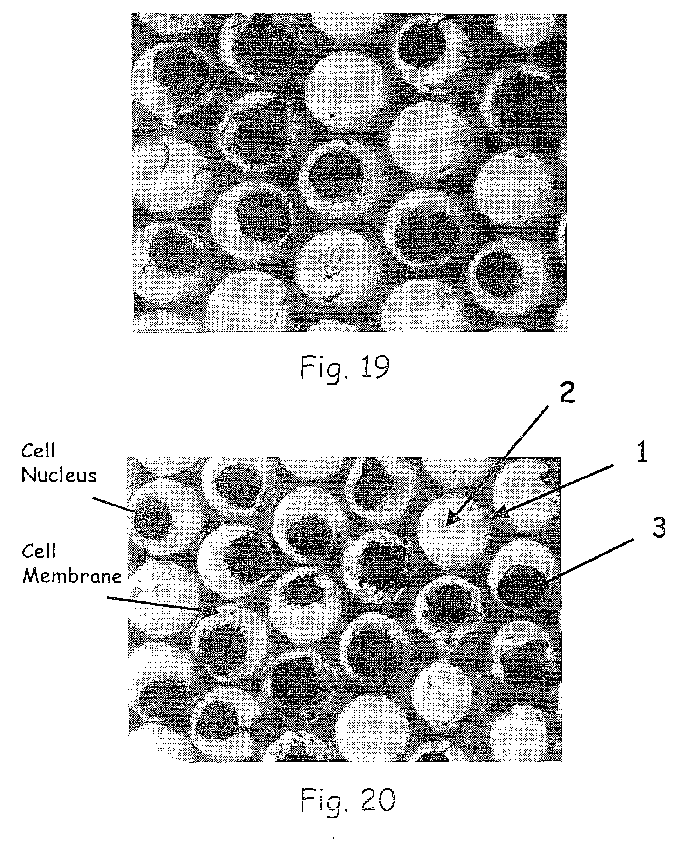

(f) One undesired outcome of the electroplating technique is the flat spaces (or very moderately curved) created among holes as can be seen in Figs.19-22 of US patent 5,506,141. Usually their overall area is more than 4 times greater than that of the overall area of the upper opening of the holes. Hence, a high proportion of the loaded cells settle down between holes rather than inside them, a fact which decreases loading efficiency, causes waste of cells, which might be curtail when the sample size is limited.

(g) Trapped cell toxicity induced by the metallic CC is inevitable due to the release of metallic ions in physiological environment. This prevents or sabotages long-term measurements, incubation and/or growing of the trapped cells. Coating of the CC surface with pure silicon or silicon derivatives does not abolish this toxic effect since metal ions diffuse from the CC bottom (second face) up through the lower openings of the CC holes to the trapped cells.

(h) The level of smoothness of a hole-trap wall, as well as of the intermediate spaces, is quite limited (see Figs. 20-22, 36 and 32 in US patent 5,506,141) due to intrinsic limitations of manufacturing processes. A rule of thumb of in-vivo cell growing and proliferation says that the smoother the cell supporting surface is, the higher the chance to culture cells on top of it.

(i) Electromagnetic manipulation at the proximity (vicinity) of the CC metallic surface is quite limited and insufficient due to mirror effects.

(j) Fabrication of microelectrodes in the metallic traps, is impossible.

(k) US patent 5,506,141 discussed several uses of electric field in manipulated living cells (column 14, line 52 - column 16 line 21). The operation efficiency of these kinds of uses strongly suffers from electrical screening effects caused by the free ions of the buffering physiological suspending media. Moreover, an additional undesired effect is electrolysis, which alters the suspending media characteristics and might cause cell death as mentioned in US patent 5,506,141(column 14, lines 64-65): " The use of an electric field as the driving force can lead to electrolysis problems". Actually, this was the reason why that idea never came through with this CC.

Taking into consideration the above listed shortcomings, it is a major object of present invention to provide a device for observing, measuring, studying, examining, assessing, monitoring and controlling a wide scope of functions in a single, identifiable, individual viable cell either manually or automatically, using either a one-step procedure, a multi-step procedure, a series of same procedure or a sequence of different procedures.

It is a further object of present invention to provide a cytometer having a transparent cell chip (TCC) that enable the viewing of an individual cell and consequently, morphologically examinable. It is a further object of present invention to provide a cytometer having TCC with fabricated wells containing one or more transparent microelectrodes functioning, *inter alia,* as (bio)chemical sensors. It is yet an additional object of present invention to provide a cytometer having a TCC comprising hexagonal wells with no, or minimal, space in-between them.

It is yet a rather important object of present invention to provide a screening platform utilising, by large, any measurable optospectroscopic, electrochemical and electromagnetic features, static as well as time dependent, for the determination of the effects of a wide range of biological-active materials on cells, while being individually examined, as well as on individual cell lysate.

## SUMMARY OF THE INVENTION

The invention provides an Interactive Transparent Individual Cells Biochip Processor (ITICBP) Device, for assessing a single, individual living cell at identifiable location or assessing a group of cells each at identifiable location, comprising: (a) a transparent cell chip (TCC) containing optically transparent wells each has a bottom and it fits in size to hold a single cell, or any defined number of cells, or other defined particles; (b) means to direct the cells and force them to enter into the wells, or to place them in the wells directly, or to exit or remove them from the wells; (c) a holder for such a TCC; (d) means to transfer solids, liquids, and cell suspensions to the TCC; (e) means to transfer individual viable, and/or non-viable, cells or group of cells or cell fragments; (f) means to measure and assess cell morphology, cell activity, cell physiology, cell metabolism, cell affinity and viability and changes that may occur as a result of presence or absence of contact with other cells and/or particular biologically-active materials; (g) means to assess, monitor and analyze chromatic images and scanning electron microscope images; (h) a computer embedded system to control functions of the device, record each of the individual overall cell and device status and activities, analyze the data and provide information and results.

In accordance with present invention, a new Transparent Individual Cell Processor (ITICBP) device is provided. This in-vitro, high-throughput device, has the capability of storing/holding individual live cells within an identifiable controlled micron-sized transparent wells highly packed in a hexagonal array. The cells may be examined, either individually and/or in groups. The ITICBP device is characterized, *inter alia,* by the following functions and properties:

1. High quality transparency that makes the individual cell visible while being held and maintained via the well and consequently, morphologically examinable.

2. The individual well is fabricated with one or more transparent microelectrodes, and it is controlled and monitored via integrated compatible electronic circuit. The microelectrodes are useful in handing and maintaining the cells (entrapment, movement, stimulation, fusion) applying delicate and localized Converging and Alternating Voltage (CAV) in detection of changes in electrical characteristics of the cells. In addition, suitable substrate-coated microelectrodes (electrochemical biosensors) are useful in measuring cellular metabolism activity as manifested by production and secretion of various products.

3. The device performs cell selection and separation utilizing optical tweezers, by applying a local electrical voltage in conjunction with overhead rinsing and/or selective cell killing by means of controlled laser beam power.

4. The ITICBP device can accommodate, simultaneously and in a non-disturbing way, large cell populations, on a cell-by-cell basis. For example, it may easily comprise 9 fields of 150x150 micron-sized-wells each with separating channels in between, thus permitting simultaneous exposure of the 9 fields (each field populated with 22,500 individual cells of same or different source and/or type) to at least 9 different biological and/or biochemical and/or flourophoric - active agents.

5. Cells can be grown within the wells of the cell carrier (CCP), which may contain, or coated with, various biologically-active materials (including photo-activated molecules).

6. Biochemical changes in the investigated or examined individual cell, can be either simultaneously, or sequentially, determined (or monitored) by both fluorescence measurement using a specific fluorescent reporter and/or by electrochemical measurement, utilising specific substrate-coated fabricated transparent microelectrodes, providing data relating to either intra- and/or extra-cellular metabolites.

7. The ITICBP device may be either cleaned and sterilised and thus re-used and/or be disposable. It is designed for a wide spectrum of cell's function studies in biological, clinical and toxicological laboratories' environments.

8. The ITICBP device and accessories comprise of two sub-systems:

a) The sub-system comprising the TCC platform and device measuring facilities (hereinafter referred to as "the platform and measuring system") containing optical, electronic and other measuring tools as well as means for control, data-acquisition and analysis.

b) The sub-system comprising the computer-controlled activity carried out in each individual well (hereinafter referred to as ITICBP system)

The ITICBP is mounted on a computer-controlled stage and positioned to place the investigated cell at the point of interrogation (at the center of an excitation laser beam or field of measurement/observation/manipulation). The address of each cell, determined by its location on the ITICBP, is maintained throughout a series of measurements and manipulations to which the cell is subjected. Holding the cells on the ITICBP, allows them to be maintained under the favorable conditions while various stimuli are added or rinsed away.

## LIST OF FIGURES 1 to 58 (of total 72 Figures)

FIG 1 illustrates a partial upper view of the TCC, built up of hexagonal wells.

FIG 2 depicts individual cells within the wells (one cell per well) of the TCC, wherein no accumulation of cells between the wells is possible.

FIG 3 presents a cross section of wells depicted in FIG 2.

FIG 4 presents a scanning electron microscope (SEM) showing the upper view of highly packed hexagonal arrayed wells.

FIG 5 focuses on a single well from the SEM of FIG 4.

FIG 6 provides an isometric view of SEM emphasizing the sharpness of the well's wall.

FIG 7 focuses on a single cell occupied within a single well.

FIG 8 presents a SEM picture showing a cell within a well having a flat bottom.

FIG 9 provides a transparent light image (x40) of T jurkat cell within wells of the TCC. Occupancy percentage is >90%.

FIGs 10 and 11 present transparent light images (x100) emphasizing the ability of visually observing individual live cells within the wells (a single cell per well) of the TCC.

FIGs 12-15 demonstrate a unique feature of the device providing transparent and fluorescent images of same individual cell in a population. FIGs 12(x40) and 14 (x100) demonstrate transparent images, whereas FIGs 13(x40) and 15 (x100) demonstrate fluorescent images.

FIGs 16-18 focus on another unique feature relating to tracing interactions between cells within a single well. FIG 16 demonstrates (using a transparent light image x100) two interacting cells within a well, located at the upper right corner of the picture. FIG 17 demonstrates same couple of cells, using fluorescent image (x100). FIG 18 depicts the same couple of cells after 15 minutes of interaction.

FIGs 19-21 provide chromatic observation of the individual cells within the wells.

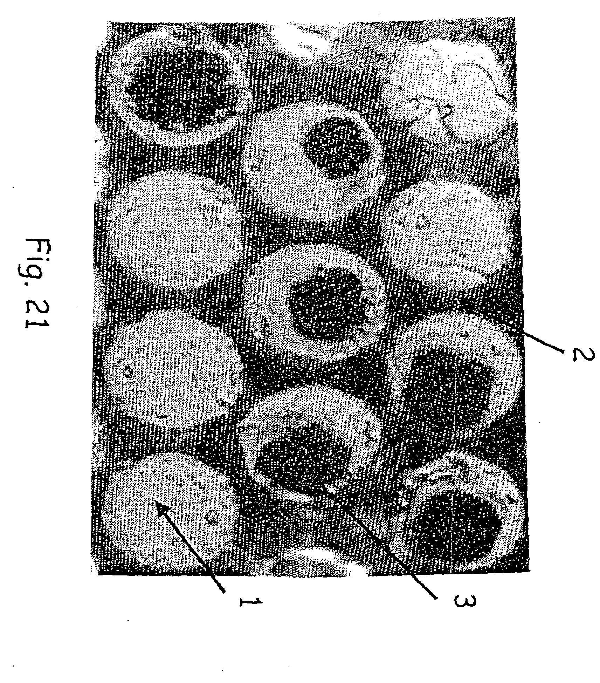

FIG 19 presents the chromatic images of Giemsa treated cells FIG 20 emphasizing the cell nucleus and cell membrane of same cells of FIG 19. FIG 21 demonstrates a high resolution magnified picture of cells treated as described in FIG 19.

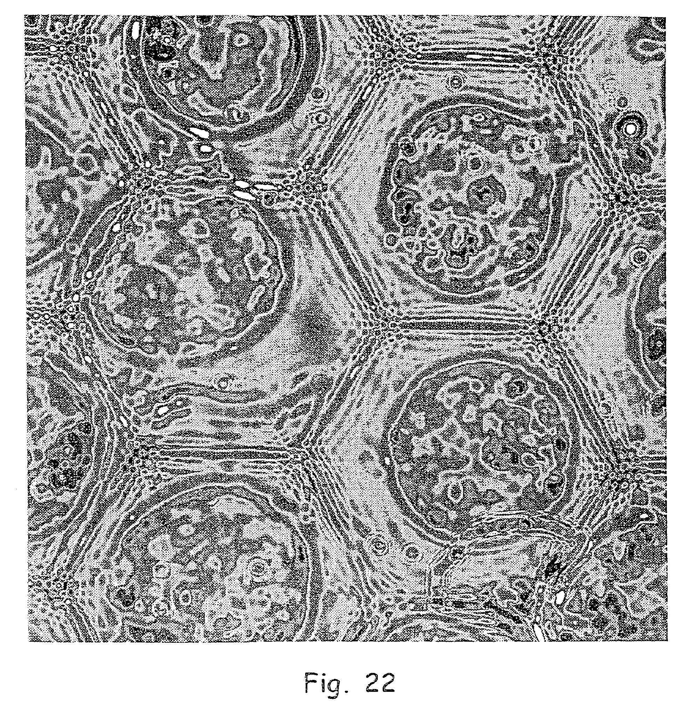

FIG 22 demonstrates the use of image analysis (IA) tools for examination of sub-cellular organelles.

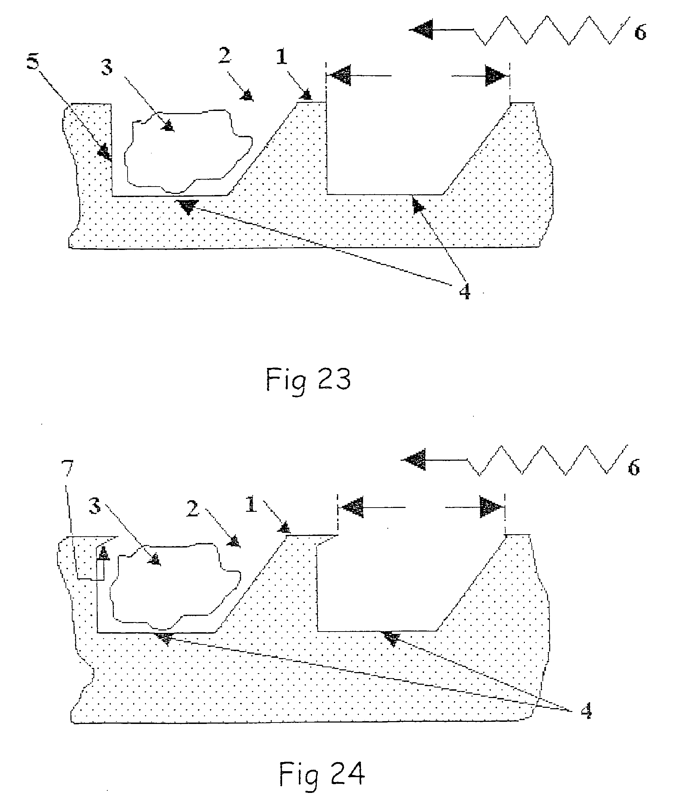

FIGs 23 and 24 depict wells having asymmetric cross section. FIG 24 includes a stopping tooth as an integrated part of the orthogonal wall.

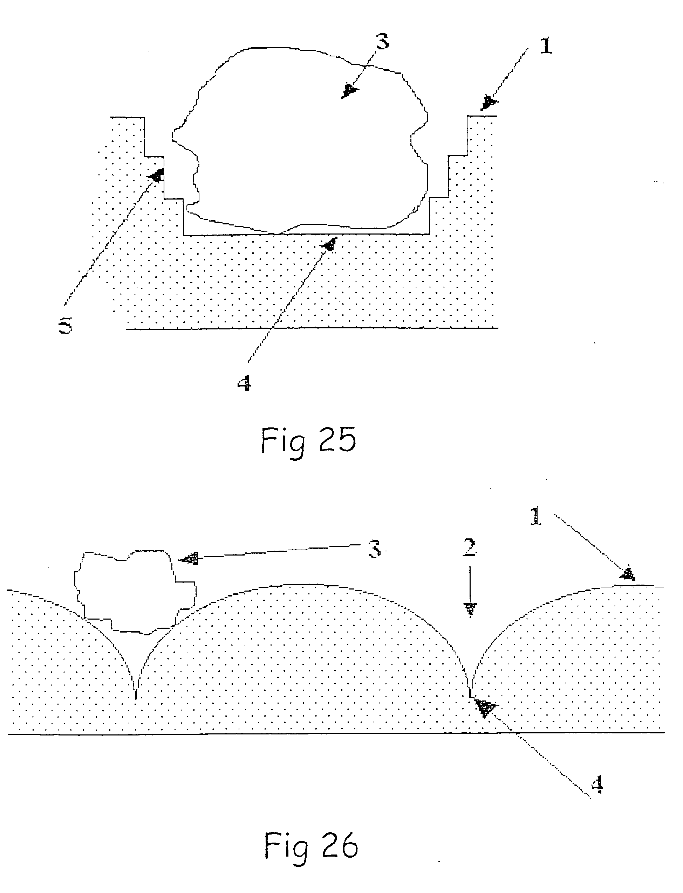

FIG 25 depicts wells having stairs-like walls and symmetrical cross section.

FIG 26 illustrates an array of wavy-repeating rounded hills, wherein cells are localized in valleys formed between the rounded hills.

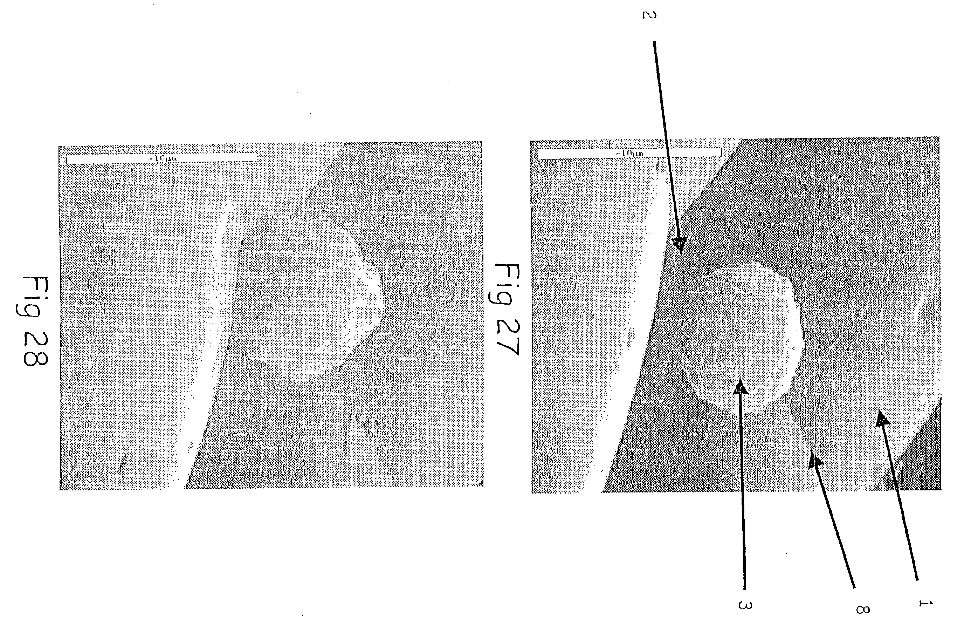

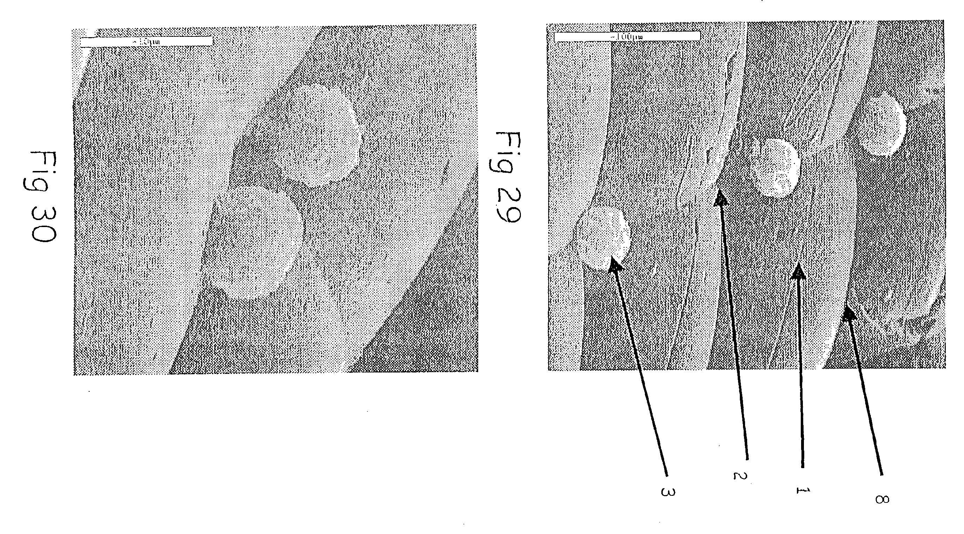

FIGs 27 - 30 show the SEM photographs of the valleys of FIG 26 that are applicable as channels for transportation of both solutions and cells. FIGs 27 and 28 show a lymphocyte before and after localization, respectively. FIG 29 shows three randomly lymphocytes in proximity to their location. FIG 30 relates to the possibility, if so desired, of having more than one cell per well, whenever cell-cell interaction is examined.

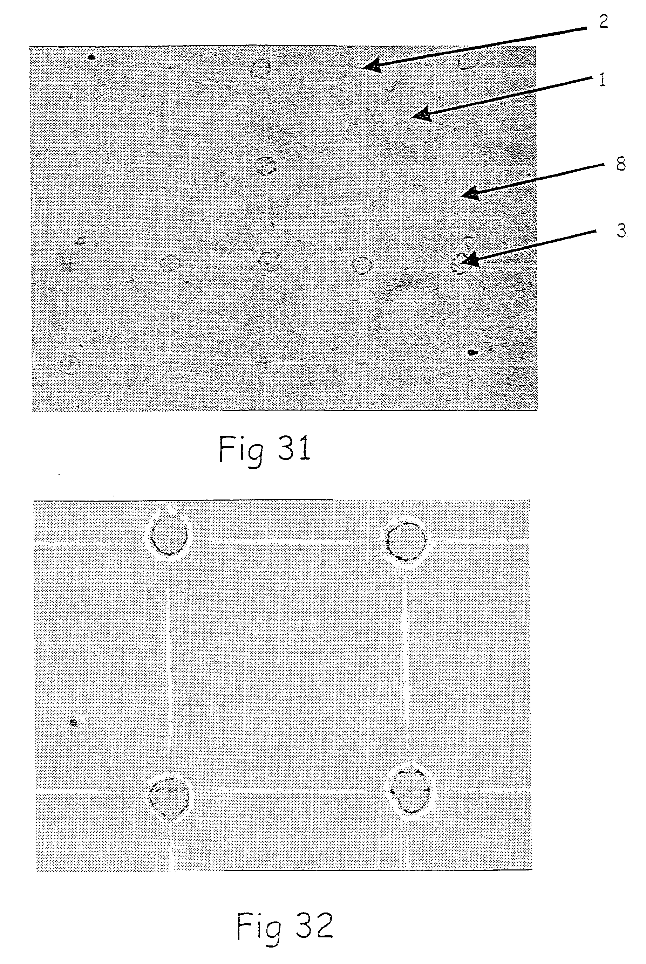

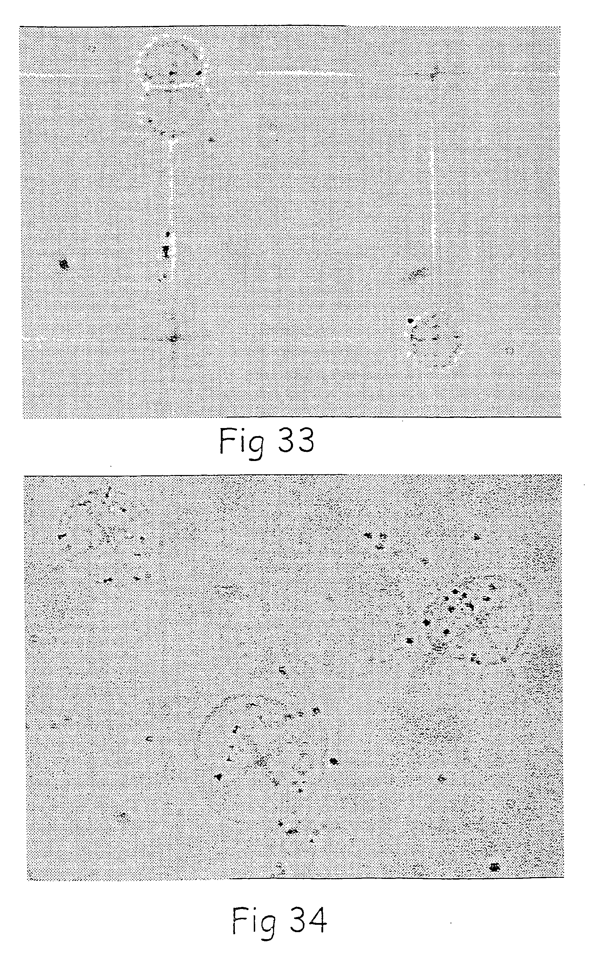

FIGs 31-34 present transparent light images of the "wavy hill array configuration". FIG 31 depicts the upper view of the wavy hill array (x40) emphasizing the circular circumferences of the hills and the lymphocytes that are held or localized in few of the intersection points. Same phenomena is demonstrated in FIG 32 (x100) and in FIG 33, in which a location for cell-cell interaction is observed. FIG 34 shows T jurkat cells greater in size compared to the peripheral blood lymphocytes.

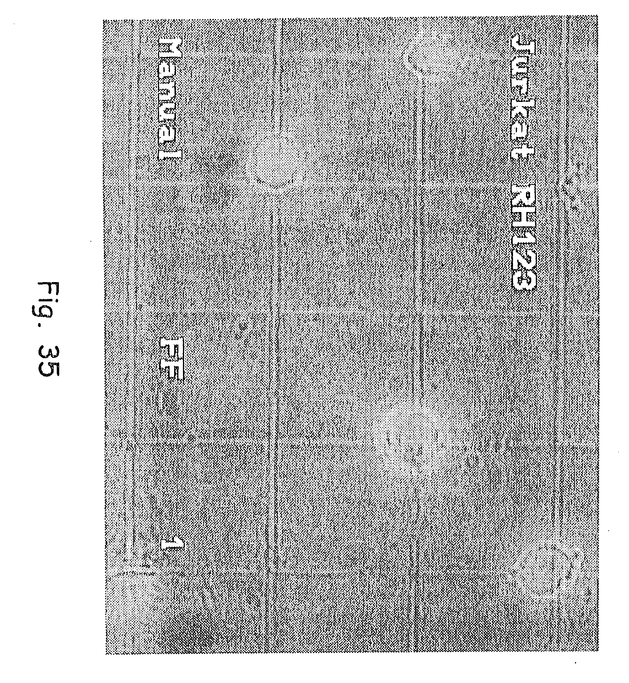

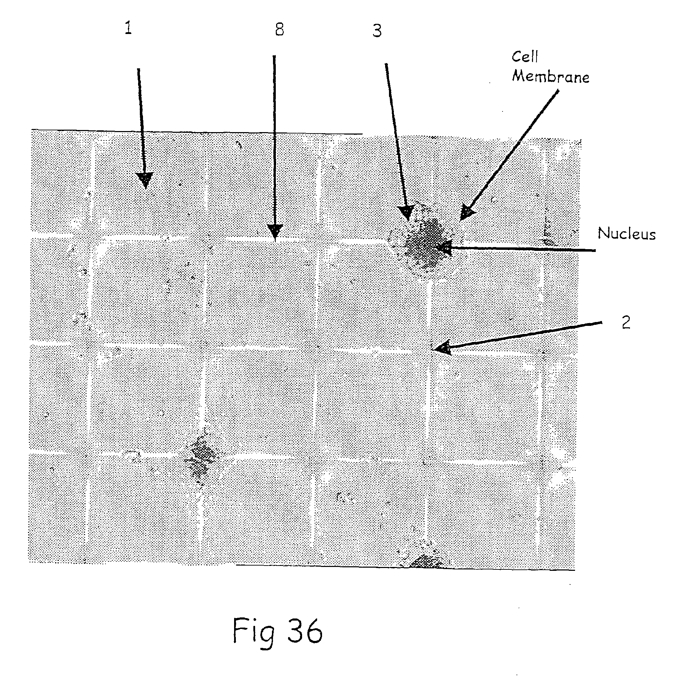

FIGs 35 and 36 demonstrate fluorescence and chromatic images, respectively, of the wavy-hills array configuration. Cell membrane and nucleus are distinguished in the chromatic image (FIG 36).

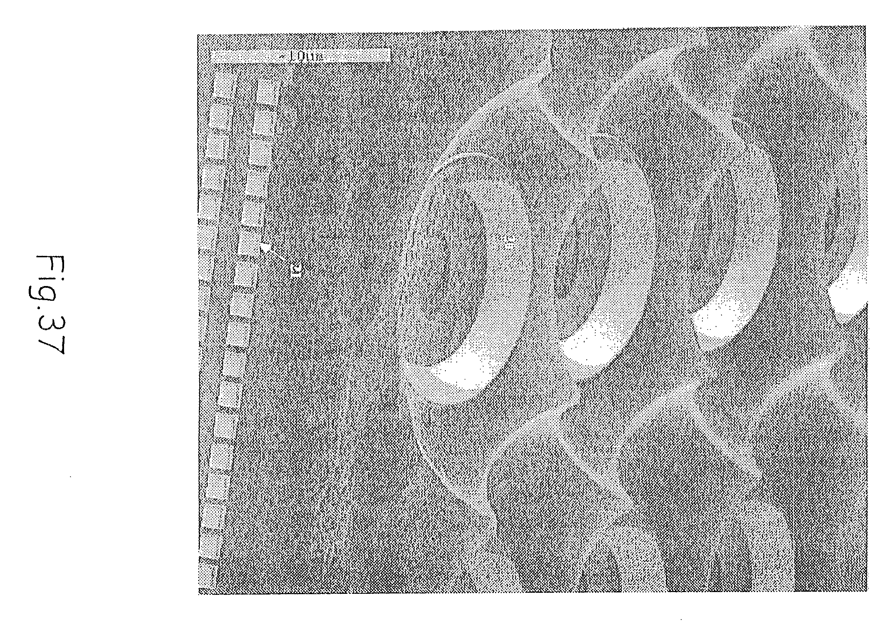

FIG 37 relates to the electro-chemical measurement capabilities of the device.

The figure demonstrates a well containing a circular electrode (20) attached or deposited onto the inner surface of the well.

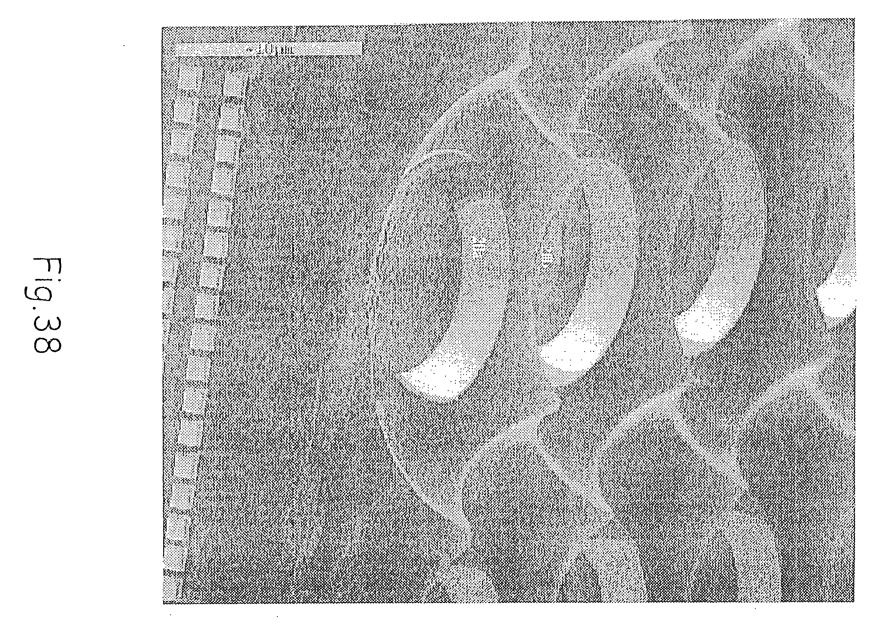

FIG 38 depicts the possibility of having multi-electrodes (20a and 20b) within a single well.

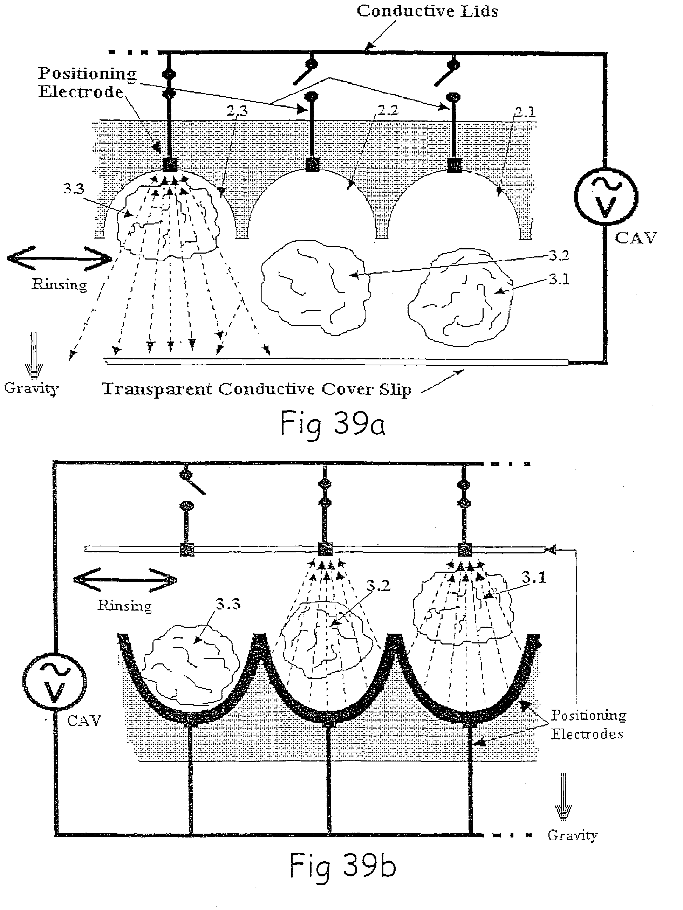

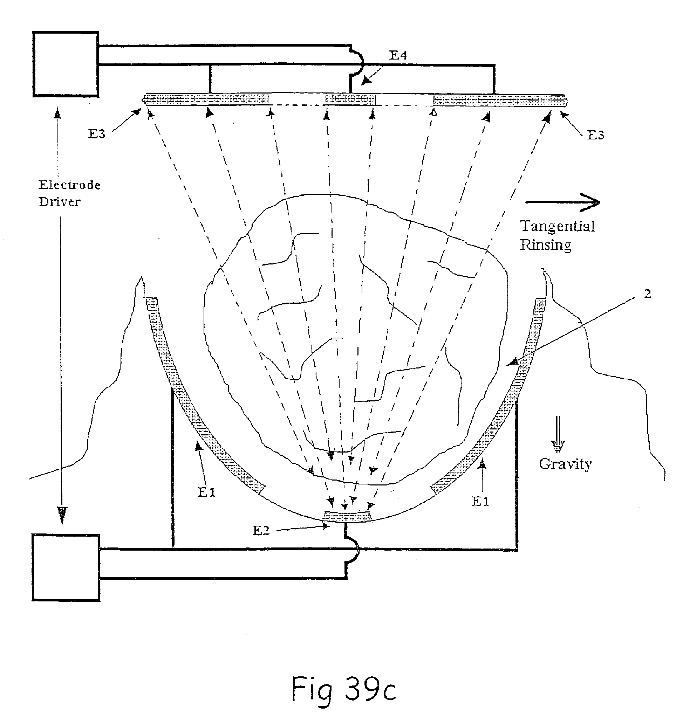

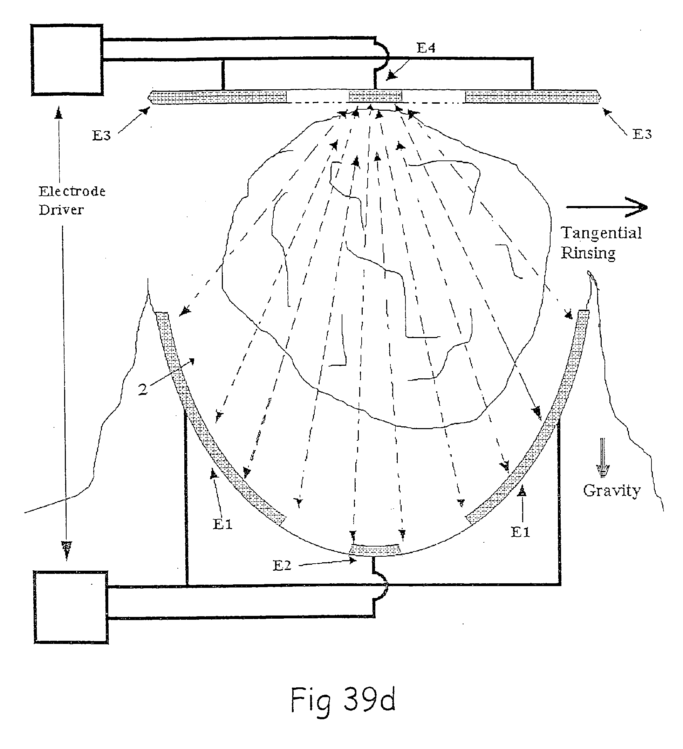

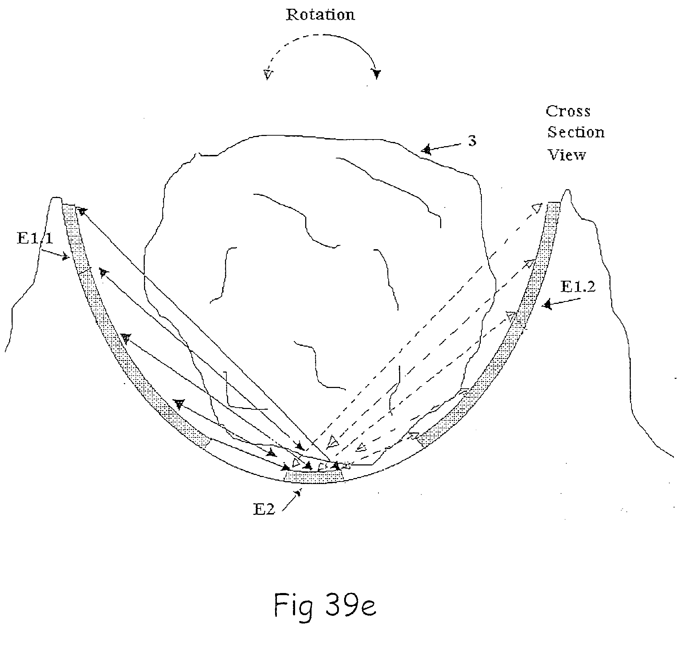

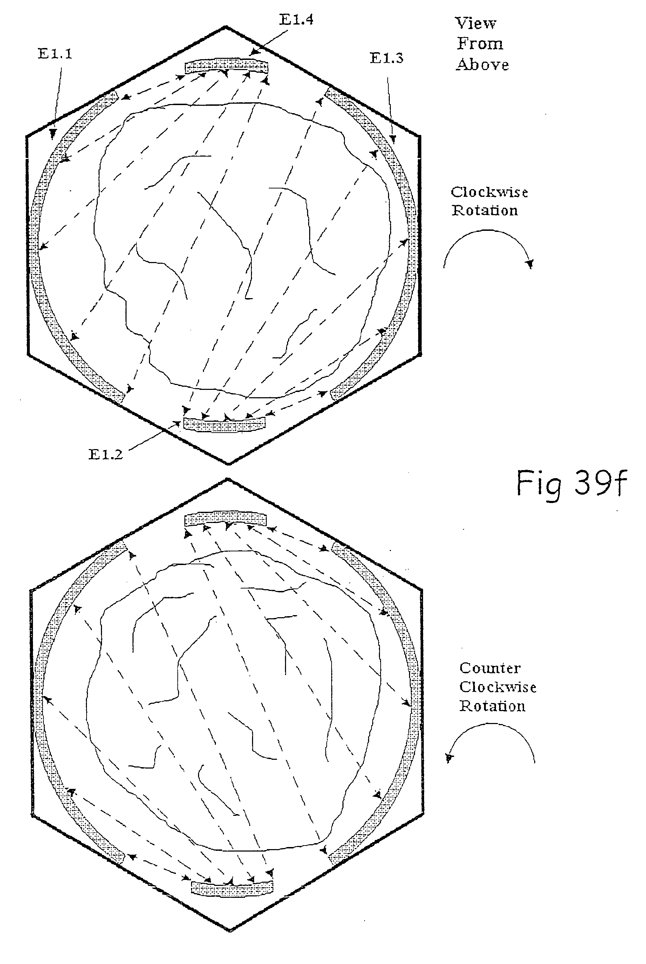

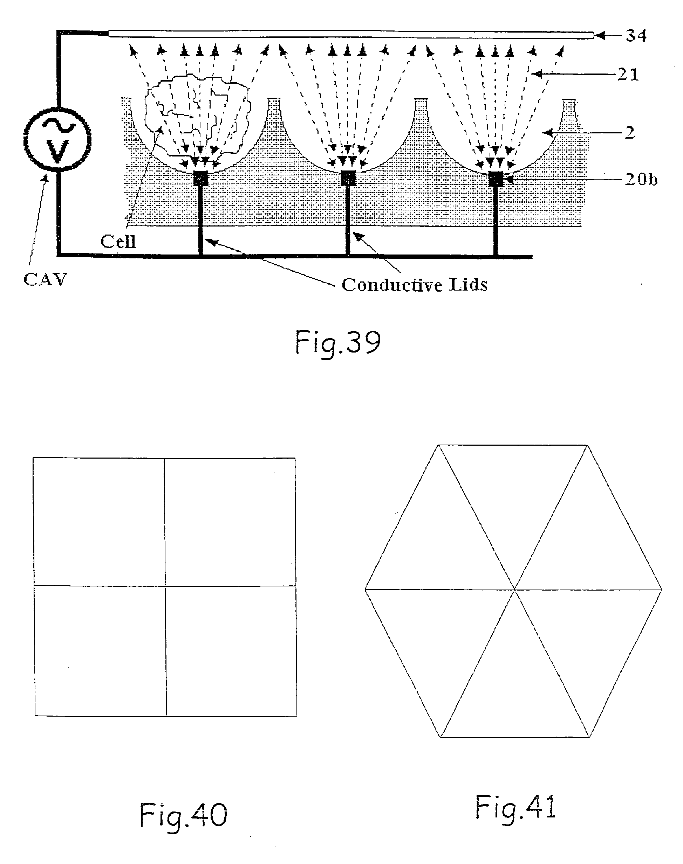

FIGs 39-39f illustrate various arrangements of cell positioning electrodes and operation of CAV circuit.

FIGs 40 and 41 demonstrate the wide scope of well packing configurations. FIG 40 illustrates an array of square-type wells and FIG 41 relates to an array of triangle-type wells.

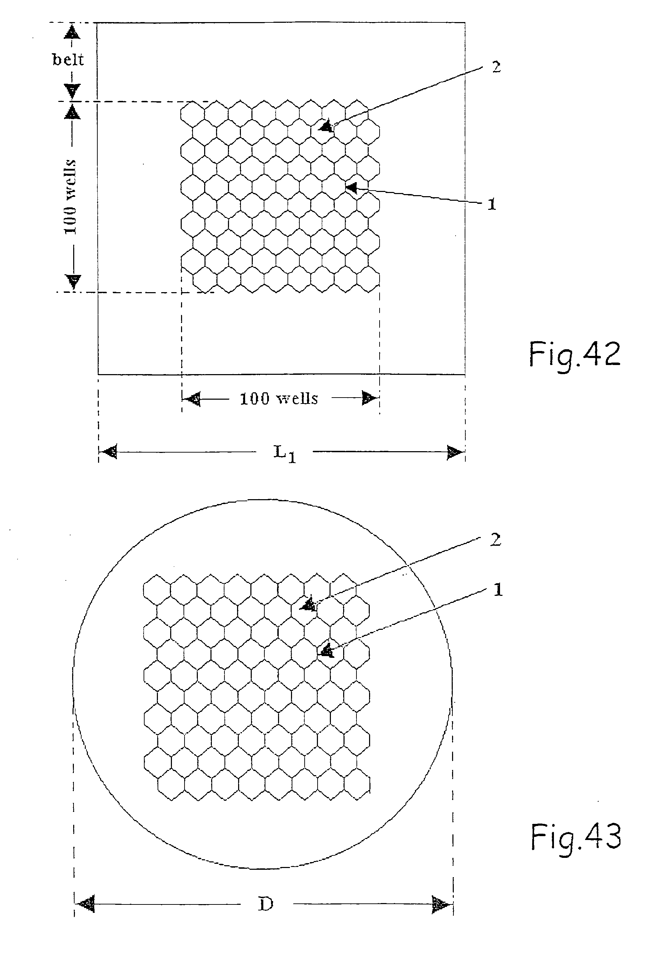

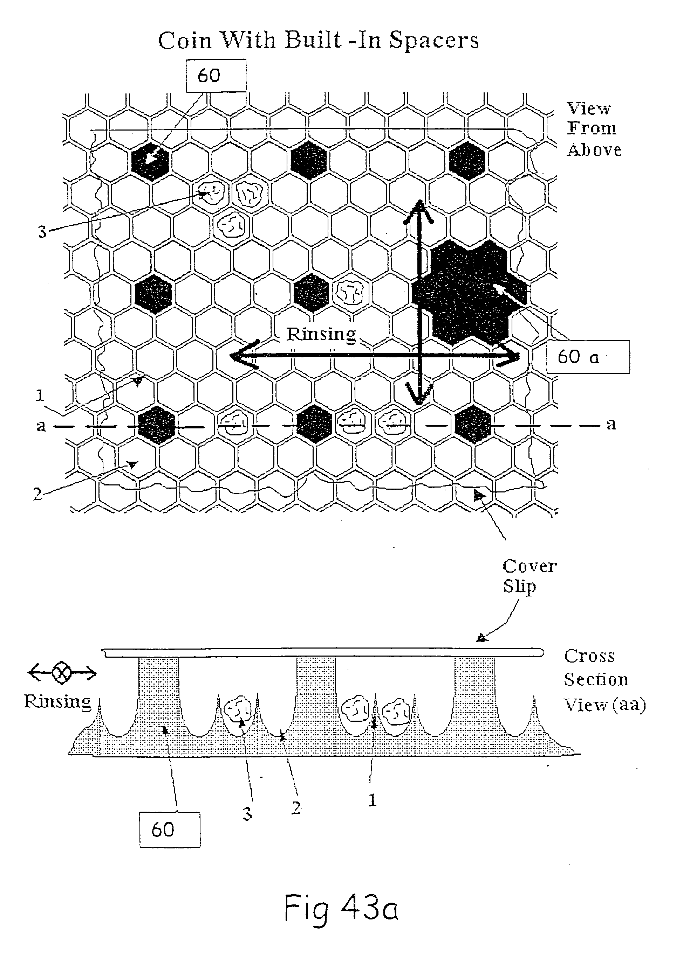

FIGs. 42 and 43 illustrate a transparent coin containing in its center a matrix of pre-determined number of wells. FIG 42 relates to a transparent square coin being a base for a matrix of 100x100 wells, whereas FIG 43 relates to a transparent circular coin being a base for the same matrix of wells.

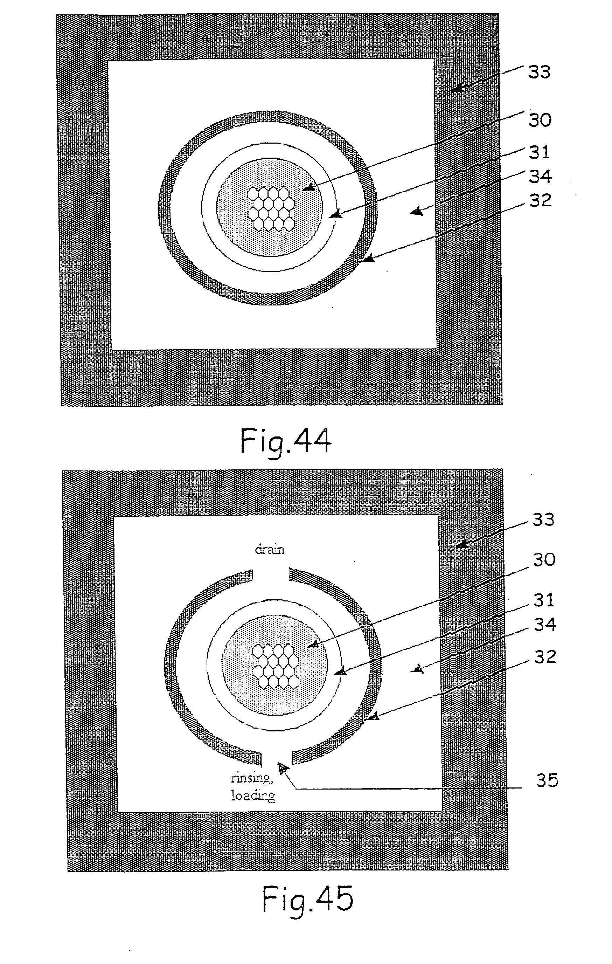

FIGs 44 and 45 provide a surface view of circular coin. Fig 45 depicts coin holder which has two openings in its belt wall opposing each other.

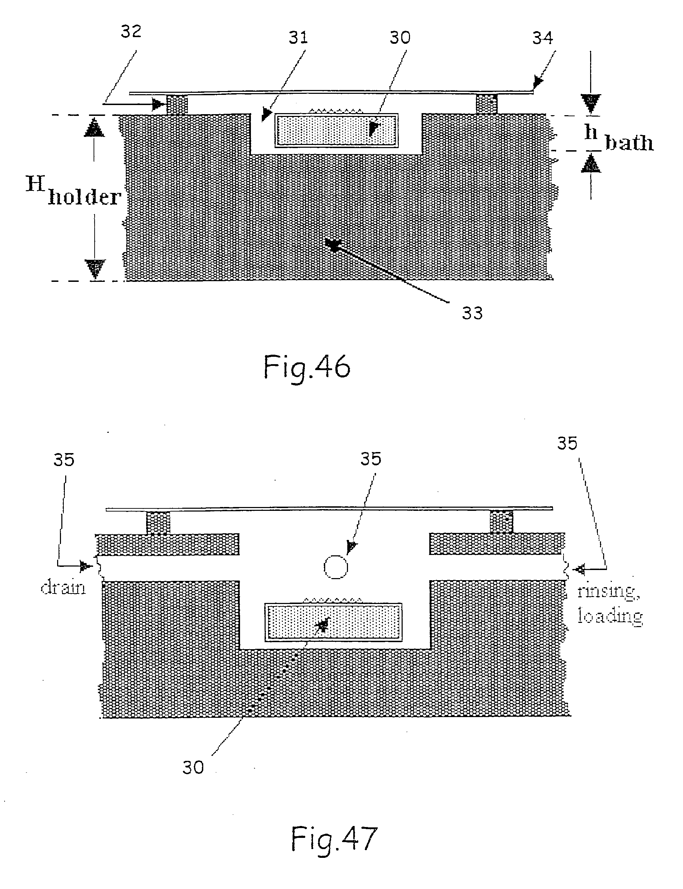

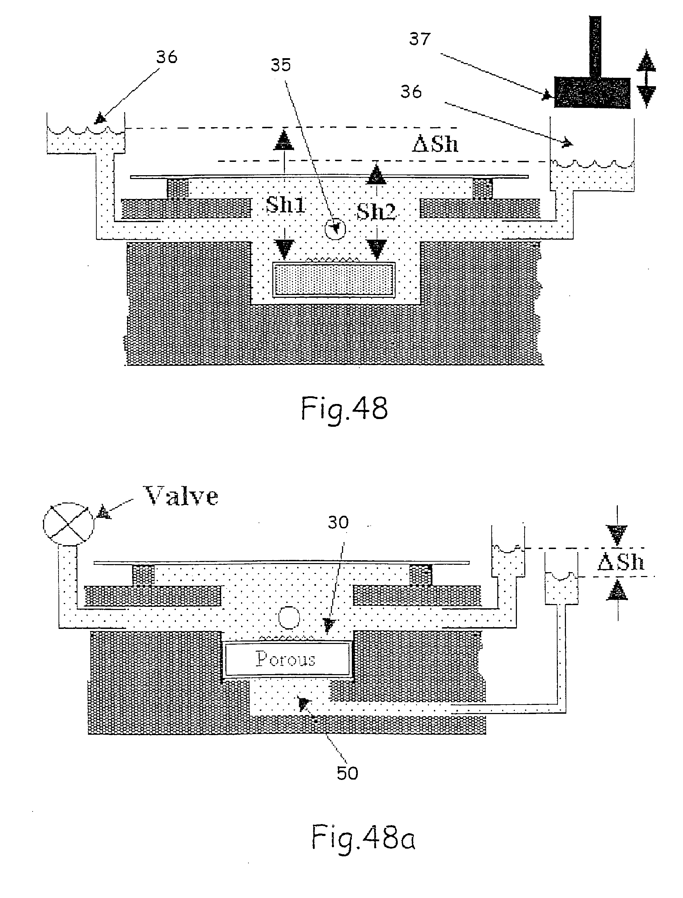

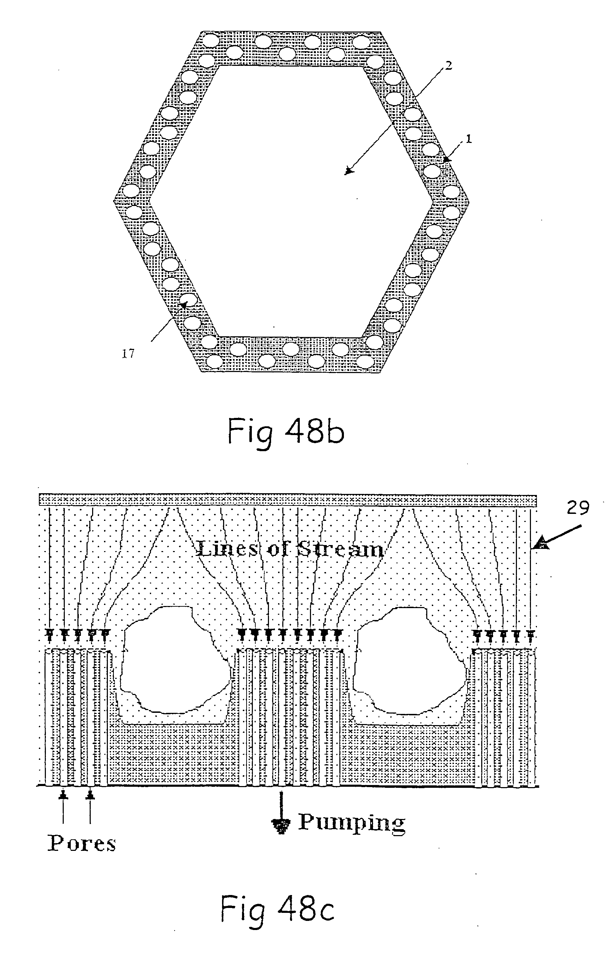

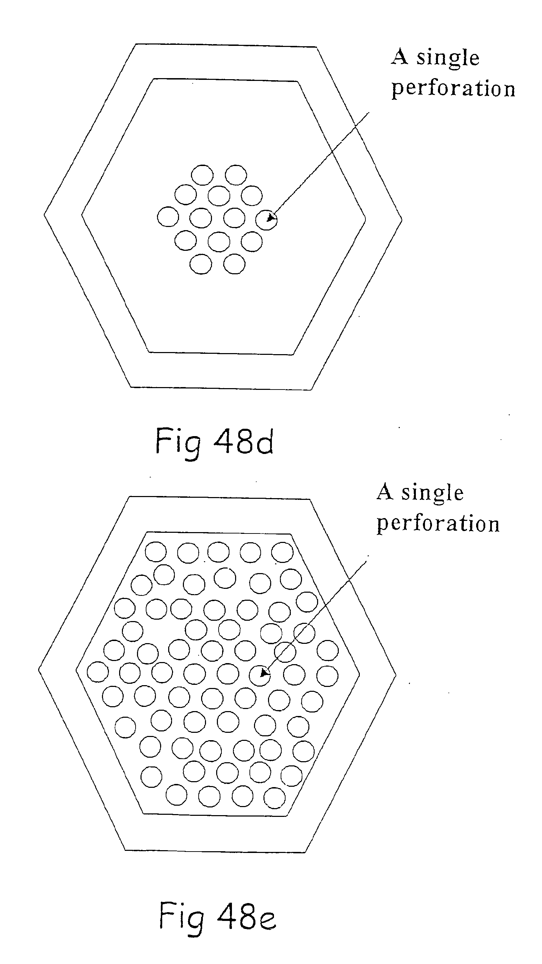

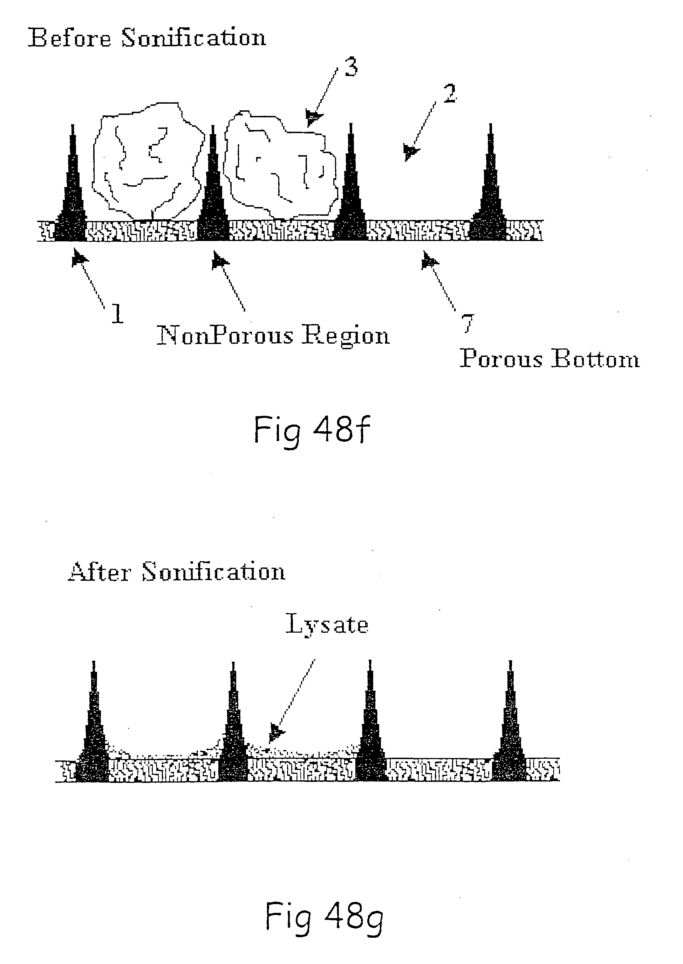

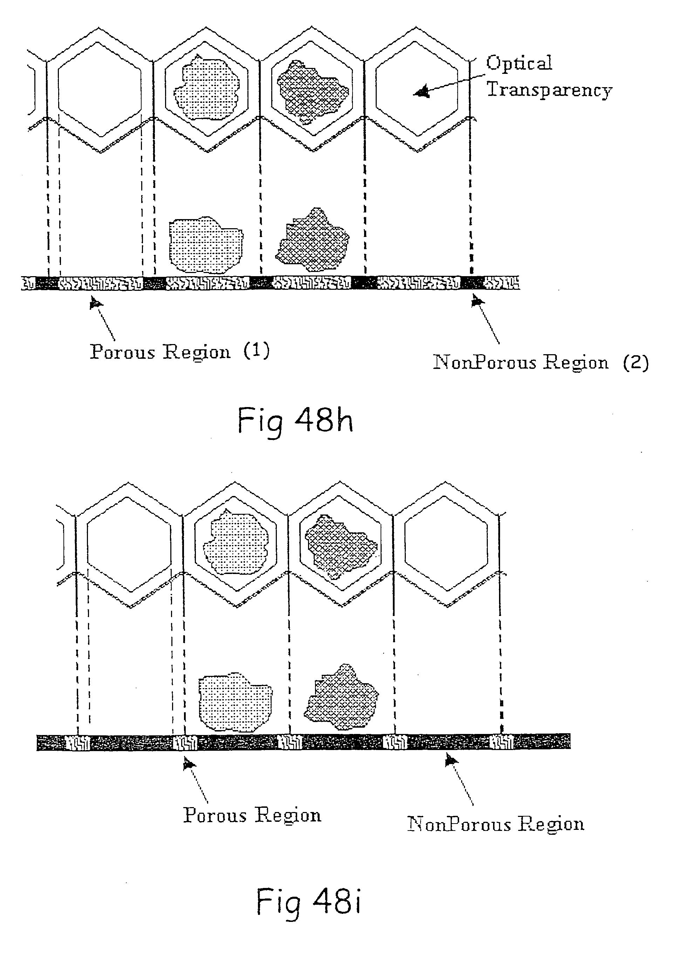

FIGs. 46-48 depict a cross section of a coin and its holder. FIG. 47 illustrates routes for loading cells, rinsing solutions and draining. FIG. 48 focuses on liquid containers and creating a suitable pressure for moving the solution inside and across the holder.

FIGs 48a-i illustrate perforated TCC. FIGs 48b and 48c show perforated well wall. FIGs 48d and 48e demonstrate perforation at the bottom of each well. FIGs 48f and 48g focus on bottom perforated wells before and after sonication treatment of cells. FIGs 48h and 48i describe porous and non-porous regions in TCC.

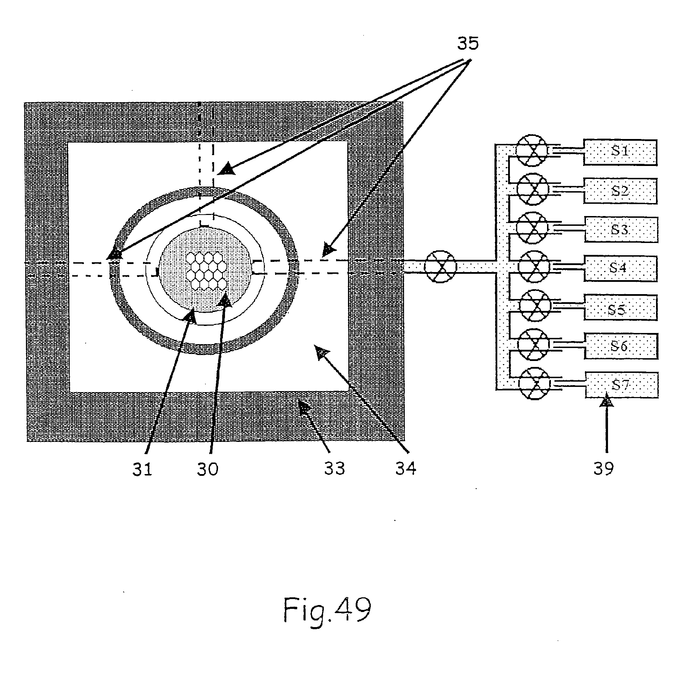

FIG. 49 illustrates the upper view of a multi-reservoir system to provide the coin and its holder several types or different solutions.

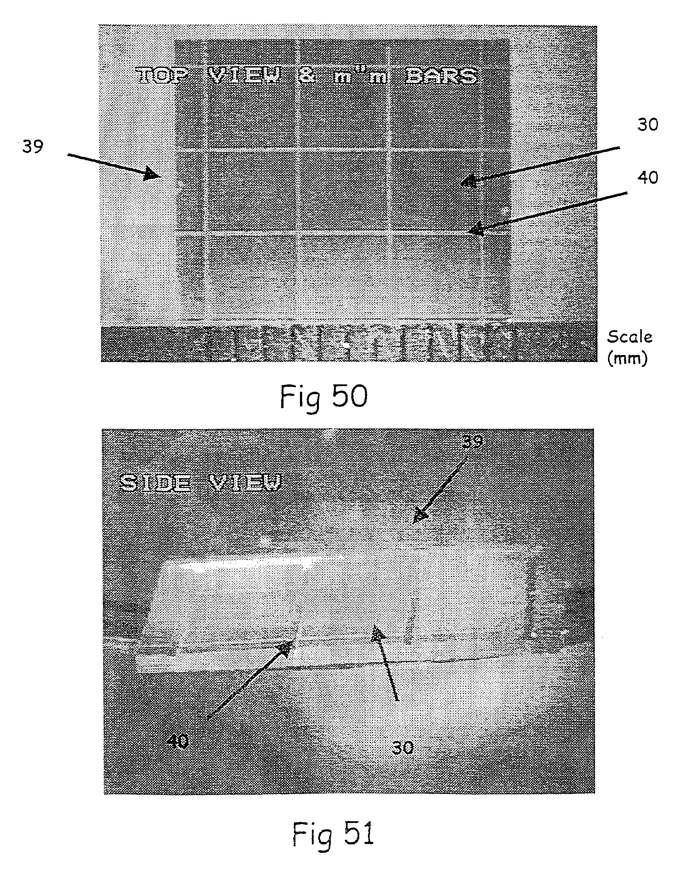

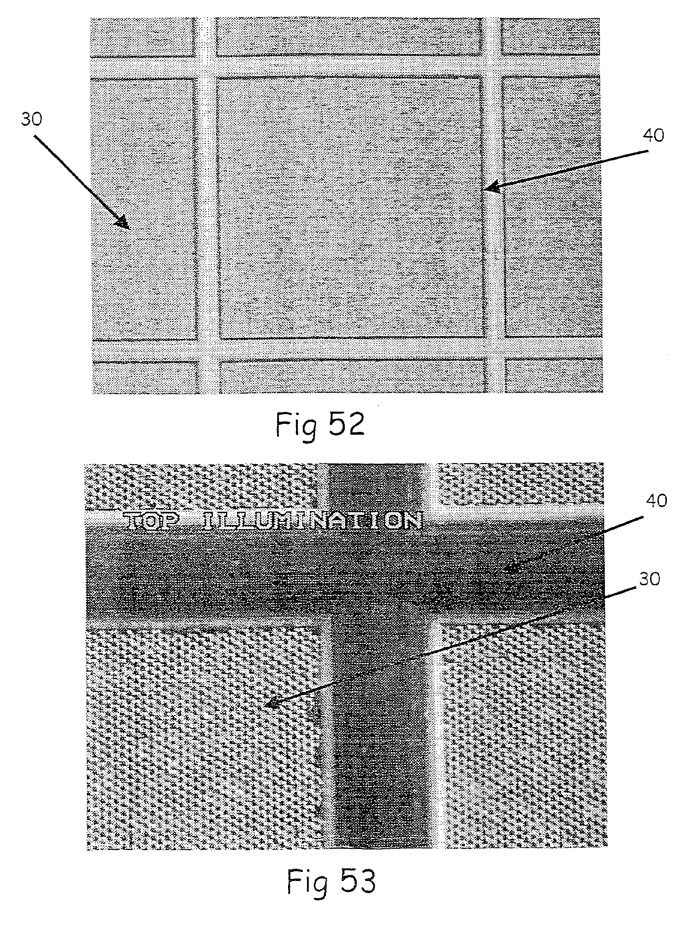

FIGs. 50-53 present a TCC consisting of an array of coins (MCA). FIG. 50 demonstrates an upper view of 9 well fields. FIG. 51 represents an isomeric view of same 9 fields, whereas FIGs. 52 and 53 demonstrate a close-up view of such fields.

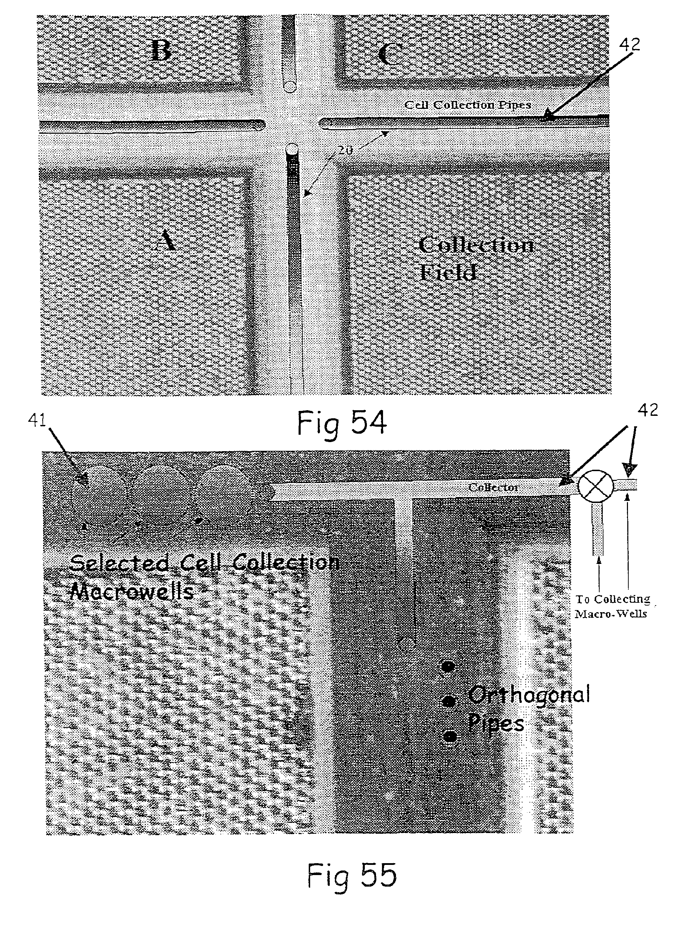

FIGs. 54 and 55 relate to transfer of cells out of their wells to either a collection field (FIG. 54) or to specially designed macro-wells (FIG. 55).

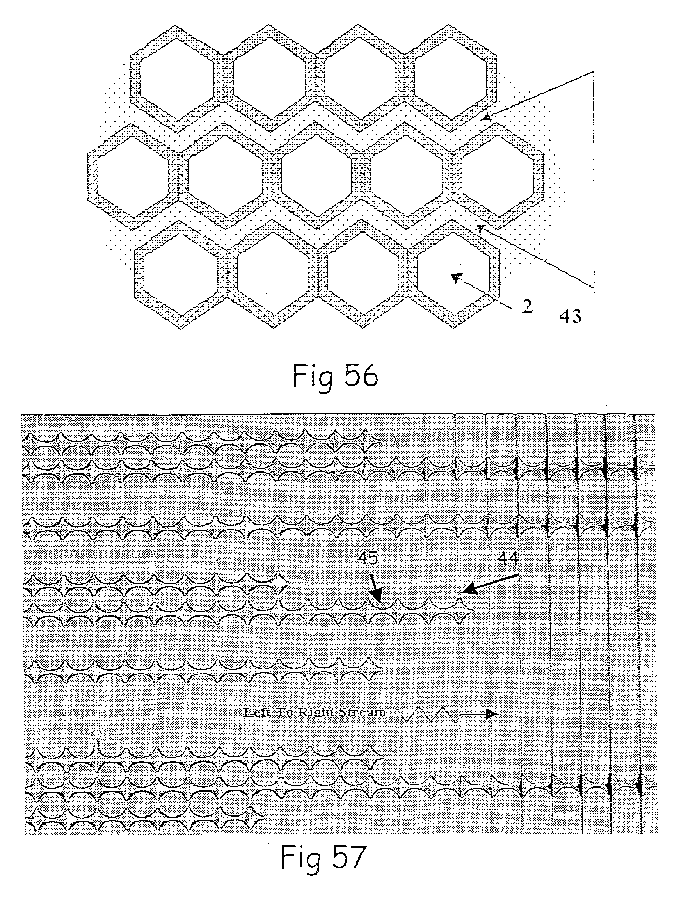

FIG. 56 illustrates passages between wells for moving suspensions and/or solutions on the surface of the TCC.

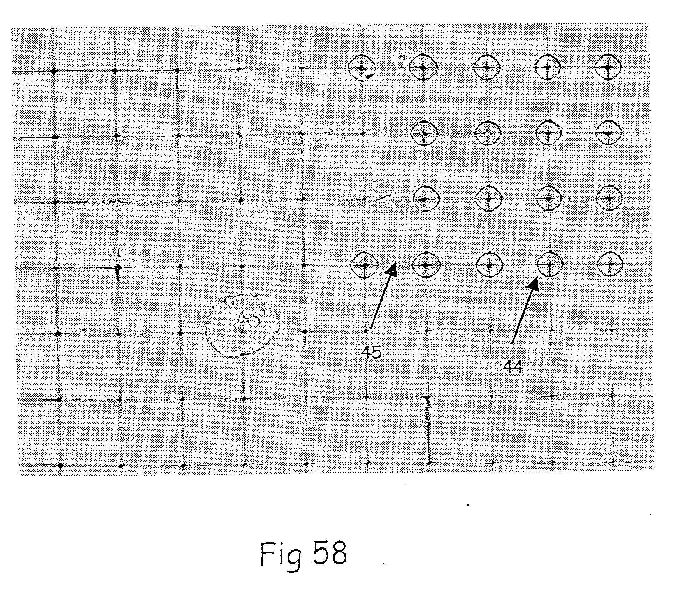

FIGs. 57 and 58 illustrate a TCC consists of wavy rounded hills in which liquid is moved in the valleys and accumulated as ponds in the intersections of the valleys (served as wells). FIG. 58 demonstrates the ponds with no movement of liquid between them.

## DESCRIPTION OF THE INVENTION

## Chapter A

## ITICBP Device: Principles of Device Structure and of Cells maintaining,

## Handling and Treating.

The following chapter relates to major features employed in the ITICBP device of present invention for selecting and analyzing a particular population of cells of a certain type contained in a biological fluid from other populations of cells. In addition, a further selection of a special sub-population may he made from the particular population selected initially. More specifically, as an example, there will be described selecting and analyzing T jurkat cells. It should be appreciated that the ITICBP device's well array of the present invention thus uniquely select populations and sub-populations of viable cells in extraordinary degree of purity, according to their well dimensions and other measurable parameters.

It is clear that cell-by-cell analysis provides far more information for the understanding of biological implications of the phenomena under study in compare to bulk or flow measurements.

The present invention elegantly and most efficiently makes it possible to quickly select individual cells for further analysis, in a high throughput manner, and an accurate process. Equally important, the present invention, in terms of both, system and method, provides capabilities for separating biological cells from one another by placing each separated cell at a known address, to which one can return, for repeated cell observation and/or analysis.

Briefly, in accordance with the present invention a large number of cells, such as for example, lymphocytes in the blood, T jurkat cells line, lymph node cells, tumor cells, and other representing groups or populations of cells, may be subjected simultaneously to selected tests and subsequently cells that are responding to these tests and/or stimulations, namely, have the same particular property, are separated for further analysis. The address of every cell exhibiting said property is known and recorded. These selected cells may represent for example, a particular subgroup of lymphocytes within the larger entire group of lymphocytes. Once the cells in the subgroup have been identified, they (together with the rest of the lymphocytes, if so desired) may be subjected to one or more additional tests. Each cell in the subgroup may be individually explored, analyzed and investigated by directing the investigative instrumentation to the cell's unique known location or address. Thus, once the cells in the subgroup have been further identified, as having a particular interested property, they are subsequently investigated, while the rest of the cells, though belonging to the same subgroup, are ignored. Consequently, once the cells of interest have been identified only they are to be further studied, thereby limiting investigation time to the subgroup of cells, which are of real interest. Furthermore, since the investigation is done on a cell-by-cell basis, a better and more precise data is obtainable for increased diagnosis accuracy

## A1. Description of the ITICBP Device

### (a) Transparent Cell Chip (TCC)

In the following a detailed description of the ITICBP device, including preferred versions, configurations, cells handling, observing, manipulating, controlling, measuring, data accumulating and the device's integrated multi-functional capabilities are discussed and depicted. The figures use the same numbers for describing the same components whether they are in different figures or in different view of the same ones.

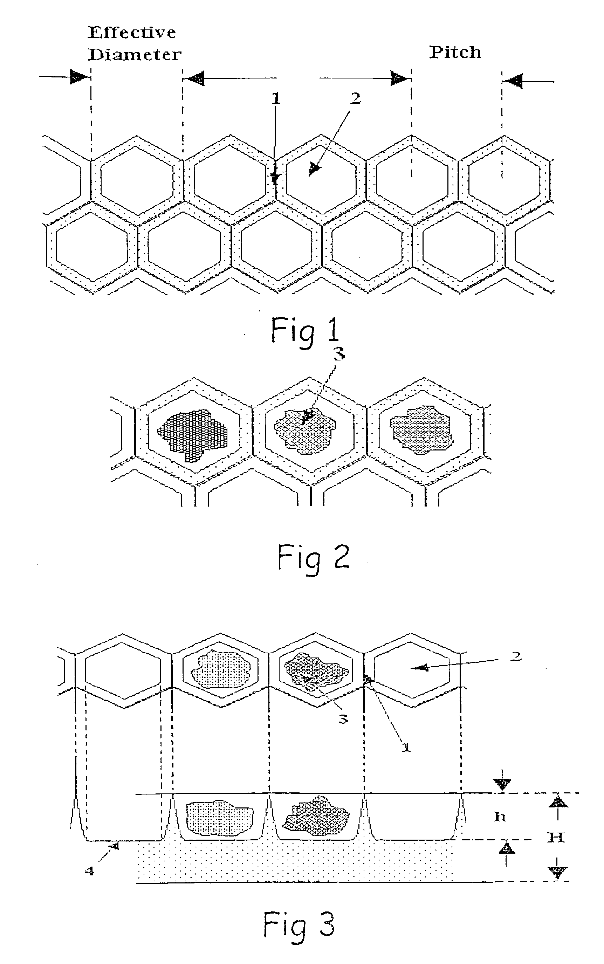

The separation between cells to be investigated is performed by means of a ITICBP device's Transparent cell chip (TCC) consisting of arrays of wells organized in a units named "coins". Partial upper view, which builds up of hexagonal wells, is shown in Fig.1. The TCC containing high density packed wells due to their hexagonal configurations, which have a designed effective diameter, and are pitched in a desired distance. The space between adjacent wells (1) and the space within each well (2) are both designed to accommodate with a single cell (3), as shown in Fig.2 (sometime, to accommodate with more cells per well, when, for example, cell-cell interaction is under investigation). The hexagonal high packing configuration with enough narrow space in between, forced the occupation of wells by the cells sedimentation, with the absence of any induced external forces with no cells remaining between the wells. A cross section of Fig. 2 is shown in Fig.3. The well depth (h) is defined by the size of the cell to be held within. The depth of the TCC (H) is variable.

A prominent feature of the present invention relates to the bottoms (4) of the wells (2) which are optically transparent and graded in order to permit visually observation of the measured cells.

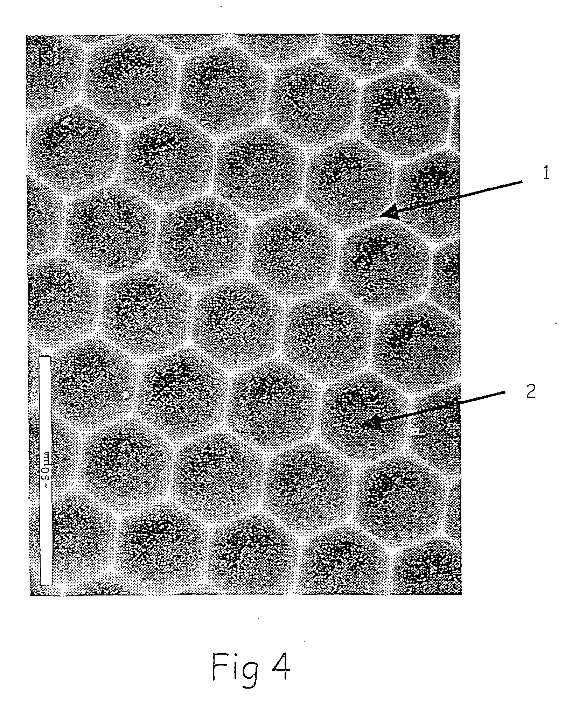

Figure 4 is a scanning electron microscope (SEM) picture, showing the upper view of highly packed arrayed hexagonal wells. The magnification's scale is given at the bottom left side of the figure. In this example the effective well diameter is 20 $\mu$m, pitched at 20 $\mu$m, having 10 $\mu$m deepness. The space between the wells (2) are the bright regions (1). A closer SEM look at one well is given at

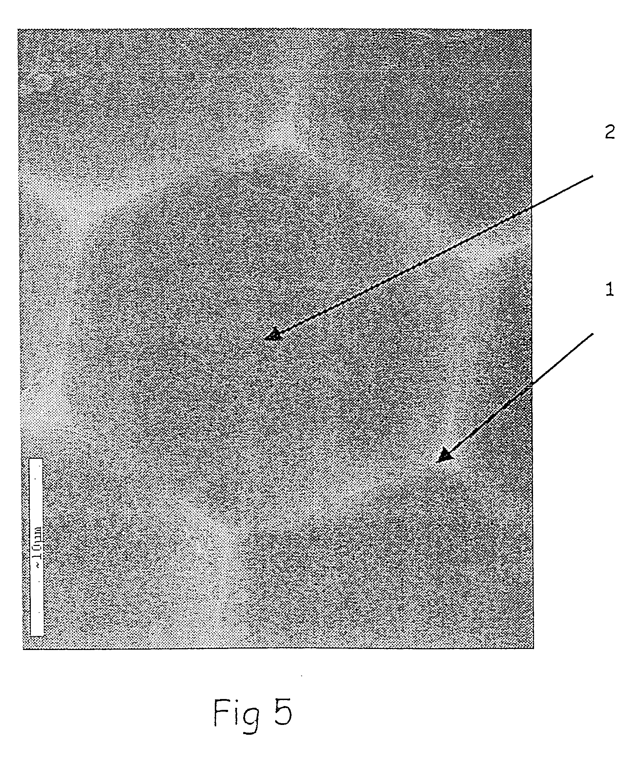

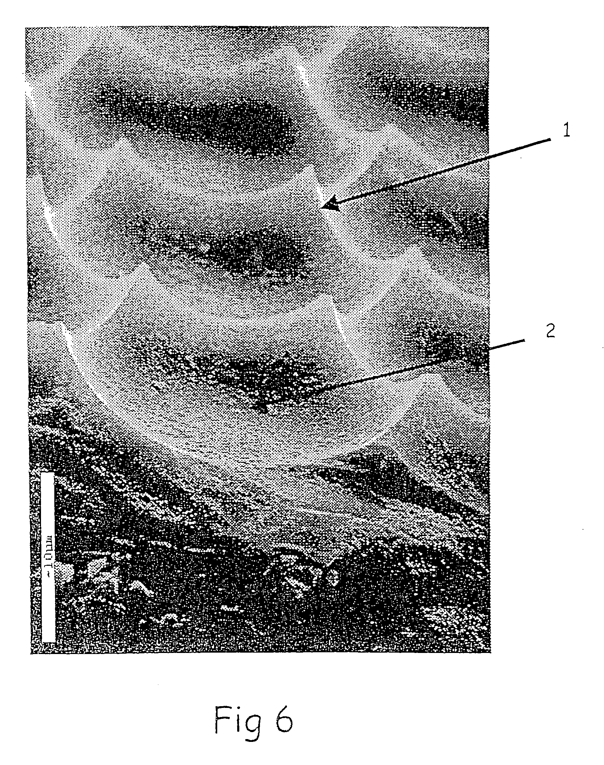

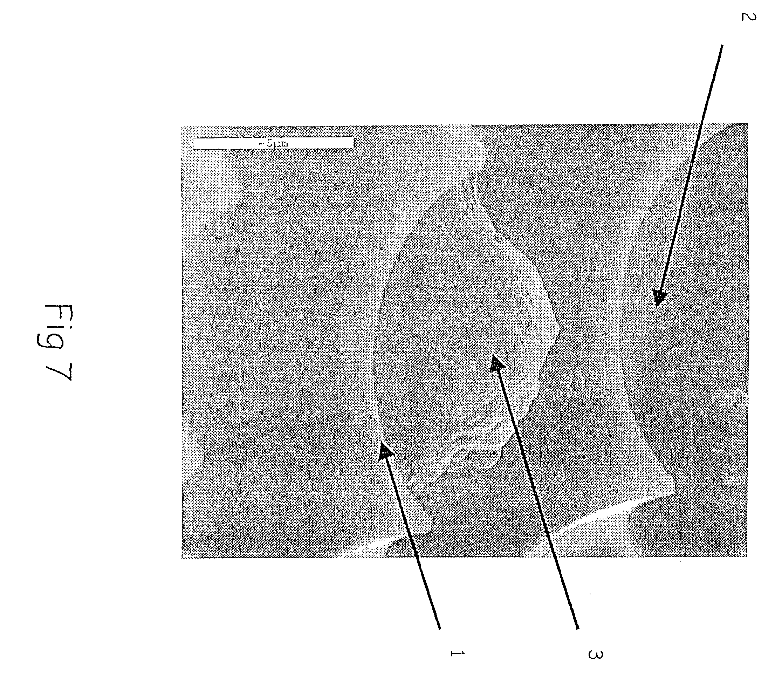

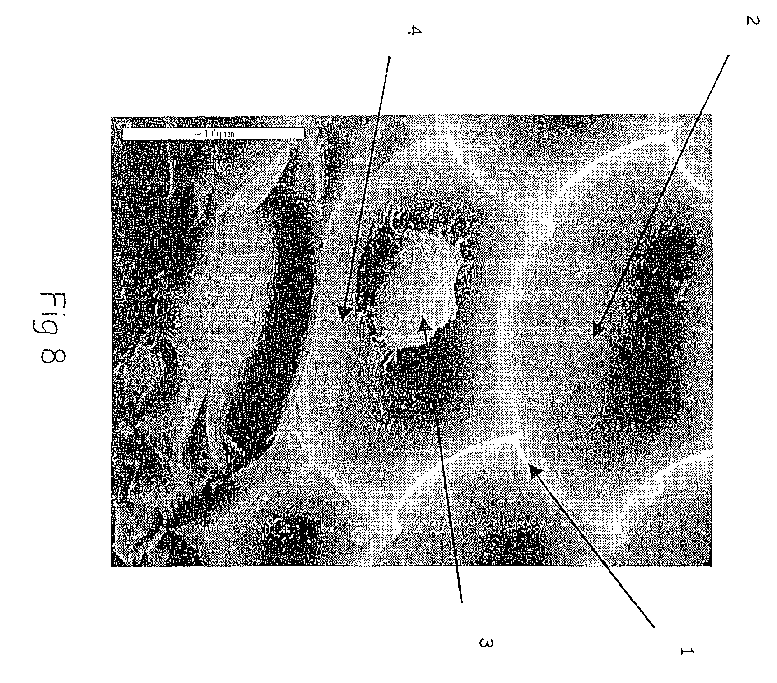

Fig.5. The sharpness of a well walls is evident and seems to be less then $\mu$m. Isometric view of few wells is given in Fig.6 and of single well, occupied with a single cell in Fig.7. Here, again, the high dense packing is clearly demonstrated. A cross section (SEM) of a well is shown in Fig.8, the flatness of the well bottom (4) and is high optical quality, is evident.

One of the simplest cells loading procedure is the administration of the cell suspension over the TCC surface. Immediately (<10 sec) following loading, cells sediment on top of the surface, forced to settle down on the bottom of the wells due to said packing configuration.

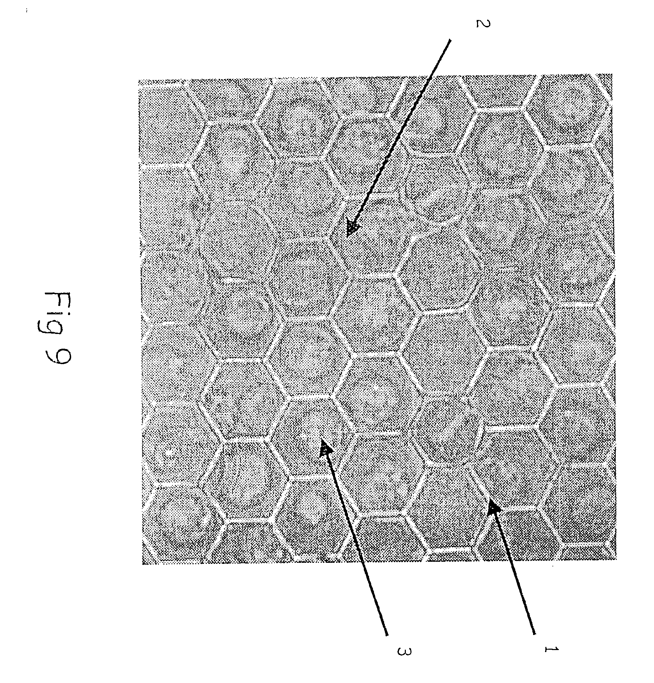

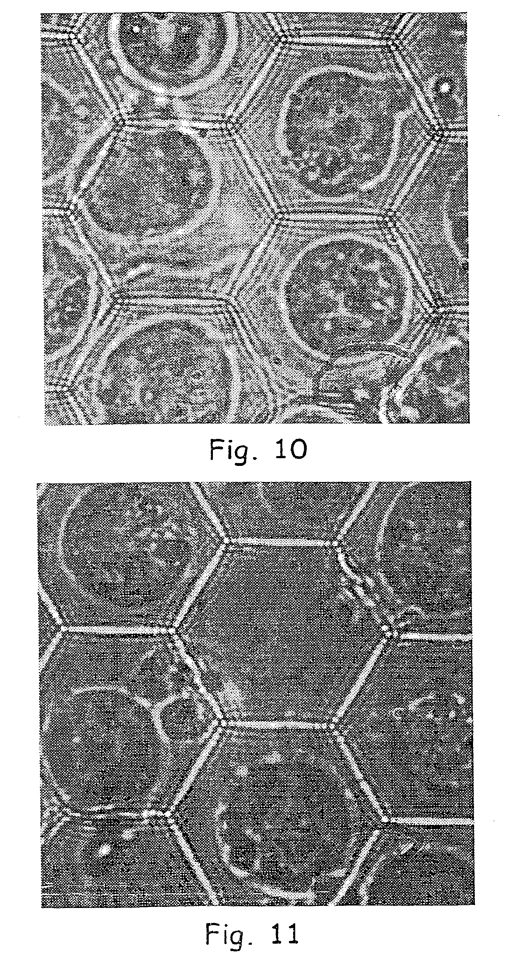

Figure 9 is a transparent light image (x40) of T jurkat cell pictured 5 seconds after the above said loading procedure. Occupancy percentage is > 90 % and can be easily pre-determined by controlling the cell concentration in the suspending media. Figures 10 and 11 are additional transparent light images (x100) which emphasis the ability of visually observe individual live cell, each handled in a micro Petri dish like well. The intracellular compartments and organelle structure are evident.

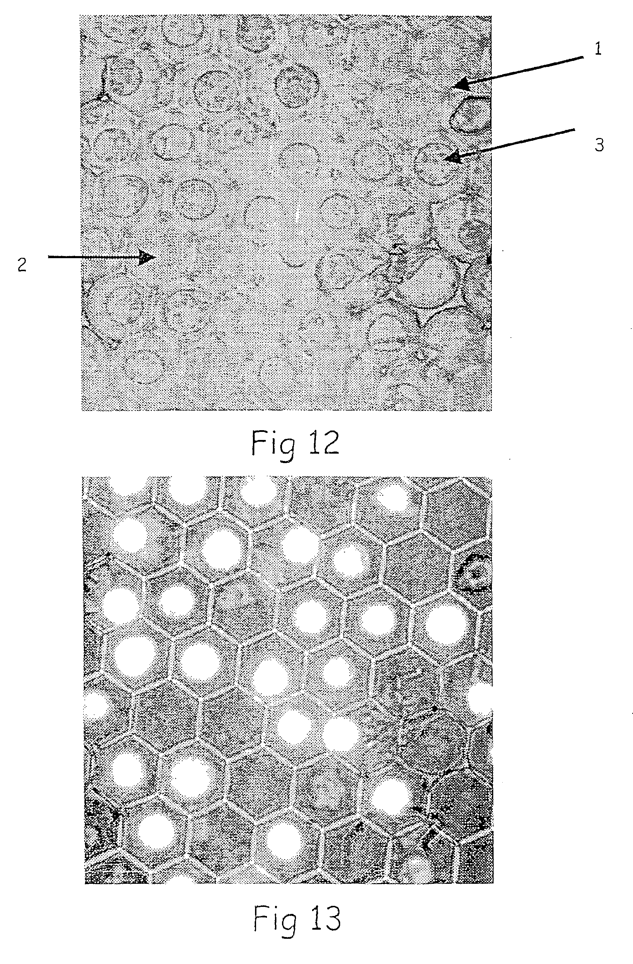

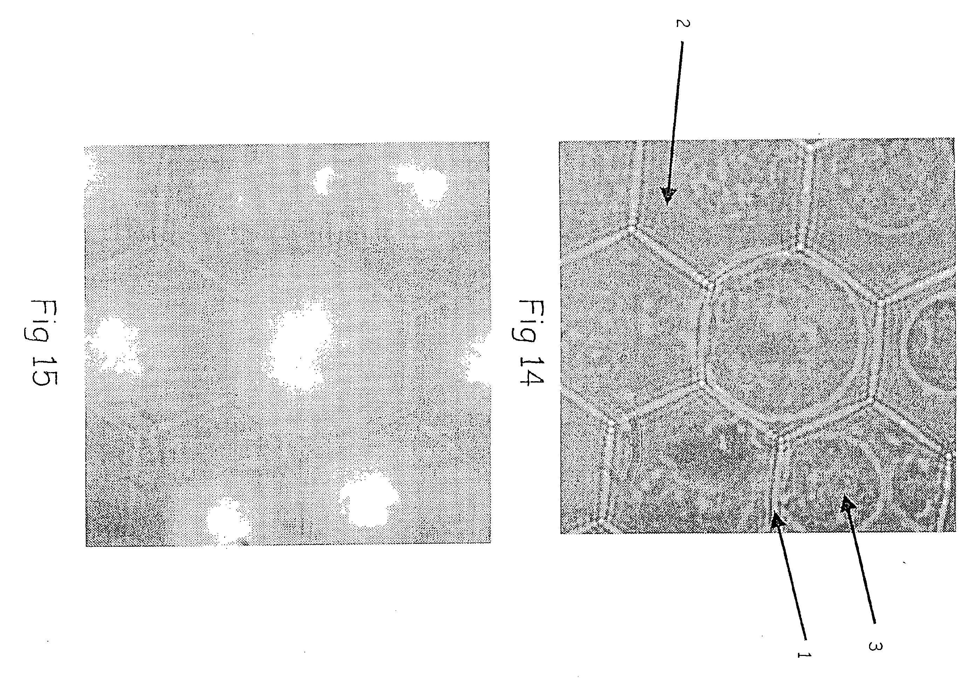

Figures 12-15 present the exclusive feature of the ITICBP device which may exhibits transparent and fluorescent images of the absolutely indefinable same individual cell in a population, while repeatedly manipulated without losing its identification. Figures 12 and 13 are magnified x40, whereas figures 14 and 15 are magnified x100 of T jurcat cells.

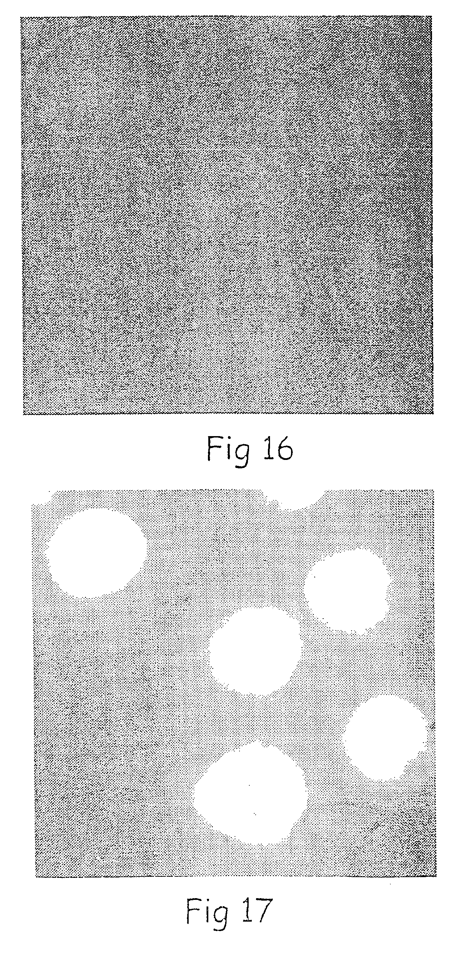

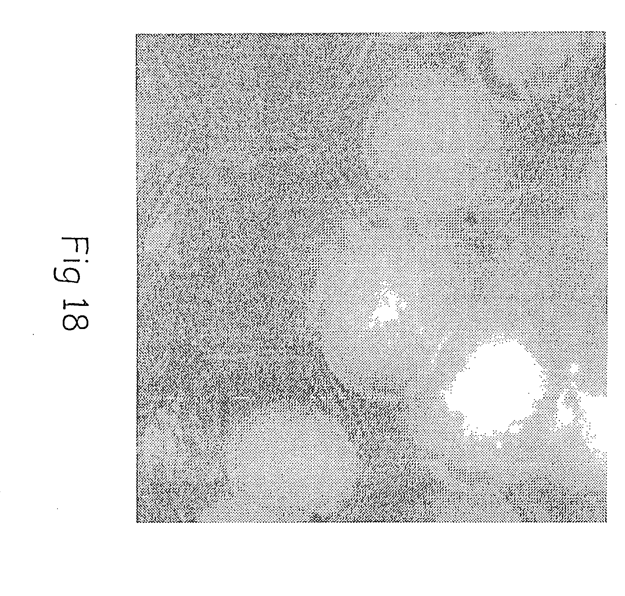

The ITICBP device uniquely enable to observe, study and trace cell-cell interactions. Figures 16-18 show the kinetics of an interaction between a killer (effector cell) and a target cell. Figure 16 features a transparent light image of interacting cell couple, located at the upper right corner of the figure. An initial cell attachment is observed. Figure 17 demonstrates a fluorescent image of the same interacting couple of cells, while Figure 18 shows photograph of the same field taken about 15 minutes later. Practically, the measurement procedure was carried out as follows: first FDA stained target cells were loaded. Control measurements of either morphometric as well as vital fluorescence parameters (fluorescent intensity, polarization, energy transfer, etc.) were then recorded. Later, the FDA stained effector cells (the killer) were loaded on top of the target cells and both morphomtric and fluorescence data were repeatedly recorded, yielding kinetics of effectors-target cells interaction. It should be pointed out, however, that the above-described procedure is an example and any type of cell-cell interaction could be explored and studied. For such studies, a specific TCC is available, containing specially designed wells, for harboring couples of cells.

The importance of the ability to trace cell interactions is far reaching, obvious and need no explanations. Drug developments, especially of those types, which play a role in the regulation of immunity, as well as in drug resistively, are deeply associated with effector- target interactions and have a high significance in basic research, as well as in the pharmaceutical industry. Obviously, questions concerning the kinetics of individual couples of cells in a population as manifested by both morphometric and fluorescence indicators and/or parameters while being explored, cannot be addressed by either presently existing sophisticated flow-cytometers and/or by advanced static-cytometers. In contradiction, the ITICBP device and methodology enables the addition of chromatic observation to the previous discussed morphometric and fluorescence data.

Figures 19-21 present the chromatic images of individual cells located within wells. The membranes and nucleus of each individual localized cell are distinguishable. Practically, after finalizing all predetermined vital experiments, cells within the wells are subjected to fixation solution followed by chromatic staining. In these particular figures, Giemsa-treated cells are presented.

It is clear that the analytical power of the present invention can significantly be strengthened by the adaptation of image analysis (IA) tools, which enables the examination of sub-cellular organelles in a high throughput performance. Figure 22 is an example of IA performed on individual cells, held within the wells. The color spectrum indicates levels of optical density. Similarly, after finalizing all planned set of vital measurement, cells on TCC can fixed and prepared for SEM observations (Figs 6 and 7).

## (b) Well's inner configuration

Fig. 23-26 demonstrate some examples of cross sections of various wells to emphasize the high versatility of well's inner structure and configuration.

Fig. 23 depicts wells having asymmetric cross section in a transparent cell chip (TCC). The left wall (5) of the well is perpendicular to the optically transparent well bottom (4), while the opposite wall has a moderated slope. This structure is designed to better maintain and hold cells within their wells when rinsing stream (6) direction is from right to left. Figure 24 illustrates a stopping tooth (7) as an integrated part of the well's orthogonal wall, yielding an undercut region beneath it, for facilitaing maintaining and holding the cell within the well.

Figure 25 depicts wall like stairs.

Figure 26 presents wavy-repeating rounded hills structure array, were cells are being localized at the intersection of "deep" valleys representing the well's bottom (4). The valleys can be used as channels for the transportation of both solutions and cells as will be shown hereinafter. Figures 27-30 are SEM photographs of the rounded hills design. The figures respectively show a lymphocyte before (Fig.27) and after (Fig.28) localization at the valley's intersection well (2) having the lowest topographical point (about $10\mu$m deep and about $50\mu$m pitched). The valleys between the hills (8) serve as routs for transportation of solutions and cells. Fig. 29 demonstrates three randomly lymphocytes either already localized in their wells (valleys' intersections) or in proximity to the wells. Fig. 30 emphasizes the possibility, if so desired, of having more then one individual cell per well for the examination of cell-cell interaction.

Figs. 31-34 provide transparent light images of cells maintained and hold by wells of TCC having these "wavy rounded hill array configuration".

Fig.31 (x40) depicts the circular circumference of a hill (upper view), lymphocytes (3) are being localized and held in few of the intersection points. This phenomenon is further demonstrated in Fig.32 (x100) and in Fig.33 (x40), in which a cell-cell interaction phenomenon is observed. In Fig. 34 T jurkat cells are demonstrated. These cells are larger then peripheral blood lymphocytes ($\sim15\mu$m compared to $\sim7\mu$m, respectively). Cell fluorescence and chromatic images, localized in the above said wavy-rounded hills array are shown in figures 35 and 36.

The ITICBP device and methodology provides, *inter alia,* elctro-chemical measurement capabilities. Each well is micro-fabricated with one or more transparent microelectrodes, that individually controlled and monitored via integrated compatible electronic circuit.

These microelectrodes are used for several applications such as, for example, cells entrapment (by means of delicate and localized Converging and Alternating Voltage), cells movement, electrical stimulation, facilitating cells fusion, detection of secreted biochemical materials, monitoring electro-biochemical reactions, etc.

Furthermore, microelectrodes conjugated with corresponding electrochemical- or bio-sensors are used for detection of cellular metabolism activity. These microelectrodes, coated with specific sensing compounds to detect pre-chosen cellular reactants, are located within the well, in the near vicinity of each individual cell. Briefly, various versions of bio-sensing ITICBP device, may be constructed. Fig. 37 depicts a TCC of ITICBP device containing wells constructed with a circular microelectrode (20) attached or deposited onto the inner surface of each well or any group of selected wells. The electrode is made of any appropriate matter, e.g inert metals such as copper, gold, nickel, silver, or semi-conducting material such as doped germanium or silicone or other electrically conducting material. The electrodes may or may not be electrically insulated by means of coating or depositing the side of their surface exposed to the well with insulating material such as plastic-polymers, glass, wax, pure silicone, and others as may be dictated by analysis needs and conditions. Each of the electrodes, transparent or opaque, is provided with an electrically conducting lead (21), transparent or opaque, embedded in the body of the TCC in such away that does not interfere with other leads and opto-spectroscopic measurements. Each lead is extending out from the TCC body to the interface electronic circuit as shown in Fig. 37. It should be pointed out that each of the electrodes is separately addressable and can pass or collect electrical signals bi-directionally, either in the direction from the cells to the interface electronic circuit or from interface electronic circuit to the cells. The controlled electrical signals provided to the cell produce an interaction between the cells and the electrodes or between the cells and their surrounding reagent solution to which they are exposed.

Controlled CAV electrical signals provided to the microelectrodes may be used to induce electric field that attract and repel the cells alternatively and thus position them in a precise location within the wells. Alternatively, this same electrode may be used as reacting-biosensor electrodes. Then, the collected signals, via the same electrodes, may be used for any measurement purposes such as extra-cellular acidification ($pH_{ex}$) measurement, selective intracellular oxidation-reduction processes which inducing cell secreted products such NO, O etc. Fig 38 presents a TCC of ITICBP device containing wells constructed with multi-electrodes (20a and 20b). Both, peripheral and central electrodes may be installed in same cells. Furthermore, the number of electrodes is variable and depends on their planned roles.

The following is a brief overview concerning electro-chemical measurements:

Each well or any selected number of wells in ITICBP device is constructed with a central electrode (20b) at their bottom as shown in Fig. 39. The central electrode (referred to as cell positioning electrode) can be easily seen in Fig. 38 (20b).

Controlled CAV signals provided to the plate of central electrodes induce an electrical field (21) that attract or repel the cell in each well and position it precisely within the well in the desired position.

Briefly, by applying a non-homogeneous alternating electric field via CAV, a cell may be confined in a volume of space defined by the geometry of the electrodes and its own shape. The electric field produced by two electrodes has been proven to attract a variety of cell types. We have conducted cell entrapment experiments using 2MHz CAV which have shown that cells are attracted from distances up to 200$\mu$m, to a couple of about 1$\mu$m electrodes diameter with separation distance of 150 to 50$\mu$m between them. In the case of ITICBP device the reference (second) electrode is a transparent metal coated cover slip, which is localized in a suitable distance from the cells plane to enable sufficient electrical field created by a given voltage (potential gap) as was demonstrated in Figure 39.

More specifically, ITICBP device is designed to attract and/or repel the cells alternatively via controlled delicate and localized Converging and Alternating Voltage (CAV).

One example is demonstrated in Fig.39, described above. Each well or any selected number of wells in the TCC is constructed with a cell positioning electrode tip (20b) at their bottom. A transparent or opaque plate (34) (can be made from regular microscope cover slip at suitable size to cover te overall TCC well field) is mounted at a given distance from the tip above and parallel to the TCC plane. Controlled CAV signals provided to the plate and cell positioning electrodes induce an electrical field (21) that attract the cell (3) in each well (2) and position it at a precise position along the gap between the plate and electrode tip.

Another arrangement, where gravity is used for the avocation of cells from wells while CAV selectively hold cells in their wells, is shown in Fig.39a. Operating selectively one of the cell positioning electrodes, while keeping other electrodes off, will yield the attraction of cell-3.1 to its well-2.1 and simultaneously the release, due to gravity of cells-3.2 and 3.3 from their corresponding wells-2.2 and 2.3. The falling cells do not accumulate in the interrogation regions since tangential rinsing is simultaneously performed.

In fact, in this example cells observation and definition for selection, either to stay in their wells or to be evacuated from, is carried out previous to the operation of CAV. The selection stage can be executed when the wells are inversely positioned, as in Fig.39a, while their bottoms support the cells. Alternatively, the cell positioning electrode may be located, electrically separately from the cover slips, while the internal walls of the wells being the same one electrode, as shown in Fig.39b. The cell positioning electrode tips are located opposing the bottom center of each of the wells correspondingly. Now, when selectively operating CAV in such an arrangement the lines of the electrical field-current will be converge upward, towards the plate electrode, yielding selective drawing-out (repelling) forces on pre-selected cells, opposing gravity. The above-discussed arrangement addresses the need of simultaneous measurements and cell selection. Again, sweeping of cells out of the interrogation region is carried out by means of tangential rinsing.

A versatile ITICBP version for simultaneous multi-handling of cell is demonstrated in Figs.39c-d, where a cross section of single representative well is shown. The well contains at least two electrodes: E1- a ring like electrode, and E2-a tip electrode, each controlled by different electrical circuit and separated by a non conductive space in between. Opposite the well, attached-to the cover slip, a similar electrode arrangement is situated containing at least two electrodes: A flat ring electrode-E3 and a tip electrode-E4 where by in between a non-conductive space is present. Again, different electrical circuits control E3 and E4. In this example (Fig. 39c) the two tip electrodes, E2 and E4 are located opposing each other. The area electrodes E1 and E3 are similarly related.

One option of operating the electrodes is as follows: in order to attract a cell to the well, electrodes E3 and E4 are electrically connected and acts as one large electrode opposing the tip electrode E2 (E1 is disconnected/not operating). Now, introducing of CAV to this arrangement will cause the fields-current lines to converge towards E2 and as a result to the attraction of a cell to the well. Inversely, in order to repel the cell out of the well, electrodes E1 and E2 are electrically connected and acts as one large electrode opposing the tip electrode E4 (E3 is disconnected/not operating). This time, introducing of CAV to that arrangement will cause the fields-current lines to converge towards E4 and as a result to the repellency of a cell out of the well (Fig. 39d).

One immediate outcome of such an electrode arrangement is the possibility to upward-downward shake/vibrate the cell in its milli-nano (micro-micro) liter volume ($\mu\mu$L). This, for example, ensures better contact between the held cell and its environmental suspending media.

This arraignment includes, as described previously, the option of tangential rinsing, which allows the sweeping of cells out of the interrogation region.

It should be emphasized that the above description is given as a non limiting example. Cells in wells can be manipulated differently utilizing different electrode shape, location, and relative positions as well as by operating them via computer programmed combinations and time order. For example, Fig, 39e depicts an arrangement where electrode E1 of Fig.39c is divided into two autonomic electrodes E1.1 and E1.2 which controlled by different circuits. Thus, a well will contain two opposing electrodes on its inner slopes where, lower in between, a tip electrode is situated at the center of the well bottom. Now repeated introduction of CAV to E2 and E1,1 (left, full lines) will acts to rotate the cell clockwise, while repeated introduction of CAV to E2 and E1.2 (right, dashed lines) will rotate the cell counterclockwise in its well.

Alternatively, four inner circumference electrodes, located on the well inner walls slope. As shown in fig.39f., two of them E1.2 and E1.4 are tip like, opposing each other. The couple E1.1 and E1.3, opposing each other too but are significantly larger then the first couple. All electrodes are controlled via separated electric circuits. Now, viewing a well from above one can understand that when CAV is introduced to the combination E1.1-E1.4 and E1.2-E1.3 it will act to rotate the cell in its well clockwise (full lines), while introducing CAV to the combination E1.1-E1.2 and E1.3-E1.4 will act to rotate the cell in its well counterclockwise (dashed lines).

This possibility to rotate individual cells to any aspired direction might have enormous importance for the morphological, fluorescence and chromatic observation of individual cells in there corresponding wells, as well as for further cell manipulation such as cell scissoring and intracellular implementation.

### (c) Wells Packing

Nature has taught us that hexagonal packaging is the most efficient way to pack. The TCC of the ITICBP device is built of arrays of hexagonal wells which do not leave space between the individual cell wells, thus theoretically and practically demonstrates an approximation of one hundred percent loading efficiency, which is important feature when cells sample size (the available amount of cells for examination) is limited. This situation is common, for example in cases of lymph node touching for pathological examinations in cancer assessment, or the small amount of cells found in saliva for lung cancer evaluation.

As for measurement procedures and cell handling manipulation, they are both much more easily and smoothly performed when using array of hexagonal wells which avoid disturbance of non-relevant cells (non-selected cells). However, the present invention is not limited to the most efficient and preferable high packed hexagonal geometric configuration, but also includes other packed configurations and arrangements, as well. Two trivial examples are arrays consisting of square and triangle wells (Figures 40 and 41).

### (d) The "Coin": Constituting a Basic Unit of the TCC of the ITICBP Device

The "coin" which is the TCC's basic unit on which the array of wells are located, may have any desired shape. Obviously such a coin was designed to permit its stable attachment either to another coin or to a holder which allows the handling and maintaining the biological sample (loading, rinsing, etc.) as well as carrying it as an integral part of the ITICBP device.

Among many options of coin configurations, the square (Fig. 42) and circular (Fig. 43) geometrical shapes, are discussed. Figs. 42 and 43 illustrate a transparent coin containing in its center a matrix of 100x100 wells, leaving a well-free space to be used for the attachment of the coin to another coin or to a holder for performing any desired manipulation such as measuring, feeding, rinsing, etc. The square side length L1 (Fig. 42) and the diameter D (Fig. 43) of said coins may be determined according to any desired need. The same is true for the matrix size, shape and dimensions of the well arrays.

Other types of coins are designed to address studies and applications which call for accurate volume of the desired reagents (which the examined cells have to be exposed to). A schematic layout of such a typical coin is depicted in Fig. 43a. The coin contains integrated build-in spacers (60) which aimed to support any type of covering means, among which are microscope cover glasses, plastic and other suitable polymers, and any kind of flexible layers such as formvar films, teflon films etc., the spacers can be homogeneously or non-homogeneously distributed and localized in the well's field and may have different diameters or cross sections (60a).

**(e) A Coin: holding and handling**

Herein, the holding and handling of coins as an integral part of the TCC of the ITICBP device, are discussed. Figure 44 depicts an upper view of a circular coin (30) encircled by a bath (31), both surrounded by a belt wall (32) being a part of a coin holder (33). The belt wall supports a transparent or partially opaque plate (34) that can be a regular microscope cover slip of a suitable size to cover the overall TCC surface and has a height, which leaves under it a space, for maintaining solutions. Figure 45 demonstrates another version of said coin holder which has two openings (34) in its belt wall (32), opposing each other, to permit loading of cells, rinsing and drainage (35). A schematic cross section of the said coin holder system is shown in Fig.46. Anther holder version is given in Fig.47 in which three orthogonal pipes (35) are drilled in the coin holder body to permit transportation of any desired solution and cell suspension. The solution (or suspension) flow rates can be controlled via pumps and valves which connected to those pipes (flumes). One of many possible ways of controlling the flow rate is shown in Fig.48. Two of the pipes are connected to two solution reservoirs (36), which a difference (?Sh) in their solution height (Sh1 and Sh2) forms a gradient that can be easily determined. The pressure in the reservoirs (36) can be easily controlled, for example, by means of pistons (37). The examined sample of cells may be exposed to various types of solutions and/or reagents and/or suspensions via controlled multi-reservoir system (39) as schematically shown in Fig. 49.

In Fig.48a the well's coin is made of porous material, made, for example, of polycarbonate, nylon, laminated and/or non-laminated teflon, cellulose acetate, glass filter, cellulose ester and similar or derived materials, all made with, or without, an internal web support. This allows passage of solutions normal to the plane of wells. Such arrangement upgrades cell manipulation possibilities and permits to maintain the cells in their wells with comfort. The use of porous coin may be associated with the existence of lower drainage, which is situated beneath the coin. Again solution streams can be controlled by both ?Sh and valves. The examined sample can be introduced to various types of solutions, reagents and suspensions via controlled multi-reservoir system as schematically shown in Fig.48a.

In fact, the use of such permeable material as the TCC is much more far reaching than the 'holding-handling' aspect. For example the use of suitable pore size together with gentle suction might enable the collection of cell secreted molecules - filtrate, well-defined by their molecular weight, on the bottom of each of the wells. The filtrate can then be marked by specific/nonspecific fluorescent/chromatic/radio- active indicators and accordingly detected.

This capability opens a new chapter in cell-biochemistry since for the first time, and exclusively it enables, in cell population, individual cell biochemistry (ICB) or dialysis (ICD), when cellulose is used.

Utilizing this concept, the biochemistry of cellular non-secreted materials can be investigated, on an individual cell basis as follows. Upon completion of all vital measurements, cells are being burst by sonication, detergents or by other means, while gentle suction across the wells is conducted. The released cellular or intracellular filtrate of each individual cell or sample is gathered on each of the corresponding well's bottom and then subjected to the required investigations. It should be emphasized that the latter issue (the 'bursting' procedure) holds true for non-porous wells since the chance of diffusion of material from one well to its neighbor is negligible.

**(f) A TCC Consisting of Multi- Coins**

An example of a TCC consisting of an array of coins (multi-coins array, MCA, 39) is provided in Fig.50. It contains 9 coins (30), i.e. nine different fields of wells, each built up of matrix of 150x150 wells and surrounded by separating channels (40) of about 0.3 mm width. One significant role of such a channel is to prevent penetrating of substances from one field to its neighbors. Hence, channels include any physical barrier between adjacent fields such as a suitable sized wall.

As can be appreciated (cf. the scale in mm), the dimensions of a single field is about 3x3 mm, and this is in confirmation with the fact that the diameter of each hexagonal well is about 20 $\mu$m.

Isometric, and two close up views of the said MCA are given in Figs. 51-53 respectively, were in Fig. 53 the dense packing of wells is evident. Each of the well-fields-coins can be distinguishly marked anywhere on its surface, for example, by a set of numbers, letters, their combination, or any other shape or color, all optically visualized or magnetically coded.

It should be pointed out that said MCA layout is only an example and thus any kind of outer frames, internal patterns and dimensions of both fields of wells (coins) and their separating channels are within the cope of present invention. MCA might have many applications: For example, as an ideal lab on a cell chip (LCC) component, where cells from the same batch are placed on different and separate coins, which are coated with different reagents or alternatively, treated with different materials. This might have an immediate application in using a high throughput and miniaturized LCC for diagnosis and prognosis, where minute sample size of the same source can simultaneously be tested using few diagnostic reagents.

**(g) Cells Handling and Manipulation: Selection, Collection, and Transfer of Cells**

Lifting up and transfer of cells out of their wells in the TCC of the ITICBP device can be done either by inducing computer controlled moving electrical field in the gap between the wells and the cover slip or by the use of the computer controlled optical tweezers as described bellow. Regardless of the method used for lift up of the selected cells, they are transferred either to the micro flumes (42) that are positioned between the well fields - A, B, C or to the collection field (Fig 54), or to any addressable field such as selected cell collection macro-wells (41, Fig. 55) as dictated by the test conditions. An under pressure condition exists in the particular flumes collectors (42) in order to suck the cells and steers them to predefined macro wells as shown in Fig. 55.

**(h) Handling and Manipulating Suspensions and Solutions**

Micromanipulation of the suspensions and/or solutions on the surface of the TCC can be established by constructing the well field with controlled gaps (43) between the wells in such a way as to create small open or closed passages as shown in Fig. 56. In such a case, the width (diameter) of the open channels is of the order of the wall width, thus preventing stable localization of cells in between neighbors wells. One example of solution flow in the valleys between the rounded hills of the packed wells described in Figs. 27- 30 is shown in Fig. 57. A flow of a solution from the left to the right of the picture is seen. As previously mentioned, wells in this arrangement or structure located at the valleys intersections between wavy rounded hills separated by valleys. The lowest points in these valleys are positioned in the center of each intersection. These intersections (wells) create small ponds (44) of the accumulated solution whereby the shallowest valleys serve as routes for connection between adjacent ponds (45). By controlling various parameters such as temperature, fluid suction rate, etc. an interruption (46) of continuity of flowing fluids between the ponds is induced, thus creating separate and unconnected ponds (47) as shown in Fig. 58.

**(i) Cells Treatment and Manipulation: Vertical rinsing of cells**

Vertical tiny perforations (in the order of 1000 Angstrom) of the well sides, as shown in Fig. 48b are created by ion bombardment technology. These perforations enable vertical rinsing of the cells. The size of the perforations is so small as compared to the light wavelengths used in connection with the ITICBP that no optical interference is caused. A cross section of the perforated well walls with the flow lines is shown in Fig. 48c.
Another version of a perforated ITICBP whereby the perforations are done at the center or at the entire bottom area of the wells is shown in Figs. 48d and 48e. This version is otherwise identical to the ITICBP described above. As in the first perforated version of the ITICBP, the sizes of the perforations are well within the light wavelength diffraction limit and thus cause no optical interference.
The advantages of vertical rinsing are discussed in the section describing the porous coin. Different perforation patterns can be made depending on the biological test conditions. It should be strongly emphasized, that the present invention includes and relates to any type of porosive material, which at least the bottom of the well and/or its walls are made of, and which contains, at least one single pore (perforation) per well bottom and/or wall, localized, either centric or eccentric, through the bottom and/or the wall.
An example for the use of porous ITICBP TCC can be seen in Fig. 48f and 48g. In the first, cells are situated in their wells which have a porous bottom (7). Following observation the cells are exposed to sonication causing their eruption, while simultaneously a gentle suction through the ITICBP is performed, ensuring the forceful sedimentation of each individual cell lysate at the bottom of its well.
Fig. 48h shows an array of porous material (1) separated in-between by areas which are non-porous (2), where the diameter of each island is suited to hold the examined sample/individual cell. Such an array can similarly be used for the well array described above. It should be mentioned that in such an arrangement the islands might be non-porous, while the areas separating them (in-between) are porous (Fig. 48i). In both cases the island planes are optically transparent, thus permitting high quality morphological inspection of the held sample-perisland.

**(j) Cells Analysis: Methods of illumination and light collection from cells located on the ITICBP Device**

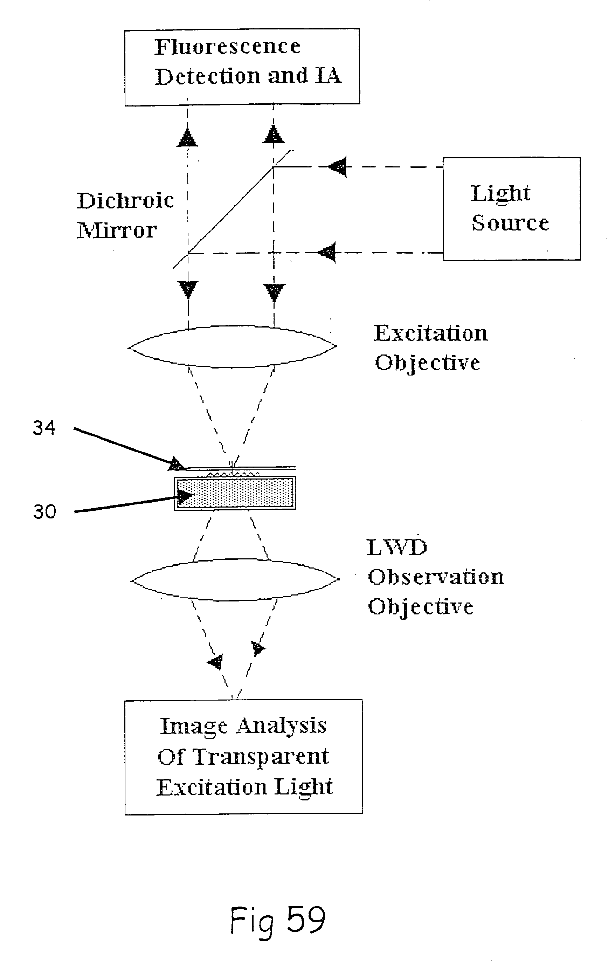

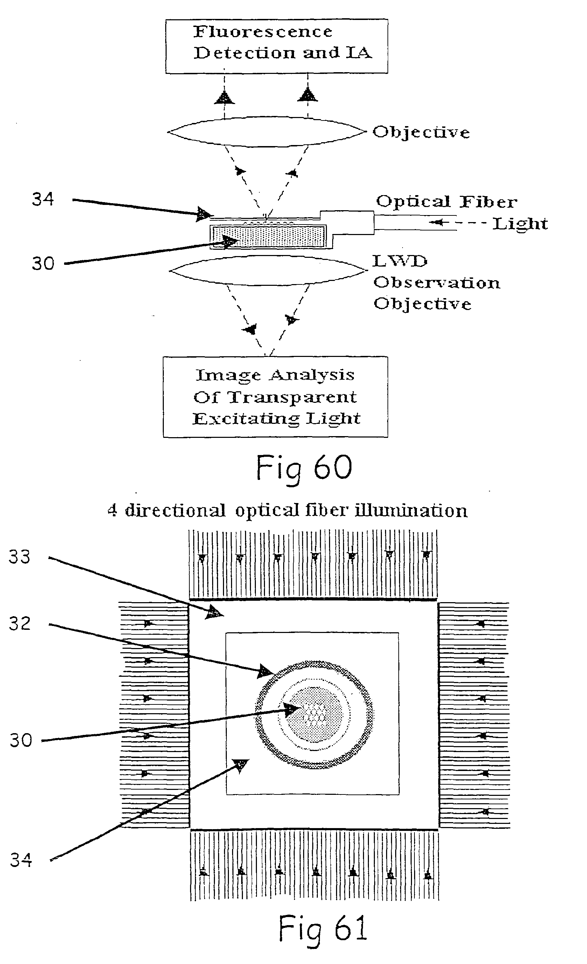

By large, held cells in the ITICBP device may be viewed and examined by the routinely used epifluorescence methods, utilizing regular or inverted microscopy. Still, the transparency of the cell chip enables viewing the cells from beneath the TCC by utilizing the excitation (epi-illumination) light as transmitted light. A schematic optical layout is depicted in Fig. 59.
Since the cell chip of the ITICBP device is transparent, illumination of the held cells can be carried out at the TCC plane, while the eliminating light propagating at the same plane. In Fig. 59 a fiber optics lead positioned at the side and parallel to the plane of the cell TCC illuminates the cells. The fluorescence emitted from the sample is collected orthogonal to the cells plane through an optical system localized above the sample and picked up by a detection device to provide the data required for image analysis (IA) or other detecting arrangements. Long working distance (LWD) objective lens situated underneath the transparent cell chip is used for cells observation and imaging by collecting the reflected and refracted light from the cells. The proposed optical arrangement is superior to that of epi-illumination (Fig. 60). Unlike the epifluorescent microscope systems, whether top view or inverted, where the illumination and emission of light are para axial, this system does not have the drawbacks plague the epi-fluorescent systems since the illumination and the emission are orthogonal to each other, thus minimizing scattered light interruption. This arrangement enables concurrent collection of fluorescence emission from the cell and observation of its morphology.

By using this illumination configuration evanescent wave traveling along the bottom of the wells is generated, as well. Detection of substances deposited on the bottom of the well in minute quantities is possible by measuring the emitted fluorescent light. This feature is applicable for the detection of mono-layer fluorescent molecules. This procedure, for example, can be used for detection of efflux or secretion of molecules from the cells. For example, specific antigen which is coated onto the well inner surface, can bind these molecules and become fluorescent upon this binding.

In another illumination arrangement shown in Fig. 61, four bundles of fiber optic leads are connected in parallel to the TCC to each side of its vertices.

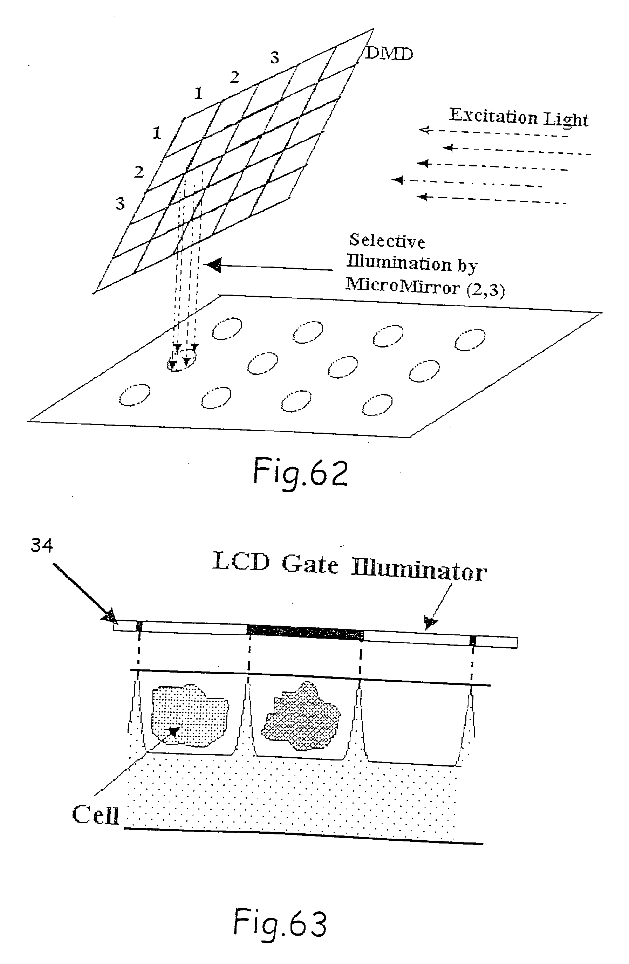

Fig. 62 shows the illumination of the TCC using an addressable Digital Mirror Display (DMD) directed towards each well. Using this method enables the illumination of any individual cell, or selected group of cells, positioned in identified wells, simultaneously.

Another illumination method applying Liquid Crystal Display (LCD) technology, in which LCD covers the upper or the lower surface of the TCC coin or holder as shown in Fig. 63. Switching selectively the LCD pixels let the illumination light to reach individual cell or any selected group of cells.

It should be mentioned, that the above described methods of cells illumination are examples to the wide versatility of possibilities exist for illuminating the TCC of the ITICBP device.

## (k) Cell per well per FOP bundle - LCD selective illumination emission optical arrangement.

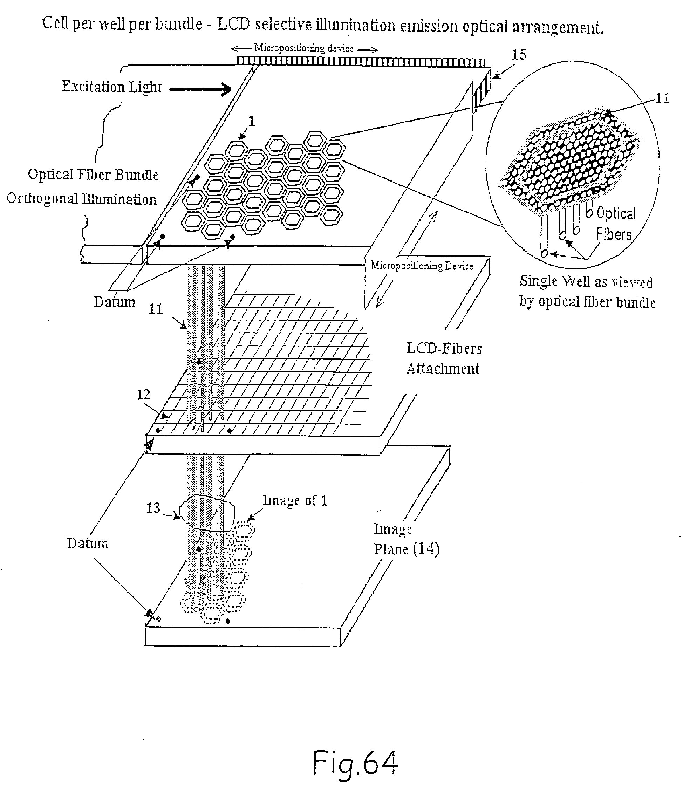

The method of utilizing fiber optics (FOP) for illumination individual cells in the TCC and measurements of their emission thereof is shown if Fig. 64. The ITICBP is optically attached, either directly or by means of optical mediating material or agent, to a FOP bundle (11) consisting of a large number of sub-micronic fibers (blow out). The size of the FOP bundle is such that it can cover either the whole area or just a portion of the TCC. The bundle is made of two sections (11) and (13) separated by a two-dimensional LCD array (12) plate. Each one of the LCD elements is electrically controlled either to pass or block, fully or partially, the passage of light between one side of the bundle (11) and the other (13). This illumination arrangement is designed to be bi-directional, illumination of the cells and their emission pass through the FOP bundle and LCD. The light emitted/scattered from the cells passes through one side of the FOP bundle through the LCD array to the other side of the FOP bundle, and finally falls onto an imaging device (14) (e.g. CCD or other sensing device) on which the whole or selected portions of the TCC are imaged.

The resolution and the size of the image is determined by the nature of the biological tests upon which design criteria regarding the number of fibers in the bundle and their thickness, the resolution of the imaging device, and the construct of the LCD array plate are laid down. Illuminating of any individual or selected number of cells in the TCC is done by a light source attached to the FOP bundle which is controllably switched by the LCD array. The disposable TCC are frequently replaced according to specific research under investigation. A quick and user-friendly replacement mechanism (15) is provided, which attaches and removes the TCC to and from the surface of the FOP bundle. This mechanism enables either sliding or lifting of the TCC onto the FOP bundle surface (optical bench). These operations are designed not to disrupt or interfere with the illumination or emission quality of the LCD-FOP-CCD SYSTEM. The location of each well on the TCC is determined both by its image as well as by that of the datum points both of which are obtained on the image plane (CCD). Both these coordinates are calculated by the imaging device computer, thus enabling a fully controlled LCD array switching, in order to provide selective illuminating light to the cells on the TCC.

Illumination may be performed from above the TCC (orthogonally or by wide-angle, an incident angle smaller than the critical angle) or from below.

In both cases, the image of each individual sample per well is viewed by the detection system (CCD) through a relevant bundle of fibers (13) thus defining the bundles pertinent to each well. The final outcome is cell per well per bundle - LCD selective illumination emission optical arrangement.

The combination of fiber optics with the ITICBP device is superior and much more practical than the idea of having permanent wells at the end of each fiber. The reason for that is the following: Biological samples, whether cells, sub-cellular organisms or solutions, leave sediments on all supporting surfaces, which becomes more difficult to clean as the supporting surface is more grooved. Optical cleansing solutions, weak acids, and even with the combination of sonication, cannot be repeatedly used with no damage to the optical quality of the supporting surface. Hence, the disposable TCC in combination with the FOPs suggest a convenient and practical solution to such a problem.

**(l) The combination of ITICBP device methodology and micro-optics**

The typical dimensions of cells and their wells in the TCC of the ITICBP device are of the same order of the integrated microlenses arrays used in micro optics components. The ITICBP device takes advantage of the new advances in micro-optics technology and integrates micro-optic systems.

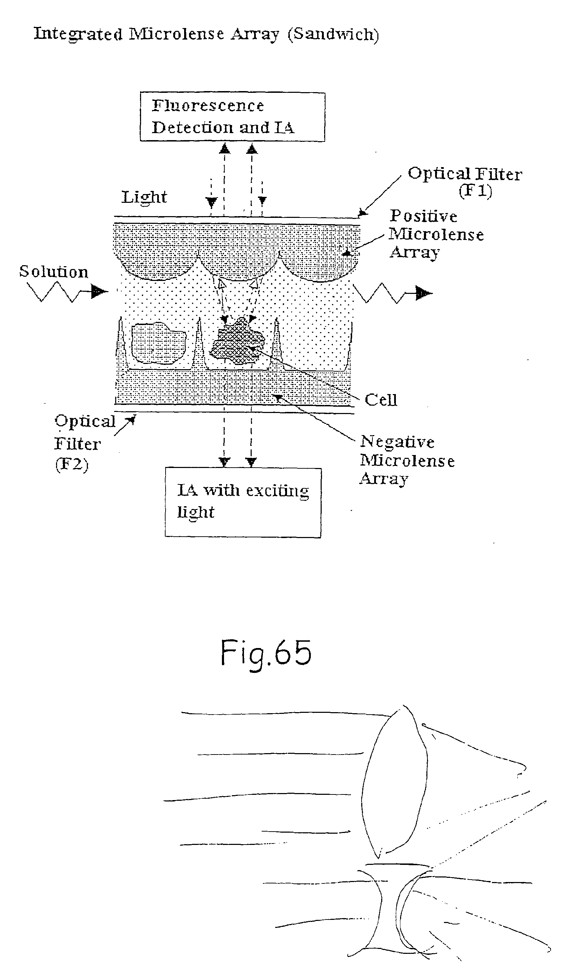

A two dimensional array of positive transparent micro lenses is positioned right above the TCC in such away that the focus of each lens is exactly directed towards the cell within its well, as shown in Fig. 65. An illumination light crosses the microlense which directs it towards the cell, then it illuminates the cells and the emitted light propagates back to the same microlense from which it is collected in the manner as described above. Wells can be practically considered as negative lenses which can be used optically and not only mechanically, as was discussed above. The Upper and the lower surfaces of the microlenses and the TCC, respectively may be provided with corresponding filters of any desired wavelength, as require the test conditions.

A more complex optical arrangement of this device includes a variety of micro optic assemblies. The space between the microlenses array and the wells is such that fluids, reagents and cell suspension required for the tests can easily flow and reach the corresponding wells in the TCC. It should be stressed that these combinations of negative and positive microlenses might end with ITICBP kits which will enable to provide cell examination with no need of a microscope (a suitable two dimensional detector array is sufficient).

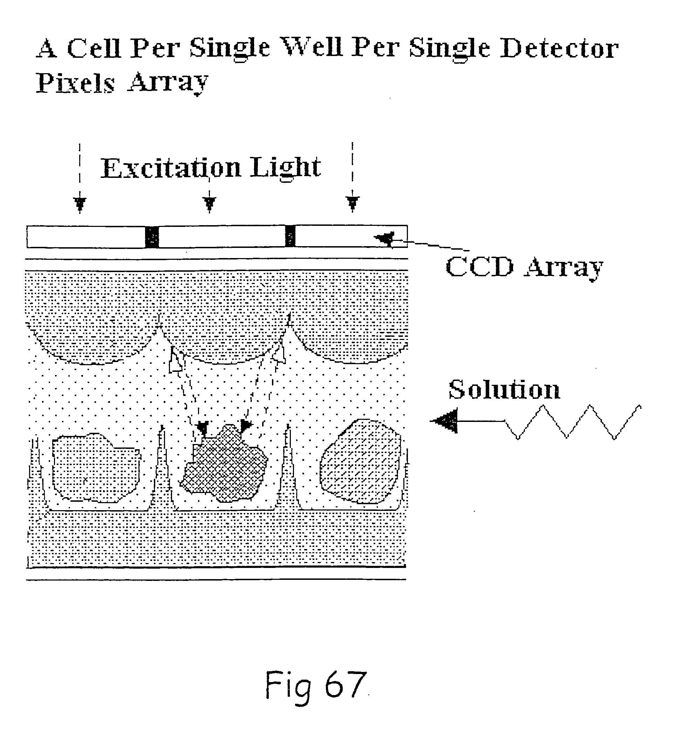

In another arrangement described in Fig. 65 the TCC and the microlenses are constructed in a sealed assembly. Containers that optionally connected to the assembly provide fluids and cell suspension into the assembly (Fig. 65a). A vacutainer provides suitable and controlled suction force that sucks the cells and reagents into the assembly. Otherwise the assembly is identical to the one described above. As an example, insertion of either biological material or markers can be carried out via a needle which penetrates the inner volume of the assembly via silicon plug. In a slightly different arrangement the sealed assembly is provided with a CCD array (Fig. 67), including in a kit form. This arrangement enables an independent self contained measurement system.

**(m) The Integrative Cell Manipulation and Measurement System**

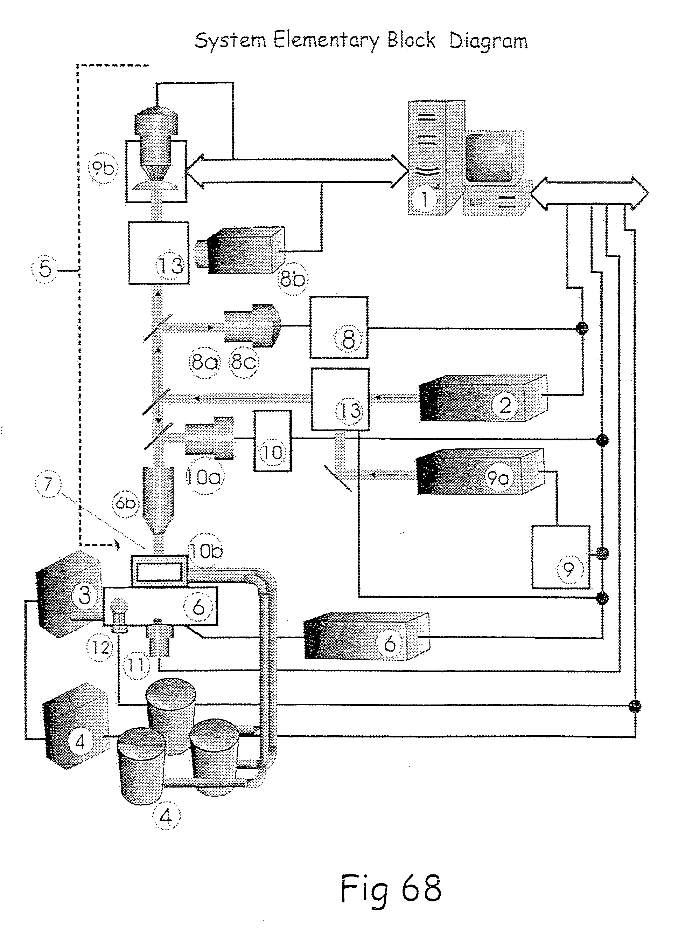

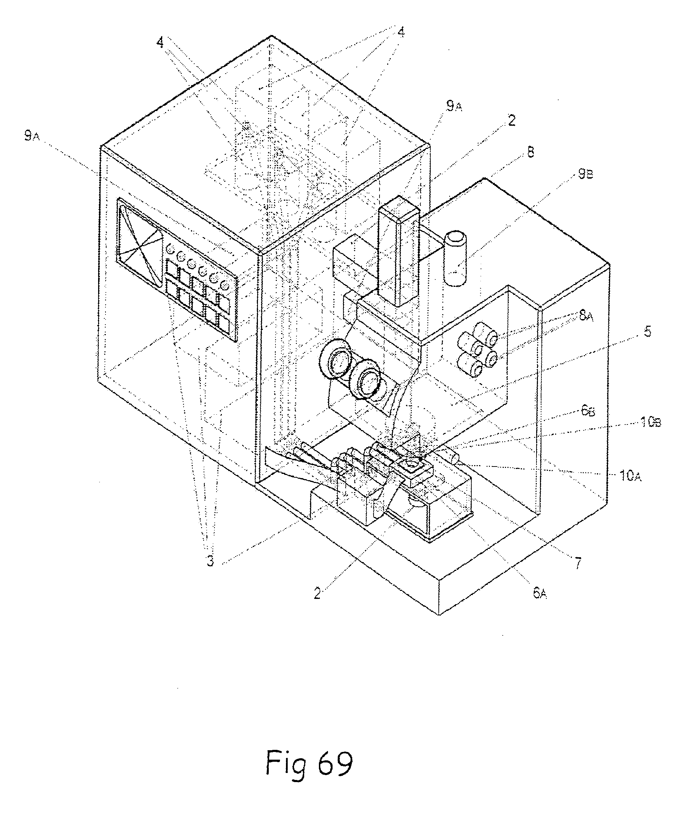

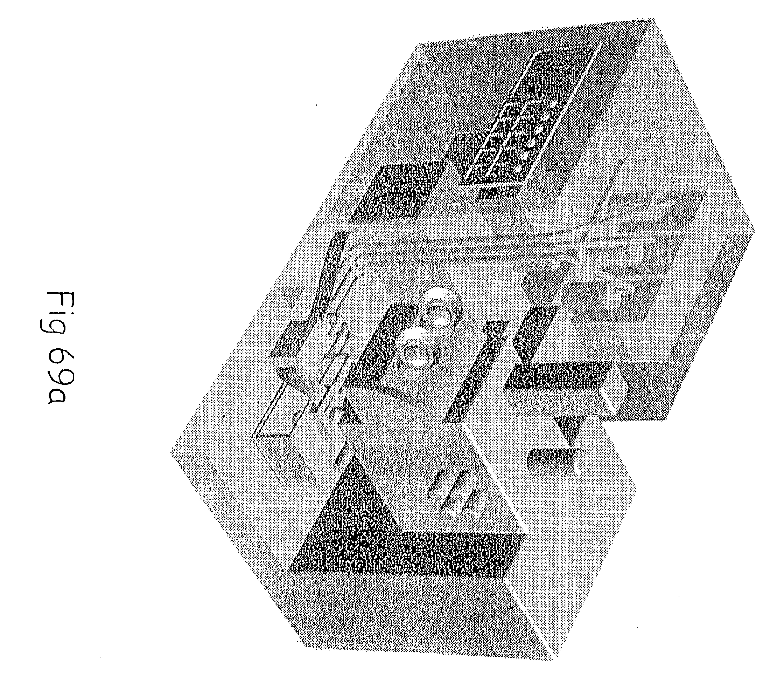

In the figures 68, 69 and 69a to which attention is directed, an overall individual or grouped cell manipulating, scanning, measurement, and analysis system is shown. The system consists of:

(1) Central control computer.
(2) Light sources such as, but not limited to, multi line continuous and pulsed lasers, spectrally continuous or specific lines discharge lamps.
(3) Electronic high speed bi-directional multiplexed signal generation and acquisition circuit.
(4) Solutions containers including appropriate electronic circuitry for time, dose, and path control.
(5) A straight and inverted combined microscope that includes all of the appropriate optical amplification, spectral filtering, and beam shaping devices for illuminating and consequently observing the samples captured in ITICBP.
(6) A computer controlled sub micron scanning mechanism that comprises of (A) a stage that can be randomly accessed in X, Y, Z, and in three rotational axes $\theta$, $\varphi$, $\partial$, (B) an optically controlled field of view by means F-$\theta$ lenses and light steering by galvanometer deflected mirrors. The scanning motion ranges and speeds are controlled by the central computer and dictated by the biological test conditions.
(7) The ITICBP as described above.
(8) Measurement transducers such as (A) High sensitivity PMT's, (B) CCD cameras. (C) CCD Line arrays enhanced with MCP (Micro Channel Plates) etc.
(9) Measurement and signal processing sub system devices: (A) Fluorescence lifetime and polarization decay measurements. (B) Spectral analysis of the observed light signals by means of spectrometer, acoustic tunable filter and alike.
(10) Means to mechanically manipulating captured cells or transferring them to or from the ITICBP wells such as (A) Optical tweezers, (B) Micromanipulators, etc.

The biological test, in general, comprises of loading cells onto the ITICBP (7) wells manipulating them with various reagents precisely dosed at given times, providing them with electrical signals, or performing other operations such as fixation. Observation and measurement of their reaction by means of the instrumentations as listed above follow or performed concurrently with these operations and cell manipulation.

The central computer (1) controls all of the test sequence of events such allocating reagents at the right time, controlling the sub systems as listed above, acquiring data from cells and their surroundings in the ITICBP (7) wells, performing data management and communication, data analysis, and all user interface functions. The computer creates databases on mass storage media onto which it stores and retrieves the measured and analyzed data.

Various light sources (2) are used depending on the nature of the tests conducted on the system. For lifetime measurement a pulsed or sinusoidal modulated laser beam is used, otherwise a power controlled single line, multi-line, or continuous spectrum light source is used. Upon illuminating the cells sample with and or a combination of the light sources (2) fluorescence is emitted and light is refracted of the cell surface or its organelles. These light signals pass through the microscope (5) and its optical system were they are filtered, or attenuated as desired by the test condition and finally picked up by the one or more of the measurement transducers (8). Depending on the nature of the biological test, the electrical signals are steered by the computer to the proper subsystem device (9) such as the lifetime (9-A) the spectrometer (9-B) or the electronic bi-directional signal processor (3). The signals processed by these devices are fed back to the computer for further processing and analysis. The physical manipulation of an individual cell or any number of cells is also controlled by the computer subject to the result of the data analysis or other desired parameter by means of the micromanipulator (10-B) or the multi optical tweezers (10-A). Selected cells are lifted off the wells they are positioned in and up to surface of the ITICBP and moved or transferred laterally to another location.

## A2. Cell entrapment by means of dielectrophoretic forces utilising delicate and localized Converging and Alternating Voltage (CAV).

The individual well in the cell carrier of ITICBP device, in addition to its unique configuration-designed to hold and maintain a single cell, it utilizes dielectrophoretic forces acting on a cell for it's temporarily entrapment.

A dielectrophoretic force is a time-dependent electric field. In short and at the simplest level it is based on the fact that the forces resulting from electrical fields can be determined via the induced dipole $\overline{p} = q\overline{d}$ being proportional to the applied electric field $\overline{E}(t)$. wherein, $\overline{d}$ denotes the vector between the two induced charges. As long as the dimension of the dipole $|\overline{d}|$ is small compared with the characteristic length of the electric field non-uniformity, the force is proportional to the field gradient, to a good approximation. This results in a (time-dependent) dielectrophoretic force $\overline{F}_d = \overline{p}\nabla\overline{E}$, and a rotational torque (rotating fields) $\overline{F}_t = \overline{p} \times \overline{E}$ (Mahaworasilpa et al., 1994; Fuhr et al., 1992). However, in the ITICBP microstructure array, the investigated cell/particle dimensions are of the same order of magnitude as the electrical cage size (distance between the electrodes). Hence, the cell/particle 'feels' the non-uniformity of the imposed field on its own size scale, dragging it along the converging lines of the electric field-current due to CAV.

The electrode size, shape and location are specially designed in order to focus the electric lines at the center of each of the wells composing the ITICBP array. Thus, dielectrophoretic forces and their phase relations become useful for single cell holding and circulation in each of the ITICBP microstructure array.

To determine the electrical potential $\Phi$ in the cage we have, besides the boundary conditions, to satisfy the Poisson equation, $\nabla[\varepsilon(\overline{r})(-\nabla\Phi(\overline{r}))] = p(\overline{r})$, and conservation of charge, $\nabla\overline{j}(\overline{r}, t) + \dfrac{\partial}{\partial t}\rho(\overline{r}, t) = 0$, where $\nabla$ denotes the del vector operator (p and j are the charge and current density and $\varepsilon$ is the dielectric permissivity). In order to neglect magnetic fields, the electric currents are assumed to be small. Provided Ohms law $\overline{j} = -\sigma\nabla\phi$ ($\sigma$ is the specific conductivity and $E = -\nabla\phi$), is valid, one obtains:

$$\nabla\left[\left(\sigma(\overline{r}) + \epsilon(\overline{r})\frac{\partial}{\partial t}\right)\nabla\Phi(\overline{r}, t)\right] = 0$$

By these equations it is also possible to describe the observation of electrolyte streaming under inhomogeneous high frequency electric fields, which should be considered in the dielectrophoretic phenomenon.

The behavior of cells and numerous microparticles in uniform and non-uniform AC electric fields was investigated by Pohl (Pohl, 1978) in the 1970's and discussed in his pioneering monograph. Especially the motion of individual cells in non-uniform AC fields, called dielectrophoresis (DP), was studied in detail in the following decades. However, less is known about the behavior of cells under electric field influence in highly conductive media. Recent progress in understanding of the electrochemical processes at ultramicroelectrodes (width less than 15 $\mu$m) led us to re-check the possibility to generate strong AC fields in original culture media. The key to the problem is the electrochemistry at the surfaces of small electrodes, which is dominated by boundary effects and non-linearity. Additionally, the smaller and closer to each other the electrodes are, the stronger the field gradients become and the lower are the necessary signal amplitudes. Therefore, miniaturization of the electrodes, development of multi-electrode systems, the insulation of terminal wires as well as the coating of the electrodes by thin dielectric layers (thickness several hundred nanometers) and a flat arrangement of the electrodes with good thermoconducting properties (silicon) are necessary. All these preconditions can be fulfilled by hybrid microsystems fabricated in semiconductor technologies (Wang et al., 1993; Schrelle et al., 1993).

## A3. Disposable close/open cell-chip chamber

One of the main aspects of the ITICBP is its being disposable close/open TCC chamber device with potential use in the laboratories of general analytical chemistry where working conditions do not satisfy requirements of handling cell cultures. As a rule in these situations laboratory animals are used. By initiating research to develop disposable living TCC and/or disposable entire flow-through-cell TCC contribute and if successful would make a strong impact on the world-wide program objective to promote using cell cultures as a substitute for animals in biomedical drug studies.

The ITICBP methodology is also in line with the objectives of American/European (western) organisations, e.g., the European Centre for the Validation of Alternative Methods, strongly promoting scientific and regulatory acceptance of alternative methods which are of importance to the biosciences and which reduce, refine or replace the use of laboratory animals.

Rapid identification of drug targets based on the progress in genome project and proven power of combinatorial chemistry to supply with a high variety of chemical structures result in a situation that a vast number of biomedical relevant compounds should be screened to detect their toxic effect and to discover safe products.

The progress in the two mentioned areas undoubtedly requires that analytical and bio-analytical testing procedures could be made in the mode of high-throughput screening. This is partly realised by substituting animals by model cells in testing. However cultivation/handling of cells and measurements are laborious and demanding in terms of qualified personnel and special laboratory's conditions, and often do not provide any picture of dynamic events as a response of cells to pharmaceutical candidates. To facilitate pharmaceutical testing, further scientific, the ITICBP is directed to realise opportunities for "on chip" clinical testing. It proposes a step in the direction of "*on chip* " clinical testing by connecting:

a) measuring technology of electrochemistry and opto-spectroscopy based on microelectrode arrays and fluorescent reporter molecules respectively with

b) individual microwell per cell chips array (cultured cell plates and/or monolayer) of non-adherent/adherent animal or human cells.

In general, cells have been grown over an array of electrodes, but those were mainly for neurochemical studies where "naked" array electrodes were meant for simple "electrical activity" (Israel et.al., 1984), i.e., electrophysiological activity, measurements. ITICBP, on the other hand, is a functionalised array electrodes to be used as specific sensors allocated underneath each and/or in the near proximity of each individual cell in its localising well. That has not been done before.

ITICBP implantable sensors/electrodes are expected to be stable due to the fact that biocompatibility problems are less severe when working with cells (Wilson and Yibai, 1999).

Functionalisation of array electrodes, i.e., making them sensitive and selective to, for example, NO, superoxide, glutamate, will relay on a vast knowledge accumulated in the biosensor technologies.

However, the work has been carried out with functionalised microelectrodes as insertable and precisely positioned probes, but not as implantable sensors/electrodes.

The most relevant procedure to modify an array of individually addressable electrodes for the deposition/ encapsulation of biorecognition molecules will be carried out by entrapment into electropolymerised polymer layers. Other alternatives also will be used such as immobilisation of, for example, cytochrome C *NO* - marker, directly on top of the ITICBP surface, or entrapment into lipids/surfactants (Bayachou et.al., 1998). The last will be used as a basis for combined optical-electrochemical (i.e., electroreflectance) detection of free radicals with enhanced specificity. Electroreflectance is a new spectroelectrochemical technique coming into the analytical chemistry field and so far has not been used for analytical purposes.

## A4. Isolation and handling of specific cells

Handling of cells that were selected by the opto-spectroscopic system based on optical and biochemical analysis of biological processes measured on a single cell basis. Isolation or reduction of pre-selected sub-populations or individual cells or cell secreted products for further manipulations, all carried out by means of optical tweezers or active directed micropipeting.

## A5. Binding of interactive molecules to ITICBP

Coating the ITICBP surface with various biologically active molecules, including light activated molecules.

Immobilization techniques on dielectric materials are well known for more than 20 years. Several methodologies, including adsorption, covalent attachment or biotin-avidin bridges, exist for binding of biologically active material to inorganic surfaces.

Thus, immobilization of enzymes, antigens, antibodies, receptors, tetramers-peptide complexes and other high and low molecular weight compounds can be accomplished on the ITICBP.

Site-specific immobilization can also be achieved, by coating the surface with streptavidin or with deglycosylated avidin. Such a functionalized surface can be used to specifically bind a photoactive ligand of biotin, called photobiotin, providing a light-addressable surface onto which biologically active molecules can be immobilized. Furthermore, at least three principle coating approaches are provided. In the first, a field of wells (a coin) is homogeneously coated with one type of reagent, yielding individual cells responding to the same single stimulus. In the second approach, each coin of a given arrangement of fields (field of coins) is coated with a different reagent so that each group of cells, being maintained by one of the well fields (a coin), is being treated with a corresponding suitable reagent (herein, referred to as "a coated designed multi-coin array"). The third approach, calls for a designed single well per cell differential coating ITICBP. In this case, any desired single well coating distribution is available, resulting in an individual cell exposure to a predefined stimulus (herein, referred to as a ''coated designed ITICBP"). It should be pointed out, that the described approaches are not limited to intact cells only, and they include cells' lysate, as well.

Example : Activation of specific lymphocytes.

Many diseases result from defects in the immune system. Inappropriate immune response plays a role in many diseases, including cancer, rejection of transplanted organs, asthma and allergies. Two types of white blood cells cells (B and T) are central in an immune response. B-lymphocytes are responsible for producing antibodies (immunoglobulins) that protect the body by destroying foreign proteins and antigens, while T-lymphocytes recognize foreign cells (including cancer cells) and mark them for destruction.

The use of drugs or specific antigens immobilized into the ITICBP surface can be used to activate or to restrain the cellular function of these cells.

Modulation of B cell activation by specific drugs, which are immobilized to the ITICBP, can be use in drug discovery for autoimmune diseases such as rheumatoid arthritis and diabetes.

Antigens specific for T cell receptor, immobilized to the ITICBP surface can be utilized for activation of specific ligand binding T cells. This is of great need in the field of tumor immunology, where tumor-antigen-specific T lymphocytes are used for experimental immunotherapy of cancer.

## A6. Photoactivation of ITICBP binding material

Flash photolysis of a photoactivatable materal results in its release from a "caged" condition. Uncaging is easily accomplished with the illumination of the ITICBP, providing a means of controlling the release, both spatially and temporally, of biologically active products or other reagents of interest. The "caged" molecule is designed to maximally interfere with the binding or activity of the molecule. It is detached in microseconds to milliseconds by flash photolysis, resulting in a pulse of the active product.

The effects of photolytic release can be monitored either with fluorescent probes or with the electrodes embedded in the ITIPBC. Photoactivation of caged molecules (drugs, antibodies, antigens) rapidly initiate or block cellular activity thus providing tools for kinetic studies of functional characteristics mentioned above and to be performed on individual cell basis.

### Chapter B:

### ITICBP Device: Measuring Techniques and Procedures Applicable

The following features and applications are given for demonstrating in a non-limiting manner, the wide scope of measurement capability of the ITICBP device:

- Determination of extra and intra-cellular NO, $O_2$ radical and glutamate accumulated in each of the ITICBP wells and/or cells, by means of fluorescence and electrochemical measurements.
- Assessing of toxicity of biomedical substances using identifiable, individual cells.
- Monitoring structural and morphological parameters of living cells.

- Cell entrapment by means of dielectrophoretic forces utilizing CAV.
- (Bio)electrochemical measurements using integrated biosensor technology.
- Immuno-reactivity determinations by means of immunosensors.
- Estimation of individual cell intracellular enzymatic reaction.
- Respective applications:

  1. Individual cell biochenistry.
  2. Individual cell dialysis.
  3. Performing a vital and SEM examination on the same individual cell.
  4. Controlling individual cell pose according to specific measurement needs, such as, topographic distribution of fluorescence measurements within the cell, in cytoplasm vs. nucleus, permits analysis of the translocation of regulatory molecules such as, $NF_kB$, etc., and is essential for FISH analysis.

## B1. Fluorescent Testing

The ITICBP device of present invention and its peripheral measuring system harness the unique properties of fluorescence testing, including:

- Fluorescence intensity (FI)
- Fluorescence spectra (FS)
- Fluorescence resonance energy transfer (FRET)
- Fluorescence polarization or anisotropy (FP or FA)
- Time resolved fluorescence (TRF)
- Fluorescence lifetime measurements (FLT)
- Fluorescence polarization decay (EPD)
- Fluorescence correlation spectroscopy (FCS)

Fluorescence measurements are accurate, relatively sensitive, flexible, and safe.

When compared to other biochemical and cell-based labeling techniques, fluorescence has significant advantages over such methods as isotopic labeling, colorimetry, and chemi-luminescence.

It is relatively simple for modern instrumentation to reliably detect a signal being emitted from a single fluorescent molecule. In biological applications, this level of sensitivity allows molecules that may be present only in very small numbers to be easily detected and in the case of ITICBP device, their intracellular location can be determined. This high level of sensitivity also means that transient biological events can be detected very quickly, hence enabling the measurement and understanding of events that occur very rapidly inside a living individual cell. Importantly, the inherent sensitivity of fluorescence technology also permits the use of very low concentrations of fluorescent label. Compared to other labeling techniques, this adds greatly to the reliability of data, as the reporter molecules do not interfere with normal cell functions.

The specificity of fluorescent markers is generally high. They are readily available for labeling virtually any biomolecule, structure, or cell type. Immunofluorescent probes can be directed to bind not only to specific proteins but also specific confirmations, cleavage products, or site modifications like phosphorylation. Individual peptides and proteins can be engineered to autofluoresce by expressing them as AFP chimeras inside cells. The use of high affinity antibody binding and/or structural linkage during labeling provides dramatically reduced nonspecific backgrounds, leading to clean signals that are easily detected. Such high levels of specificity enable to apply the new ITICBP device for simultaneous use of several different fluorescent labels - each emitting at a unique color, in order to study and understand the complex interactions that occur among and between sub-cellular constituents in an identified, single viable cell. Alternatively, multiple fluorescent labels can be used in a series of sequential quantitative studies on same individual cell, or on a group of cells, allows measurement of multiple cellular responses, either simultaneously or sequentially. Compared to other labeling methods, detection of fluorescence reagents offers superior dynamic range, linearity and accuracy.

Nevertheless, there are few but crucial drawbacks of fluorescent markers and fluorescence methodology that makes them inefficient:

- The quality and accuracy of the measurement is determined by the method of staining. The high degree of non-uniformity of fluorescent probes made by different manufacturers and their limited shelf life has, for years, presented a major measurement problem in routine research and clinical applications, and in reaching a general consensus pertaining to the interpretation of the results. These problems are further complicated since the staining preparative techniques, such as fixation and permeabilization which may be needed to get the dye into cells, are not yet uniform and/or established. (Shapiro, 1995).

- Uniform and stable staining cannot be guaranteed even in covalently-bonded probes. Slight changes in the ambient conditions, such as temperature, pH, ionic strength and more, greatly affect the measurement results.

- To the problem of instability at low staining levels, such as in immunofluorescence, a noise resulting from autofluorescence is added. This autofluorescence is due primarily to the presence of pyridine and flavin nucleotides (Aubin, 1979; Benson et al., 1979) and limits the sensitivity of immunofluorescence measurements.

- Fluorescence signal measurement standardization. This problem is so bothersome that a special issue of the "Cytometry" journal was entirely devoted to this subject (Cytometry, October 1998).

- Fading, leakage/release of fluorescent marker from cell and interference with normal cell functions, are well known drawbacks associated with fluorescence measurements.

The ITICBP devices addresses the above drawbacks by utilising features of the individual transparent wells that, while hosting cells, allows the measurement of the cell large angle scattering pattern. Consequently, a complementary static and dynamic visible light scattering technique, which does not require special staining preparation procedures and is non-intrusive, can be performed (Schiffer et al., 1999). Furthermore, the fact that in scattering light measurements a non intrusive light energy (frequency) is used, enables very long duration monitoring (hours to days), much longer than is possible in fluorescence techniques. This, in combination with the unique properties of the TCC, opens vast options for research and development (R & D) of long term experimental biology, performed on the same individual cells and related upspring.

## B2 Differential Light Scattering (DLS)

Finding the structural parameters of living cells is becoming a central tool in the field of cell research and diagnostics. In a process such as fertility of both male and female (Pasteur et al., 1988; Schiffer et al., 1996), abnormal cell structure correlates with potential dis-functioning. In cell growth and programmed death (apoptosis), cell size is correlated with apoptotic stage, which may serve in clinical research (Soini et al., 1997). Presently, structural parameters are mainly being measured using flow-through and video image analysis methods:

- In flow-cytometer (FC) systems, cell structure is sampled by directing a jet of physiological liquid containing cells into a laser beam. The light scattered by the cell may be collected in the forward and side directions. The forward intensity is believed to correlate with cell volume (Sloot et al., 1988), whereas side scatter expresses the internal granularity of the cell. However, in practical use, these parameters turn out to be elusive in many cases, due to reasons such as optical variability (Segel et al., 1981), transition of cellular water content which is misinterpreted (Sloot et al., 1988), and the presence of fluorescent markers which change the absorbing quality of the cell and therefore the forward scatter intensity (Shapiro, 1985)..

- In image analysis (IA), digital analysis of a cell is performed using a light microscope. This demands an accurate positioning of the object plane in order to create a stable focused image of the cell on the CCD detector surface. Practically, this can not be achieved by cameras commonly used in microscopes. Even the slightest offset on a micronic scale may result in a relatively large deviation in cell size measurements (Moruzzi et al., 1988). Moreover, a number of frames are used in video image analysis, each containing dozens of cells. In this way, morphological information is obtained regarding the cell bulk, and there is no trace for the behavior of the individual cells. Thus, it is not possible to define heterogeneity within cell preparation.

In order to evaluate the discrimination power as a function of the relative diffractive index, the forward scatter signals of polystyrene beads having diameter of 6, 7, and 9 $\mu$m suspended in water and in sucrose solution were measured in the FACS. The results shown in Figure 101M indicate two major problems when increasing the refractive index: (a) discrimination power significantly decreases (the three groups of beads could not be resolved when suspended in sucrose), and (b) misinterpretation of the object volume (decreases).

The use of ITICBP device in applying differential scattering method for the quantitation of structural changes in individual cells in populations, as presented here, addresses these problems, thereby making differential scattering a most practical method for morphological measurement, both in the static and dynamic aspects of a living cell. For the illustration of the advantages of morphological quantitation of micron objects, a comparison was made regarding the resolution in size measurements following suspension of the investigated objects in solutions having refraction index close to that of the objects themselves. DLS measurement results show remarkable stability and accuracy in relation to the two other methods.

Monitoring of structural and morphological parameters of individual living cells utilizing the ITICBP device is based on the analysis of each of the cells large angle diffraction pattern.

When a collimated light beam is directed towards a cell, a scattering pattern is created, which virtually contains the cell's structural characteristics. These may be extracted when applying a straightforward analytical procedure. The first advantage of this method is the fact that the collimated beam requires no specific plane for the object, thus no focusing problems should arise. The second is that each cell is individually illuminated so that each cell behavior may be recorded. Also, the detecting CCD camera surface array may be located at any point beneath the object plane. Contrast is always sufficient, since no light is ever scattered when no object is present within the beam zone.

Basically, the scattering object may be described as a two dimensional Fourier transform of the optical profile of the scatterer.

$$(1) \qquad E(\theta,\phi) = \beta \iint_S \left[ 1 - e^{\alpha(x,y)} \right] e^{iKx\theta} e^{iky\phi} dxdy$$

where $\alpha(x,y) = D(x,y)[R_e\Delta u(x,y) + iI_m\Delta u(x,y)]$, $\Delta u(x,y)$ is the complex refractive index of the object and $D(x,y)$ is its width at any given point. $\phi$ and $\theta$ are scattering angles. When the object dimensions are considerably greater than the illuminating wavelength (i.e. live cell illuminated by visible light) and the difference between the suspending media refractive index is small such as in live cells suspended in physiological solution, Eq. 1 may be written as:

$$(2) \qquad E(\theta,\phi) = \beta \iint_s \left[ 1 - e^{iD(x,y)\Delta u} \right] e^{iKxD} e^{iy\phi} dxdy$$

This integral suggests that a scattering pattern is reasonably described as the result of phase shifts in the wave front of the illuminating beam. These phase shifts break the otherwise unidirectional wave front into an angular distribution of rays which is expressed on a distant screen as the scattering diffraction pattern of the object.

- It is possible to improve the existing resolution by illuminating the object with two wavelength simultaneously, thus measuring the cellular parameters using double illumination.

### B3. The Integration of Biosensor and Electrochemical-Based Reactions Technology into the ITICBP Device

A biosensor is an analytical device that uses biologically sensitive material to detect biological or chemical species directly, without the need for complex sample processing. It is usually made by attaching a biologically-sensitive material to a suitable transducing system, which converts the biochemical response into a quantifiable and processable electric signal.

The biologically-sensitive material can be: an enzyme, an antibody (Ab) or an antigen (Ag), a nucleic acid, a receptor or ligand, a peptide, etc. These materials are responsible for the recognition of the test mixture and provide the selectivity and sensitivity of the final device. In very simple terms, the molecular recognition is achieved by the "'lock and key" principle of the respective receptor area and the biological component (or analyte) to be recognized. When biological molecules interact specifically there is a change in one or more physico-chemical parameters associated with the interaction. This change may produce ions, electrons, heat, mass or light. These quantities which are converted into electrical signals by the transducer, are amplified, processed and displayed in a suitable form.

**(a) Bioelectrochemical sensors:** combine the selectivity of biological recognition with the high sensitivity and relative simplicity of modern electroanalytical techniques. They consist of a biologically sensitive material (an enzyme, antibody or antigen, nucleic acid, receptor or ligand, peptide, etc.) attached to an electrode, which converts the biochemical response into a quantifiable electric signal.

**(b) Immune-reactivity determination by means of immunosensors:** The ITICBP device immunosensors are based on an enzyme channeling mechanism, which in a non-limiting way is explained hereunder: An electrode covered with immobilized antibody is incubated with the sample, and the target antigen is selectively captured at the surface. The identification and quantification are accomplished using an enzyme labeled antigen in a competition assay or with a second antibody labeled with an enzyme in a sandwich assay. The sensitivity is high because the electrode senses the high local concentration of the product that is produced by the surface enzyme layer, rather than the concentration of the product in the bulk solution.

**(c) Electrochemical-Based Reactions:** Electrochemical-based reactions are well established in the characterization of bioactive surfaces. Amperometric and potentiometric enzyme electrodes are known and have been commercialized and their use in diagnostic devices has been an especially active research area in the past few years (Weetall, 1976; Weetall, 1993). Electrochemical techniques can be further exploited in the investigation of structure function relationships in thin films on electrode surfaces. For example, impedance measurements and chronoamperometry reveal information on the dielectric constant and transport properties of thin films on electrodes.

**(d) A modified electrode for monitoring nitric oxide in cancer cells.**

A phthalocyanine modified electrode, capable of measuring nanomolar concentrations of nitric oxide was applied in monitoring of NO released by cancer cells after their exposure to activators or inhibitors (Raveh et al., 1997). Recently, a biosensor for the detection of gene expression in-situ has also been developed. Genetically engineered cells with the reporter gene Lac Z were used for on line monitoring of the cell response to heavy metals. In the presence of Cd or Hg ions these cells start to produce the enzyme β-galactosidase and the enzyme activity inside the cells was followed electrochemically (Biran et al., 1998).

**B4. Estimation of intracellular enzymatic reaction carried out in a single individual cell**

An enzymatic activity is usually characterized by two parameters: $V_{MAX}$- the maximum enzyme production rate (velocity) of a product (P) out of a substrate (S) at a saturation concentration of the latter, and $K_M$ - the Michaelis - Menten constant, which is reciprocally proportional to the enzyme affinity to the substrate.
The relation between $V_{MAX}$ , $K_M$, the substrate concentration [S] and the initial velocity V, at which S converts to P, is given by the Michaelis - Menten equation:

$$V = \frac{[S] \cdot V_{MAX}}{K_M + [S]}$$

Unfortunately Eq. 1 is accurate only for a homogeneous medium in which the following processes occur:

$$[S] + [E] \leftrightarrow [ES] \text{ and } [ES] \rightarrow [P] + [E]$$

where [E] and] *ES*] are the enzyme and the complex enzyme - substrate concentrations, correspondingly.
The determination of $K_M$ and $V_{MAX}$, utilizing Eq. 1 calls for sequential exposures and repeatable measurements of the same individual cell for various values of [*S*].
Unfortunately this requirement can not be achieved by the common cytometers: The Flow Cytometer (FC) as well as the Laser Scanning Cytometer (LSC). The FC enables the rapid measurement of the fluorescence intensity (FI) of a large cell population. However because each cell in the flow is measured only once, the kinetic curves of the FC provide sequential measurements of single cells over time but not of the same single cell. See for example:

1. Dolbcare F, Fluorescent staining of enzymes for flow cytometry, Methods Cell Biol 33:81-88, 1990
2. Klingel S, Rothe G, Kellerman W, Valet G, Flow cytometric determination of serine proteinase activities in living cells with rhodamine 110 substrates, Methods Cell Biol 41:449-460, 1994
3. Malin-Berdel J, Valet G, Flow cytometric determination of esterase and phosphatase activities and kinetics in hematopoietic cells with fluorogenic substrates, Cytometry 1:222-228, 1980
4. Nooter K, Herweijer H, Jonker RR, van den Engh GJ, On-line flow cytometry. A versatile method for kinetic measurement, Methods Cell Biol 41:509-526, 1994
5. Turck JJ, Robinson JP, Leucine aminopeptidase activity by flow cytometry, Methods Cell Biol 41:461-468, 1994
6. Watson JV, Dive C, Enzyme kinetics, Methods Cell Biol 41:469-508, 1994

Therefore, investigating different enzyme activities in different cell types or in subcellular areas using the FC gives only an average $K_M$ value for a population of cells or for specific enzymes in a cell-free system.
The LSC measures the fluorescence kinetic of individual cells under specific conditions of low cell density in the selected field and of cell types and dyes which do not suffer from fading, which disrupts the measurement [Watson JV and Dive C. Enzyme kinetics. Methods Cell Biol (1994) 41:469-508]. The LSC technique cannot ensure the accurate rescanning of the same cell after repeatable staining procedures since the cell may not have preserve its original location. Moreover, the LSC cannot ensure preservation of the cell locations and thus cell identification might be lost during repeatable rinsing and exposure to different substrate concentrations.

In order to provide the capabilities for kinetic measurement of individual cells under repeatable staining conditions, a specially designed cytometer was used. The cytometer (hereinafter referred to as Cellscan Mark S or CS-S) which, one of its versions, was described in the US Patents 4,729,949, 5,272,081, 5,310,674 and 5,506,141 found to be applicable for measuring time resolved kinetics of individual cells during cellular manipulation.

Using the unique application of the CS-S, a new method was developed in which the same cells are sequentially exposed to increasing substrate concentrations. The product formation rate is measured for each cell at every substrate concentration yielding a series of rates for the same individual cell. Using this data, $V_{MAX}$ and apparent $K_{MAPP}$ (app = apparent) values can be calculated for each cell, giving the distribution of $K_{MAPP}$ and $V_{MAX}$ of the measured population However, it should be emphasized that the process of present invention is not limited to the CS-S cytometer and any cytometer comprising a microscope, light detection means , a carrier to which cells are individually located, is within the scope of the present invention.

**Kinetic Analysis**

The kinetic parameters are derived by application of linear and nonlinear modeling. The linear model y(t=(At+B seeks parameters A and B which fit the data to a straight line equation, where y(t) is the measured quantity, t is the time, and A and B are the calculated parameters. The CS-S algorithm uses $\chi^2$ as the criteria for goodness-of-fit.

**(a) Single Step Cell Staining**

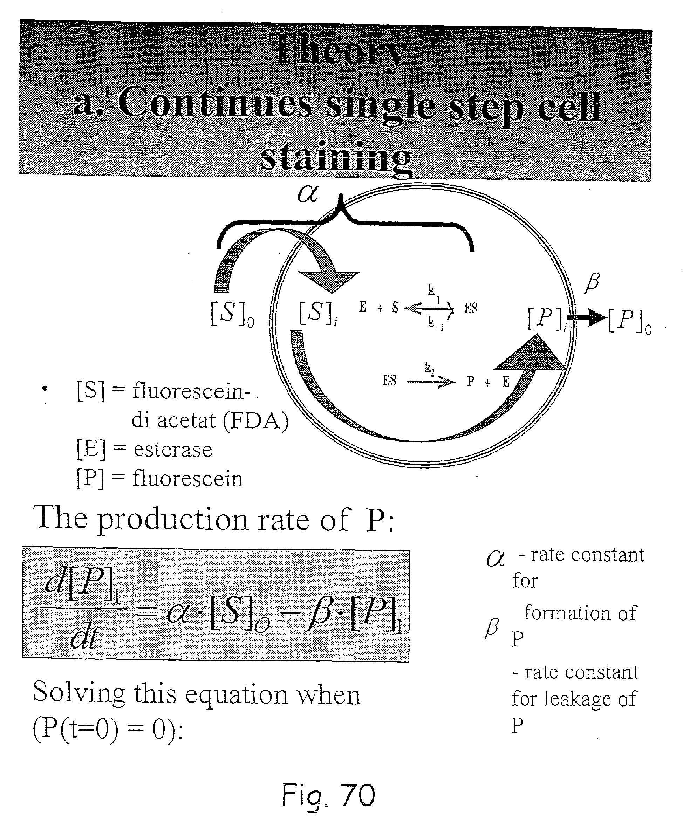

A simplified model for the description of intracellular turnover of fluorogenic substrate is presented in FIG. 70. First, the extracellular substrate [S]o permeates into the cell, becoming [S]i - the intracellular substrate concentration. Then [S]i is hydrolyzed or cleaved by enzymes to yield the intracellular (for example, fluorescent) product [P]i, which may be released from the cell into the medium and become $[P]_O$.

As was previously shown [Bedner E, Melamed MR, Darzynkiewicz Z, Enzyme kinetic reactions and fluorochrome uptake rates measured in individual cells by laser scanning cytometry, Cytometry 33:1-9, 1998] the kinetics of [P]i can be described, to a good approximation, by the rate equation :

$$\frac{d[P]_i}{dt} = \alpha \cdot [S]_O - \beta \cdot [P]_i \quad (2)$$

Where $\alpha$ and $\beta$ are the rates constants for the formation and leakage of the intracellular fluorescein. It is important to emphasize that $\alpha$ represents two processes: Permeation of S and its intracellular distribution as well as the enzymatic hydrolysis of $[S]_i$.

When solving Eq. 2, under the initial condition of one step staining] ,$P$(t=0)]l = 0 it is easily shown that

$$P(t) = \frac{\alpha}{\beta}[S]_O (1 - e^{-\beta \cdot t})$$

$$(3)$$

**(b) Sequential staining**

Another aspect of present invention relates to sequential exposures of the same individual cells to different substrate concentrations. This differs from the above case by the fact that at the starting time point of staining, with a given solution, cells are already being stained to a level of:

$$\left[P(\tau)\right]_i = \frac{\alpha}{\beta}M\left[S\right](1-e^{-\beta\tau}) \tag{4}$$

$\tau$ stands for the time point of terminating the staining with a given substrate concentration, say $M$ times $[S]$ ($M[S]$), and initiation of staining with different substrate concentration, say $N[S]$.

Now, it is possible to solve Eq.2 under the initial conditions presented by Eq. 4. By separation of variables and integration over] $P]_i$ between the concentration limits $[P(\tau)]_i$ and $[P(t)]_i$; and integration over time between the time points 0 (when staining solutions are being replaced) and t, one gets:

(5)

$$\int_{[F(\tau)]_I}^{[F(t)]_I} \frac{d[F]_I}{[F]_I - \frac{\alpha}{\beta}N[S]} = \int_0^t -\beta dt \Rightarrow \ell n\left(\frac{[F(t)]_I - \frac{\alpha}{\beta}N[S]}{[F(\tau)]_I - \frac{\alpha}{\beta}N[S]}\right) = -\beta \cdot t$$

Converting the logarithmic expression into exponential one and introducing $[F)\tau)]_I$ of Eq.4 into Eq. 5 yields:

$$\left[F(t)\right]_I = \frac{\alpha}{\beta}M\left[S\right](1-e^{-\beta\tau})e^{-\beta t} + \frac{\alpha}{\beta}N[S](1-e^{-\beta t})$$

When single step staining is performed (starting of unstained cell, $M$=0), only the last term of Eq. 6 remains, which is consistent with Eq. 3.

As long as the expression exp-)$\beta$t) $\cong$ 1-$\beta$t holds for the duration of the observation interval of the individual cells in given conditions, regardless of their staining history, each of the exponential terms in Eq. 6 can be replaced, without losing accuracy, by its first two terms of the power series. Hence, Eq. 6 may be linearly approximated to give:

(7)

$$[F(t)]_I = \left\{ \begin{array}{l} 0 < t < \tau \\ t > \tau \end{array} \right. \rightarrow \begin{array}{l} \alpha[S]Mt \\ \alpha[S]\cdot(M\tau + Nt) \end{array} \rightarrow \left\{ \begin{array}{l} \dfrac{d[F(t)]_I}{dt} = \alpha[S]M \\ \dfrac{d[F(t)]_I}{dt} = \alpha[S]N \end{array} \right\}$$

Eq. 7 should be interpreted as follows: for 0< t<$\tau$, staining proceeds according to $[P(t)]_i = \alpha[S]Mt$. After replacing the staining solution $M$ by $N$ at time t=$\tau$, the staining due to $M[S]$ remain constant $[P(\tau)]_i = \alpha[S]M\tau$, While that due to $N$ increases at a rate of $\alpha[S]N$, namely solely depending on the concentration in use.

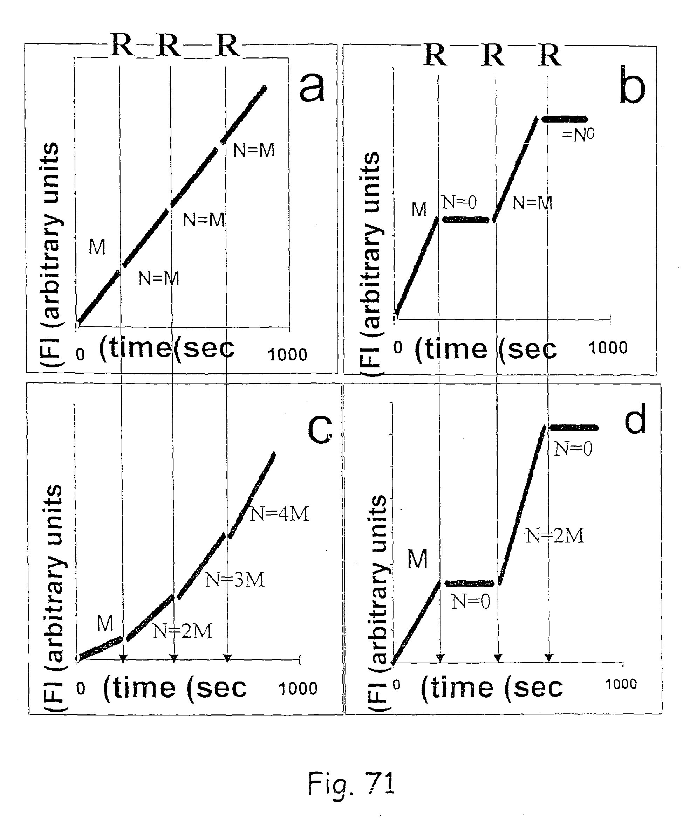

Simulations of several practical staining protocols, based on Eq.7, are graphically presented in Fig. 71 and briefly described in the following

a) Rinsing the cells with a staining solution [$N$] that maintains [$N$] = [$M$], results in a staining curve $[P(t)]_l=\alpha[S]N[\tau+t]$. At the observation time $\tau+t$ [$P$]$_i$ had a production rate of $\alpha[S]N$, the same rate as that of $\alpha[S]M$ prior to $\tau+t$ (Fig. 71a).

b) Rinsing the cells with PBS alone washed away] $M$] residues leaving the staining solution at a concentration [$M$] = 0. This action halted any further production [P]$_l$ (since $\alpha[S]N$ = 0 at the time of application $\iota$) hence [P]$_i$ line remained parallel to the time axis for the duration of the observation t. (Fig. 71b).

c) In a similar way, the cells were rinsed with a staining solution] $N$ $\neq$[$M$] that washed away [$M$] and left the staining solution at a concentration [$N$]. The production rate of [P]$_i$, as expected, changed to $\alpha$ $N$ [$S$] for the observation duration t. (Fig. 71c)

d) The last experiment, was a combination of b) and c) in succession. First the cells were rinsed at time $t_1$ with PBS and that halted the production of $F_l$. The next stage was to rinse with a staining solution [$N$] $\neq$ [$M$] replacing the PBS with a solution of concentration [$N$]. The production rate then changed to $\alpha[S]N$ for the for the observation duration t. (Fig. 71d).

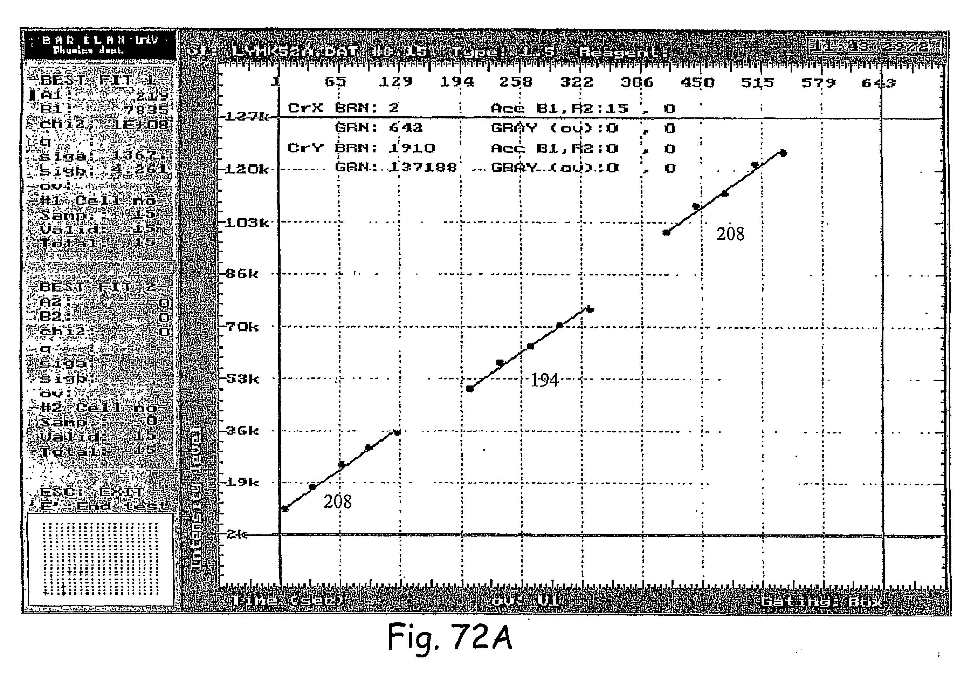

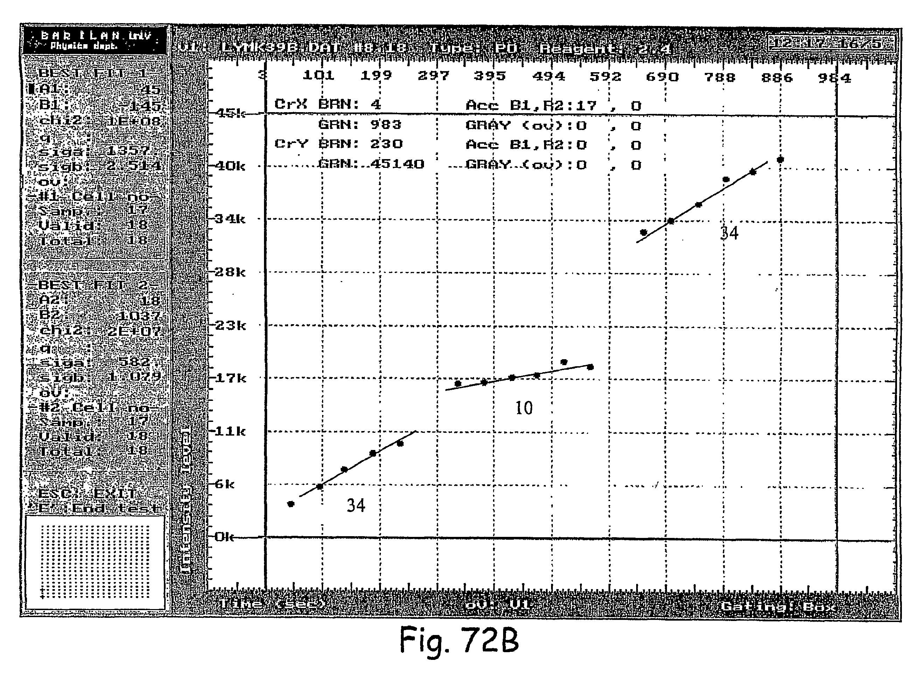

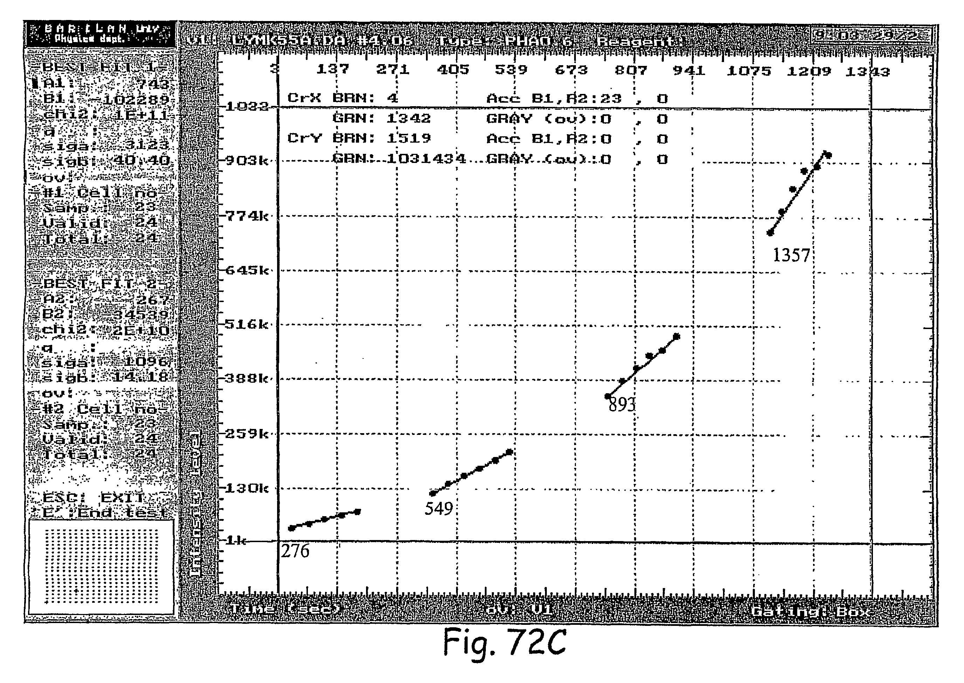

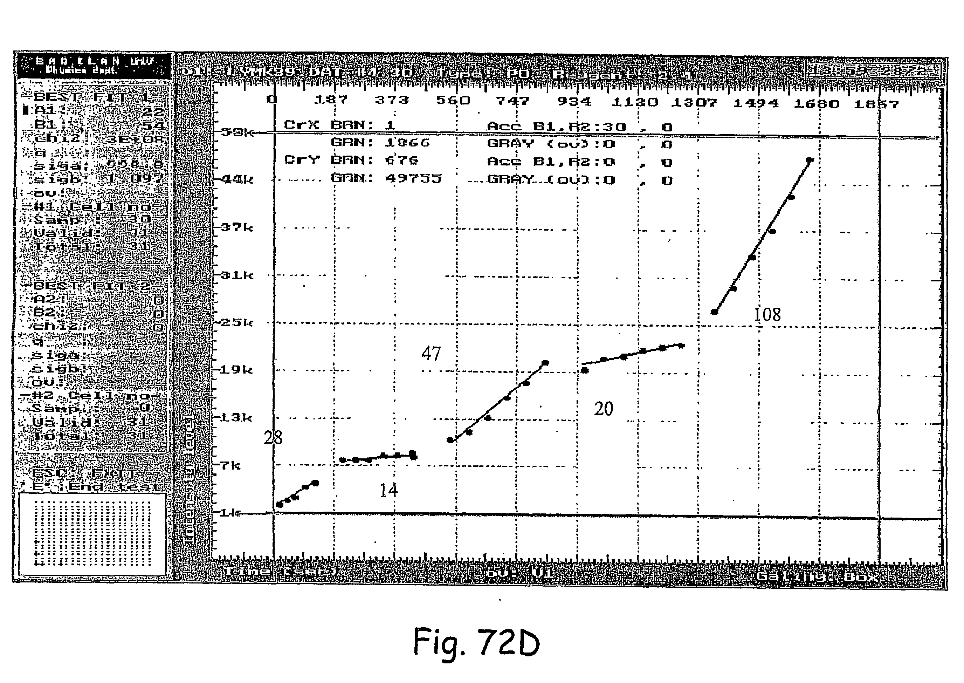

Figure 72 shows real experimental results as carried out exactly following the above mentioned simulation experiments.

Finally, the determination of $\Delta t$, the overall sequential staining experiment procedure time duration, was restricted to follow the present CS-S Standard deviation in performing individual cell FI measurements, which is < 2%.

In order not to exceed this value when linearly approximating the exponential terms, a $\Delta t$ value which keeps the ratio

$$\exp(-\beta\Delta t)/(1-\beta\Delta t) \cong 2\%$$

is sought. Hence, introducing $\beta \cong 10^{-4}$ sec$^{-1}$, which is the outcome of many hundreds of independent experiments (data not shown), yields $\Delta t \cong 10^3$ sec.

The following example is provided merely to illustrate the procedure and is not intended to limit the scope of the process and application in any manner.

**EXAMPLE:** Measuring intracellular nonspecific esterase activity in a single lymphocyte using fluorescein-diacetate (FDA) as the substrate.

## (a) Materials and Methods:

Phytohemagglutinin PHA) *HA15, Murex Biotech*) was reconstituted in 5 ml of double-distilled water and further diluted ten times. For stimulation, 10$\mu$l of this solution was added to a 90 $\mu$l cell suspension (7x10$^6$ cells/ml).

The culture medium consisted of RPMI-1640 (Biological Industries), supplemented with 10% (v/v) heat-inactivated fetal calf serum (Biological Industries), 2mM L-glutamine, 10 mM Hepes buffer solution, 1mM sodium pyruvate, 50 U/ml penicillin and 50Units/ml streptomycin.

A staining solution of 3.6 $\mu$M FDA (Riedel-de Haen Ag. Seelze-Hanover) in Dulbecco Phosphate Buffered Saline (PBS, Biological Industries) was prepared as follows: 50 mg of FDA was dissolved in 5 ml of DMSO (Sigma). 7.5 $\mu$l of this solution was added to 50 ml PBS. For 0.6, 1.2 and 2.4$\mu$M the solution was further diluted in PBS.

## (b) Preparation of Peripheral Blood Mononuclear Cells (PBMC:

30 Heparinized blood (30 ml), was taken from healthy, normal volunteers. The procedure for separating the PBMC has been described in detail, elsewhere [Sunray M, Deutsch M, Kaufman M, Tirosh R, Weinreb A, and Rachmani H. Cell Activation influences cell staining kinetics, Spectrochimica Acta A (1997) 53:1645-1653]. Shortly after removing the iron absorbing cells, the remaining cells are layered on a two-layer (100% and 80%) cell density gradient (Ficoll Paque, Pharmacia 1.077 g/ml) and centrifuged. The cells accumulated at the interface between the two Ficoll layers, were collected and kept at 37° C in 5 ml of enriched culture medium overnight. The next day the PBMC were washed and resuspended in PBS at a final concentration of 7·10$^6$ cells/ml. More than 70% of the cells were defined as T lymphocytes and viability, which was determined using eosin, was always higher than 90%.

**(c) Activation of PBMC by PHA:**

Freshly prepared PBMC ($7 \cdot 10^6$ cells/ml) were incubated at 37˚C, 5% CO2 with 5$\mu$ gr/ml PHA for 30 minutes. PBMC controls were incubated without PHA under identical conditions.

Cells were irradiated with 1-10 $\mu$W of 442 nm light from a He-Cd laser. Under the staining conditions used here, the scanning time for obtaining a count of 10,000 photons in order to have statistical photonic error of ~1% from each, dye-loaded cell varied from 0.001 sec to approximately 0.5 sec.

The acquired data, including cell position, measurement duration for each cell, absolute time, intensity at two different wavelengths, computed fluorescence polarization values and test set-up information, are displayed on the screen, on-line, graphically and numerically, and stored in the memory. Software enables the determination of the range and other statistical characteristics of all parameters, for either the entire cell population, or an operator-selected sub-population, or an individual cell, before, or during the scan.

**(d) Cell Loading:**

Loading the cells in wells was carried out, as described in Deutsch M, and Weinreb A., Apparatus for High Precision Repetitive Sequential Optical Measurement of Living Cells, Cytometry (1994) 16: 214-226. An aliquot of 80 $\mu$l of unstained cell suspension ($7 \times 106$ cells/ml) was loaded on the CC. Initial scanning was then performed in order to detect individual cell background scattering and auto-fluorescence. This undesired signal is recorded per measurement location and subtracted from the total emission signal (after exposure) in order to obtain the correct fluorescence signal.

**(e) Cell Staining and Kinetic Measurement:**

For fluorescence intensity FI(t) measurements, trapped cells on the CC were sequentially exposed to increasing concentrations of FDA in PBS staining solutions.

Following background measurement, the volume of PBS, which covers the cells, was pumped out and the following procedure was carried out:

At time point zero, 40 $\mu$l of the lowest substrate concentration solution was applied on top of the trapped cells and a pre-chosen cell field was sequentially scanned 6 times. This yielded 6 accurately timed FI data points per each individual cell at a given dye concentration. FI is usually measured utilizing epi-fluoescence optical arrangement which permits the differentiation between the excitation energy and the emitted fluorescence energy to be detected by photomultipliers, CCD detectors etc.

The above procedure is repeated for each different substrate solution used in the experiment.

This yielded six FI data points for each individual cell, per substrate concentration, from which V was extracted and the individual cell $K_{MAPP}$ and $V_{MAX}$ values were calculated. The dead time, i.e., the elapsed time from the addition of a staining solution to the beginning of the measurement, which is monitored by the computer, is about 7-15 sec.

**B5. Correlation between fluorescence and morphometric measurements and image analysis**

This section focuses on the following unique analytical capabilities of the ITICBP device's technology which complements those of flow cytometry and fluorescence image analysis:

a) the individual cells are positioned within the ITICBP identified wells during measurement so they may be examined repeatedly over time, a feature useful for studies of enzyme kinetics and other time-resolved processes;

b) sequential analysis of the same cells can be carried out using different immuno- or cytochemical stains or genetic probes, merging information on cell immunophenotype, cell functions, expression of particular proteins, DNA-ploidy and cell cycle position, and/or cytogenetic profile for each measured cell;

c) any of the cells measured can be relocated to correlate with visual examination by fluorescence or bright field microscopy or with any other parameter;

d) topographic distribution of fluorescence measurements within the cell, in cytoplasm vs. nucleus, permits analysis of the translocation of regulatory molecules such as $NF_KB$, p53, etc., and is essential for FISH analysis;

e) hyper-chromicity of nuclear DNA as measured by maximal pixel fluorescence intensity allows one to identify cell types differing in degree of chromatin condensation such as mitotic or apoptotic cells;

f) analysis of tissue section architecture and of the constituents in transected cells within tissue sections by ratiometric assays normalized to DNA content extends applications of ITICBP technology in clinical pathology;

g) because cell loss during sample preparation and staining is minimal, samples with a paucity of cells can be analyzed;

h) analyzed cells can be stored indefinitely, e.g., for archival preservation or additional analysis.

## B6. Multiparametric optical and biochemical measurements

Correlated measurements of several parameters enables monitoring of morphological and biochemical changes that may correlate to functional parameters. This approach provides an answer to the need for non interfering quantitative measurements of multiparametric changes which accompany complexed biological processes such as: apoptosis, cell cycle, cell growth and cell differentiation.

## B7. Photobiostimulation

Biostimulatory effects of low-output laser irradiation have been demonstrated at a variety of molecular and cellular levels, as well as at whole organ and tissue levels. Under certain circumstances, synergistic effects with laser irradiation have been found as demonstrated in the immune system. Evidence exists that effects occur, remote from the irradiated site, suggesting the presence of a circulatory active substance. With sufficient intensity, the stimulatory effect disappears and inhibition occurs.

## B8. Photoactivation

Flash photolysis of photoactivatable or "caged" probes provides a means of controlling the release of biologically active molecules. Since the caging moiety is designed to interfere with the binding or activity of the molecule, uncaging by photoactivation which takes microseconds to milliseconds, results in a pulse of the active product. Uncaging can be accomplished by illuminating with a laser beam. Photoactivation of caged ions, drugs or neurotransmitters, rapidly initiate or block cellular activity, or neurotransmitter action, thus providing tools for kinetic studies of receptor binding, channel opening and cellular activation.

In addition, photoactivation of caged molecules, which are essentially nonfluorescent enables the monitoring of the dynamic behavior of cytoskeletal elements, and the study of the hydrodynamic properties of the cytoplasmic matrix and lateral diffusion in membranes.

## B9. Manipulation and modulation of controlled biological processes:

Detection and selection of specific cells by their binding characteristics or metabolic behavior which might be induced by incorporation of biologically active molecules (antibodies, antigens, drugs) onto the surface of TCC's wells in the ITICBP device, and by manipulating the physiological conditions (buffers, ions, osmolarity, active molecules).

This measuring procedure is highly useful in a series of applications - among them are:

a) Selection of antibody secreting hybridoma cells.
b) Monitoring fusion of cells.
c) Cell classification.

## B10. Dynamic monitoring (time) of cellular phenomena

ITICBP device is applicable in detecting and quantifying structural variations correlated with functional characteristics during normal and abnormal differentiation, growth, aging, and behavior of specific individual cells. This is carried out by measuring structural parameters such as cell size, cell shape, cell configuration, intracellular movement, cytoplasmic fluidity or microviscosity and by means of fluorescence intensity and polarization and correlated with chromatic and IA data, as a complimentary step.

## B11. Direct visualization and activation of specific ligand binding T cells with fluorescent TCR antigen ligands - Immunodiagnosis

Recent molecular identification of tumor antigens recognized by T cells derived from cancer patients has initiated a new era in tumor immunology (Coolie, 1997). So far, nearly all of the defined tumor antigens known to simulate Cytotoxic T lymphocytes (CTL) responses consist of a short antigenic peptide associated non-covalently with the MHC class I molecule. These complexes, which are displayed on the surface of the tumor cell, are the ligands for specific, clonally distributed, T-cell receptors (TCRS) on the surface of CD8+ CTLs.

The identification of these antigenic peptides has opened new possibilities. The first is based on the potential of synthetic peptides to mimic endogenously produced antigenic peptides. The second and more interesting avenue is based on the potential use of synthetic peptides as cancer vaccines. In this regard, synthetic peptides alone or in combination with an adjuvant, might elicit potent tumor-specific CTL responses by repeated immunization of cancer patients.

This new strategy for experimental immunotherapy of cancer has generated the need for large-scale monitoring of tumor-antigen-specific CTLS. In fact, by analogy with classical vaccination, it is now essential to develop standard assays to accurately assess the impact of vaccination on the levels of responding T cells. It is currently accepted that the frequency of T cells specific for single MHC-peptide complexes in unprimed circulating lymphocyte populations may be very

## Chapter C:

### The Scope of Biological, Biochemical and Toxicological Studies Provided by the ITICBP Device

By large, the introduction of the high content simultaneous screening and non-biologically disturbing ITICBP replaces, or at least adds, another layer of screening format to extract relevant information from cells. ITICBP is designed to capture complex cellular activities like:

- Morphology changes
- Differentiation
- Locomotion
- Apoptosis
- Adhesion
- Translocations of signaling molecules
- Protein trafficking

### C1. Use of ITICBP Device as a Model for Studying Interactive Reactions of an Individual Cell with (Surface-Bound) Biologically Active Materials

A distinguished feature of the ITICBP device based on its application in a wide scope of biological studies in which an individual cell interactively responds to the presence of a surrounding biologically active material. Two major interactive studies are of great interest:

### (a) Interactive Reactions with Surface- Bound Biologically Active Materials

Immobilization techniques of materials on a dielectric surface are well known-among them are: adsorption, covalent binding, biotin-avidin bridges and others. Consequently, studies of surface-bound biologically active materials (such as enzymes, drugs, antigens, antibodies, receptors, etc') acting as activators/inhibitors/regulators/sensors of examined individual cell's functions, can be accomplished using ITICBP. For example, drugs, or specific antigens immobilized onto an ITICBP's aperture surface can be used to activate or to restrain the T-lymphocytes' cellular function. Similarly, T-cell receptor specific antigen, immobilized onto the aperture surface can be utilized for activation of specific ligand binding T-cells. This is of a great importance in the field of tumor immunology, where antigenic tumor specific T-lymphocytes are used for experimental immunotherapy of cancer.

### (b) Interactive Reactions with Photoactivatable Materials

Flash photolysis of photoactivatable ("caged") molecules provides a means for controlling the release of free (in solution), or surface-bound, biologically active materials. Photoactivation ("uncaging") can easily be accomplished with a laser (or UV) illumination of the ITICBP's aperture. The cell-interactive effect of photolytic release can be monitored either by using fluorescent probes or using electrodes embedded within the ITICBP's aperture. Consequently, photoactivation of caged molecules (drugs, antibodies, antigens etc.') rapidly initiate/inhibit the activity of the examined individual cell, thus providing tools for kinetic studies of the interactive relationships between photoactivatable material and the examined individual cell.

## C2. Use of ITICBP Device as a Model for Studying the Physiological Status of an Individual Cell Based on Simultaneous Measurement of Intra- and Extracellular Concentrations of Highly Reactive Materials (Free Radicals).

Simultaneous extra- and intra-cellular monitoring of highly reactive substances (frequently referred to as "free radicals") by determination of NO, superoxide ($O_2$-) and Glutamate levels in cells and outside of the cells.

The simultaneous, non-disturbing, real-time determination of NO and $O_2$- levels in cells is extremely important since the balance between NO and $O_2$- production participates in the control of major cell functions including reactivity, proliferation and apoptosis. Inappropriate production of these radicals and of their metabolite, leads to the development of various pathologies, up till now mostly studied on animal models. These pathologies include cardiovascular dysfunction, atherosclerosis, ischemia and neurovegetative diseases. Given the coexistence of beneficial and detrimental effects for free radicals in cells, and the intricacy of their metabolic pathways, it is crucial to be capable of simultaneous direct determination of their concentrations in cell population as a response to chemicals of biomedical relevance. There is therefore a clear demand for a potent and selective analytical device, enabling simultaneous monitoring of NO and $O_2$-. ITICBP device of present invention, is able to determine these concentrations on cultured cells, and this constitutes a major progress since it provides an alternative to the studies on whole animals and greatly complements research done with isolated organs. Glutamate is included in the scope of studies in view of being an extremely important analyte in neuronal cell metabolism, and in epithelial cell homeostasis and maintenance of normal barrier and transport functions in cells. The following is the procedure for determination of NO, $O_2$- and glutamate, produced by an individual cell (or group of cells) using ITICBP device:

- Extra-cellular NO, $O_2$-, and glutamate are determined electrochemically utilising modified microelectrodes in arrays (Barker, et al., 1998).
  Each well is fabricated with at least three microelectrodes, each coated with a "sensing chemistry" to tune specificity for these three important cellular reactants.

- Intracellular assaying of oxidative activity is carried out by determining levels of NO and $O_2$- with fluorescence probes using fluorescence intensity (FI), polarization (FP), fluorescence lifetime (FLT) and fluorescence polarization decay (FPD).

For NO the dye 4,5 diaminofluorescin diacetate. (DAF-2DA) is used. This is a cell permeable probe that is hydrolyzed by cytosolic esterases to DAF-2. At physiological pH (6.5-7.4), DAF-2 is relatively non-fluorescent but in the presence of NO and oxygen a fluorescent product, DAF-2 triazole (DAF-2T), is formed. The conversion of DAF-2 to DAF-2T accompanied changes in the spectroscopic characteristics, which result in changes in FI, FP and FLT. DAF-2 is specific to NO since it does not react neither with a stable oxidized form of NO such as $NO_2$ and $NO_3$, nor with superoxide or hydrogen peroxide. 4 amino fluorescein diacetate (4A FDA) can be used as a negative control compound.

For the intracellular detection of superoxide the "dihydro" derivatives (leuco-dyes) of fluorescein, rhodamine and ethidium can be used. These colorless reduced forms non-fluorescent leuco dyes are readily oxidized back to the parent dye and thus can serve as fluorogenic probes for detecting oxidative activity in living cells. However, they do not directly detect superoxide, but rather react with hydrogen peroxide in the presence of peroxidase, cytochrome C or $Fe^{2+}$. Nitric oxide reacts with superoxide to yield peroxynitrite which further reacts with dihydrorhodamine 123 to provide fluorescent dye (Kooy, et al., 1994). Intracellular oxidation of dihydrorhodamine 6G yields rhodamine 6G , which localizes in the mitochondria of living cells. This cationic oxidation product has longer-wavelength spectra (red emission) and can be used especially for simultaneous analysis with fluorescein derivatives.

Dihydroethidium was shown to undergo significant oxidation in resting leukocytes, possibly through the uncoupling of mitochondrial oxidative phosphorylation. Cytosolic dihydroethidium exhibits blue fluorescence; however, once this probe is oxidized to ethidium, it intercalates within the cell's DNA, staining its nucleus a bright fluorescent red.

Intracellular NO and superoxide can also be assessed utilizing fluorescent nano-sensors. In such a case several sensor delivery methods are used, including liposomal delivery, gene gun bombardment, and Pico-injection into single living cells.

In summary, ITICBP device is highly useful in assessing a toxicity status of cells, applying a combination of optical and electro-chemical simultaneous, real-time measurements of intra- and extra-cellular levels of free radicals. In other words, the device of present invention creates a basis for a new generation of toxicity testing procedures relying on measurements of concentrations of free radicals within and outside of the examined individual, single cell.

## C3. Screening for Toxicity of Potential (Bio)Medical Substances Without Disturbance of Living Cells

An important aspect of ITICBP platform is that by utilising individually addressing array electrodes (i.e., measuring local concentration of free radicals in the close proximity of individual microelectrode), the toxicity of biomedical substances can be studied without disturbance of in vivo living cells. Moreover, this is carried out and increased spatial resolution, which enables local toxicity testing to be carried on cell models if toxic compounds are locally delivered (e.g., by robotics as well as by utilising toxic-coated ITICBP).

This approach suggests a high throughput screening for detecting toxicity in pharmaceutical materials. It enables monitoring of free radical as well as other mentioned parameters, and it may increase speed of analysis resulting in a user-ready device, assisting in building-up of better diagnostic and therapeutic arsenal for health care.

Simultaneous extra- and intra- cellular monitoring of free radicals using ITICBP device, enables better evaluation of the reasons for discriminating the toxicity of biomedical relevant compounds. As an example, one of NO syntheses inhibitors (N$^w$-monomethyl-L-arginine) resulting in NO inhibition but also generating superoxide by the enzyme which causes to the injury of cells. Similar effects can only be detected by addressing dynamics (i.e., real time monitoring) of free radicals.

In summary, ITICBP device of present invention combines sophisticated, modern technologies together for performing highly advanced measurements *in-vitro* on an individual cell or group of cells, such as cultured cell lines and primary cells. More specifically, the device provides an integrated novel generic technology, of further value to industry, in particular in drug discovery and in high throughput (robotic) screening for biological active materials as well as new pharmaceutical agents.

## C4. Direct visualization and activation of specific ligand binding T cells with fluorescent TCR antigen ligands - Immunodiagnosis

Recent molecular identification of tumor antigens recognized by T cells derived from cancer patients has initiated a new era in tumor immunology (Coulie, 1997). So far, nearly all of the defined tumor antigens known to simulate Cytotoxic T lymphocytes (CTL) responses consist of a short antigenic peptide associated non-covalently with the MHC class I molecule. These complexes, which are displayed on the surface of the tumor cell, are the ligands for specific, clonally distributed, T-cell receptors (TCRS) on the surface of CD8+ CTLs.

The identification of these antigenic peptides has opened new possibilities. The first is based on the potential of synthetic peptides to mimic endogenously produced antigenic peptides. The second and more interesting avenue is based on the potential use of synthetic peptides as cancer vaccines. In this regard, synthetic peptides alone or in combination with an adjuvant, might elicit potent tumor-specific CTL responses by repeated immunization of cancer patients.

This new strategy for experimental immunotherapy of cancer has generated the need for large-scale monitoring of tumor-antigen-specific CTLS. ITICBP device is an ideal tool for performing such large-scale studies. In fact, by analogy with classical vaccination, it is now essential to develop standard assays to accurately assess the impact of vaccination on the levels of responding T cells. It is currently accepted that the frequency of T cells specific for single MHC-peptide complexes in unprimed circulating lymphocyte populations may be very low. Therefore, techniques to measure low frequencies need to be highly sensitive.

The most direct method of assessing specific T cells in a mixture of lymphocytes was recently described (Callan et al., 1998; Altman et al., 1997; Klenerman et al., 1996). The technique is based on the use of a soluble fluorescent form of the TCR ligand. Recent experiments showing that soluble class I MHC-antigen peptide oligomers (mostly tetramers) can be used to detect specific CTLs by flow cytometry, suggest that this approach could be practical (Romero et al., 1998). The procedure is independent of the TCR gene segments used by the antigen-specific lymphocyte. To bypass the low intrinsic affinity of the monomeric ligand for TCR, a multimeric peptide-MHC reagent with increased avidity for T cells was designed. This was accomplished by introducing a gene segment coding for a consensus biotinylation peptide at the C terminus of the MHC class I molecule (HLA-2).

In vitro assembled and biotinylated MHC specific complexes are forced to form tetrameric arrays by addition of fluorescently labeled-avidin molecules. Such reagents bind specific T cells, generating a signal that is 10-fold the background staining. This promising technical advance has recently been applied successfully to the measurement of CD8+ T-cell-mediated immunity to murine lymphochoriomeningitis virus, and is being tested in several other antigen-recognition systems, including tumor-associated antigens.

In accordance to present invention, a fluorescent TCR antigen ligand immobilized onto the well surface of the ITICBP device's TCC for direct visualization and activation of specific ligand binding T cells. This, among other things, allows large-scale monitoring of tumor-antigen-specific CTLS in the fields of experimental immunotherapy of cancer, as well as in the potential use of synthetic peptides as cancer vaccines.

In addition, photoreactive derivatives of the antigenic peptide are used. Provided that the addition of the photoreactive group leaves the MHC- and TCR-binding properties of the peptide intact, this is covalently linked to the multimeric MHC molecule array by flashing UV light at the sample. In this way, dissociation of the antigenic peptide is eliminated.

In the following, an example is given to emphasize the exclusive utilization of the tetramers coated onto the surface of the ITICBP device's wells in one of the leading goals of the present invention: cancer detection and prognosis by means of immunodiagnosis, in view of the pioneer work of the Cerceks.

Cercek and Cercek (Cercek and Cercek, 1977; Cercek and Cercek, 1981 and Cercek et al., 1978) discussed the excitation and emission-polarization spectra of fluorescein in living cells in relation to the application of the phenomenon of changes in the Structuredness of the Cytoplasmic Matrix (SCM) in the diagnosis of malignant disorders.

Briefly, the Cerceks performed the so-called SCM test after first trying to separate a particular sub-group of lymphocytes from other lymphocytes, as well as other types of cells by the density gradient technique. This technique, as previously pointed out (Rahmani et al., 1996; Ron et al.,) has several major drawbacks:

- First, it is very time consuming, as is appreciated by those familiars with the art and as is clearly apparent from the articles by the Cerceks.

- Second, as the Cerceks acknowledge, the finally separated cells do not belong only to the subgroup of interest, but include a large number, on the order of 50%, of other lymphocytes. Thus, the analysis of their response to stimulation of the separated cells may be very limited.

- Third, all the stimulation and response measurements, performed by the Cerceks on the separated cells, are done on all the cells in a batch (suspension), rather than on a cell-by-cell basis. However, it is clear that cell-by-cell analysis provides far more information for the understanding of biological implications of the phenomena under study.

- Fourth, and most significant, stimulation in the SCM-test is performed by incubating the separated cells with soluble tumor derived proteins (TDP), extracted from tumor tissues. This method of cell activation is most insufficient due to the fact that lymphocyte stimulation is best achieved via presentation of a given peptide in association MHC, preferably presented as solid phase.

While the first 3 drawbacks are quite successfully addressed by the Cellscan apparatus and its TCC discussed above (U.S. Patents. No. 5,310,674, 4,729,949, and 5,506), the fourth one is not.

However, in addition to the fact that the present invention and methodology are superior to the Cellscan apparatus and its cell carrier in dealing with the first 3 drawbacks, it exclusively, elegantly and most efficiently addresses the fourth one.

Practically, tetrameric complexes are bound to the surface of transparent wells (made of glass or plastic-polystyrene) of ITICBP device's cell enabling stable binding of proteins and complexes (binding of complexes can be evaluated by means of fluorescent markers and radioisotopes) in order to enhance lymphoid cell activation due to interaction with the bound complexes. For example, extracted/procured cells either from experimental laboratory animals and/or from cancer (melanoma) patients (by melanoma-specific HLA-A2 tetramers).

According to the present invention, the signaling of activation is monitored by a vast spectrum of on-line, repeatable monitoring means, discussed above, such as, electro-reflectance, electrochemical, electro-fluorescence and optical parameters associated with the same individual cells and/or group of cells while being manipulated and visually observed.

### C5. Additional Immunodiagnosis - based applications,

ITICBP device is applicable in a wide-range of immunodiagnostic assays including: detection of viral and bacterial infections and autoimmune diseases by specific activation of donor lymphocytes; individual Mixed Lymphocytes Response (MLR); screening of potential chemotherapeutic agents, drugs and growth factors; testing of viral and bacterial vaccines; allergy tests; analysis of sperm; detection of viruses and other intracellular pathogens; diagnosis of graft rejection in transplantation.

### C6. Application of ITICBP device for monitoring in vivo/in situ gene expression in individual cells

Green fluorescent protein (GFP), originally isolated from the jellyfish Aequorea victoria, has proven to be a useful reporter for monitoring gene expression in vivo and in situ (Little and Mount, 1982; Woodgate and Sedgwick, 1992).

A protein of interest is directly tagged with fluorescent GFP simply by cloning the cDNA of interest into a vector such that a GFP fusion protein is generated upon expression in transfected cells. Alternatively, GFP can be coexpressed as a second transcriptional or translational unit from the same vector expressing the protein of interest. Cells expressing GFP or a GFP-tagged protein are then detected and sorted by FACS or other cytometric analysis. Furthermore, individual cell analysis may also be used to monitor the in vivo activity of different mammalian promoters using GFP as the reporter gene.

Utilizing the GFP reporter gene assay, provides the ability to perform a continuous detecting system, since there is no need to lyse the cells or to destroy the cellular structure for the detection of the fluorescence signal.

Tagging of proteins with the green fluorescent protein (GFP) has enabled the direct visualization of real-time trafficking, expression or activation of specific proteins in living cells. Such analyses have provided crucial insight into the mechanisms involved in controlling various cellular functions.

GFP is a convenient marker for use in flow cytometry because it eliminates the need to incubate with a secondary reagent (such as dyes or antibodies) for detection. The availability of new GFP variants with red-shifted excitation spectra makes fluorescence-activated cell sorting (FACS) analysis more efficient. Furthermore, the new red-shifted GFP variants make double-labeling antibodies. However, GFP-tagged proteins are superior to conjugated antibodies in FACS applications because the cells do not have to be incubated with the fluorescent-tagged reagent and because there is no background due to nonspecific binding of an antibody conjugate. Furthermore, GFP fluorescence is stable and species-independent and does not require any substrates or cofactors.

## C7. Monitoring and controlling cell proliferation in culture

The ITICBP device is not only enable monitoring, studying and responding to stimuli, but further it allows the examination of gene action and cellular products such as cytokines, chemokines, NO and other cellular products. This is facilitated by the development of the biosensory capabilities of the device operating in conjunction with the diffractive measurements.

One extremely important feature of the system is the option of culturing cells bound to the bioreactive surfaces for extended periods of time, enabling gene expression and production of mediators, biological response modifiers, etc.

## C8. Expected Benefits of the ITICBP equipment and methodology

The overall components and corresponding methodology, all attached to the present invention are expected to penetrate the continuously expanding multi-disciplinary fields of cell-biology applications and electro-optics. The prospected developments include new designs, fabrication technologies, cell-manipulation techniques and cell- diagnostic tools. These new developments are mandatory for successful integration of micro-optics, electro-optics and cell-biology technologies. Such integration is highly valuable for high demanding applications such as cell evolution and manipulation in general and cell malignant transformation research, drug R&D and diagnosis and therapy, in particular.

Several specific capabilities of the proposed IDC and its practical implications are briefly described below:

## C9. Economic and High Throughput Screening Product

The TCC device-plate might has the standard outside dimensions of a microscope slide, maintaining few testing fields, each build up of 100x100 or 150x150 microwells. Nevertheless, the microwells are specially designed to have a total volume of about $5x10^{-12}$ liter ($5\mu\mu L$, compared to $50\mu L$ for 96 microwells available today on a plate) and optically flat, about 12 $\mu m$ diameter bottom. The reduced working volume allows for both a sharp reduction in reagents used in various fluorescent and color-metric assays and significant cost saving. The narrow diameter bottom also allows quick analysis of micro and single cell cultures since the entire amount of about 10 microwells bottom, can be visualized at x 100 magnification simultaneously, or separately by limiting the field of observation, without moving the ITICBP plate.

Both the ITICBP as well as its matching cover can be produced from clear or opaque polystyrene (black and white for fluorescence assays) and in a choice of at least four surface treatments:

- Non-treated and in standard packaging for general usage.
- Surface-treated to improve the binding of proteins to the ITICBP surface that is useful in ELISA assays.
- Surface-treatment for cell culture with each ITICBP individually wrapped and radiation sterilized.
- Surface-treated to increase wettability so solutions *spread* uniformly across the microwells.

## REFERENCES

Altman, JD Moss PAH, Goulder PJR, Barouch DH McHeyzer-Williams MG, Bell JI, McMichael AJ, and Davis MM (1996), Direct visualization and phenotypic analysis of virus specific T-lymphocytes in HIF-infected individuals. Science 274, 94-96.

Aubin J. (1979), Autofluorescence of viable cultured mammalian cells. J. Histochem Cytochem 27:36.

Barker SL and Kopelman R (1998), Development and cellular applications of fiber optic nitric oxide sensors based on a gold-adsorbed fluorophore. Anal Chem 70:100-104.

Bayachou M et al. (1998), Electrochemical reduction of NO by myoglobin in surfactant film, J. Am. Chem. Soc. 120: 9888.

Bedner E, Melamed MR and Darzynkiewicz Z (1998), Enzyme kinetic reactions and fluorochrome uptake rates measured in individual cells by laser scanning cytometry, Cytometry 33:1-9.

Benson HC, Meyer RA, Zaruba ME and McKhann GM (1979), Cellular autofluorescence - is it due to flavins? J Histochem Cytochem, 27:44-48.

Biran I, Levkov K, Hengge-Aronis R, Ron EZ and Rishpon J (1998), submitted to App. Environ. Microb., 1998.

Boddington MM, Diamond RA, Apparatus for automatic preparation and scanning in cervical cytology, Br Med J 3: 160-161, 1967.

Burger D, Gershman R, Acousto-optic laser scanning cytometer, Cytometry 9: 101-110, 1988.

Callan MF, Tan L, Annels N, Ogg GS, Wilson JD, O'Callaghan CA, Steven N, McMichael AJ, Rickinson AB (1998), Direct visualization of antigen-specific CD8+ T cells during primary immune response to EBV in vivo, J Exp Med 187:1395-1402.

Cercek and Cercek (1977), Application of the phenomenon of changes in the structuredness of cytoplasmic matrix (SCM) in the diagnosis of malignant disorders: A review, Eur J Cancer 13:903-915.

Cercek L, Cercek B and Ockey CH (1978), Fluoroscein excitation and emission polarization spectra in living cells: changes during the cell cycle, Biophys. 23:395-405.

Cercek L and Cercek B (1981), Changes in SCM-responses of lymphocytes in mice after implantation with Ehrlich ascites cells, Eur J Cancer 17:167-171.

Coulie P (1997), Human tumor antigens recognized by T cells, Mol Med Today 3:261-268.

Dawson IMP, Heanly CP, Heber-Percy AC, Tylko JK (1967), Cervical smear analyzer and reader, J Clin Pathol 20: 724-730.

de Grooth BG, Geerken TH, Greve J (1985), The cytodisk: A cytometer based upon a new principle of cell alignment, Cytometry 6:226-233.

Deutsch M and Weinreb A (1994), Apparatus for high precision repetitive sequential optical measurement of living cells, Cytometry 16:214-226.

Deutsch M and Weinreb A (1983), Validation of the SCM-test for the Diagnosis of Cancer, Eur J Cancer Clin Oncol 119(2): 187-193.

Deutsch M, Ron I, Weinreb A, Tirosh R and Chaitchik S (1996), Lymphocyte Fluorescence Polarization Measurements with the Cellscan System: Application to the SCM Cancer Test, Cytometry 23: 159-165.

Dive C, Workman P, Watson JV (1988), Inhibition of intracellular esterases by antitumour chloroethylnitrosoureas, Biochem Pharmacol 37:3987-3993.

Dive C, Workman P, Watson JV (1993), Can flow cytoenzymology be applied to measure membrane-bound enzyme kinetics, Biochem Pharmacol 46:643-650.

Dolbcare F (1990), Fluorescent staining of enzymes for flow cytometry, Methods Cell Biol 33:81-88.

Eisenthal A, Marder O, Lifschitz-Mercer B, Skornick Y, Tirosh R, Weinreb A and Deutsch M (1996), Inhibition of mitogen-induced changes in intracellular fluoroscein fluorescence polarization of human peripheral blood lymphocytes by cholchicine, vinblastine and cytochalasin B, Cell Struct Funct 21: 159-166.

Freed JJ, Engle JL (1962), Development of the vibrating-mirror flying spot microscope for ultraviolet spectrophotometry, Ann NY Acad Sci 97: 412-430.

Fuhr G, Arnold WM, Hagedorn R, Muller T, Benecke W, Wagner B, Zimmermann U (1992), Levitation, holding and rotation of cells within traps made by high-frequency fields, Biochim Biophys Acta 1108:215-223.

Green JE (1979), A practical application of computer pattern recognition research: The Abbott ADC-500 differential classifier, J Histochem Cytochem 27:160-173.

Hart L, Conovan RM, Goldstein E, Brady FP (1990), Detection of human immunodeficiency virus in infected CEM cells using a laser-based computerized image cytofluorometry system, Anal Quant Cytol Histol 12:127-134.

Israel DA, Barry WH, Edell DJ, Mark RG (1984), An array of microelectrodes to stimulate and record from cardiac cells in culture, Am J Physiol 247:H669-H674.

Kamentsky LA and Kamentsky LD (1991), Microscope-based multiparameter laser scanning cytometer yielding data comparable to flow cytometry 12:381-387.

Kamentsky LA, Burger DE, Gershman RJ, Kamentsky LD, Luther E (1997), Slide-based laser scanning cytometry, Acta Cytol 41:123-143.

Klenerman P, Phillips RE, Rinaldo CR, Wahl LM, Ogg G, May RM, McMichael AJ, Nowak MA (1996), Cytotoxic T lymphocytes and viral turnover in HIV type 1 infection, Proc Natl Acad Sci USA 93:15323-15328.

Klingel S, Rothe G, Kellerman W and Valet G (1994), Flow cytometric determination of serine proteinase activities in living cells with rhodamine 110 substrates, Meth Cell Biol 41:449-460.

Kooy NW, Royall JA, Ischiropoulos H, Beckman JS (1994), Peroxynitrite-mediated oxidation of dihydrorhodamine 123, Free Radic Biol Med 16:149-156.

Little JW and Mount DW (1982), The SOS regulatory systems of Escherichia coli, Cell 29:11-22.

Mahaworasilpa TL, Coster HG and George EP (1994), Forces on biological cells due to applied alternating (AC) electric fields, I. Dielectrophoresis, Biochim Biophys Acta 1193:118-126.

Malin-Berdel J and Valet G (1980), Flow cytometric determination of esterase and phosphatase activities and kinetics in hematopoietic cells with fluorogenic substrates, Cytometry 1:222-228.

Mansberg HP and Ohringer P (1969), Design consideration for electronic and electromechanical flying spot scanners, Ann NY Acad Sci 157:5-37.

Moruzzi JF, Wyrobek AJ, Mayall BM and Gledhill BL (1988), Quantification and classification of human sperm by computer assisted image analysis, Fertil Steril 50:142-152.

Moubarak AS and Muhoberac BB (1991), The interaction of thioridazine with cardiac cytochrome oxidase; enzyme activity and drug binding studies, Biochem Biophys Res Commun 179: 1063-1069.

Naftalin RJ, Smith PM and Roselaar SE (1985), Evidence for non-uniform distribution of D-glucose within human red cells during net exit and counterflow, Biochimica et Biophysica Acta 820: 35-249.

Nooter K, Herweijer H, Jonker RR and van den Engh GJ (1994), On-line flow cytometry. A versatile method for kinetic measurement, Methods Cell Biol 41:509-526.

Pasteur X, Maubon I, Cottier M, Azema J, Gonthier AM, Laurent JL (1988), Automated image analysis of in vitro decondensation of human spermatozoa nuclei: III Variable decondensation with and without incubation in seminal fluid, Anal Quant Cytol Histol 10:317-318.

Pohl MA (1978), Dielectrophoresis, Cambridge University Press, London.

Quantitative Fluorescence Cytometry: An Emerging Consensus, Cytometry, October 1998.

Rahmani H, Deutsch M, Ron I, Gerbat S, Tirosh R, Weinreb A, Chaitchik S and Lalchuk S (1996), Adaptation of the Cellscan technique for the SCM test in breast cancer, Eur J Cancer, 32A: 1758-1765.

Rajan DP, Huang W, Dutta B, Devoe LD, Leibach FH, Ganapathy V and Prasad PD (1999), Human placental sodium-dependent vitamin C transporter (SVCT2): molecular cloning and transport function, Biochem Biophys Res Commun 262:762-768.

Raveh O, Peleg N, Bettleheim A, Silbeman I and Rishpon J (1997), Bioelectrochem Bioenerg 43:19-25.

Rawal N and Pangburn MK (1998), C5 Convertase of the alternative pathway of complement; kinetic analysis of the free and surface-bound forms of the enzyme, J Biol Chem 273:16828-16835.

Read JS, Borovec RT, Bartels PH, Bibbo M, Puls JH, Reale FR, Taylor J, and Weid GL (1979), A fast processor for locating cell nuclei in uterine specimens, in Proceedings of the Second International Conference on Automation of Cancer Cytology and Cell Image Analysis, Pressman JP, Weid GL (eds). Tutorials of Cytology, International Academy of Cytology, Chicago IL, 1979, pp. 143-155.

Romero P, Dunbar PR, Valmori D, Pittet M, Ogg GS, Rimoldi D, Chen JL, Lienard D, Cerottini JC, Cerundolo V (1998), Ex-vivo staining of metastatic lymph nodes by cII MHC tetramers reveals high numbers of antigen-experienced tumor-specific CTL. J Exp Med 188:1641-1650.

Romero P et al. (1998), Novel methods to monitor antigen-specific CTL responses in cancer immunotherapy, Mol Med 7:305-312.

Rozdzial MM, Pleiman CM, Cambier JC and Finkel TH (1998), pp56Lck mediates TCR zeta-chain binding to the microfilament cytoskeleton, J Immunol 161:5491-5499.

Schiffer Z, Ashkenazi Y, Tirosh R, and Deutsch M (1999), Fourier analysis of light scattered by elongated scatterers, Applied Optics 38:3626-3635.

Schiffer Z, Keren-Tal I, Deutsch M, Dantes A, Aharoni D, Weinreb A, Tirosh R, Amsterdam A, Fourier analysis of differential light scattering for the quantitation of FSH response associated with structural changes in immortalized granulosa cells, Molecular Cellular Endocrin 118, 145-153 (1996).

Schnelle Th, Hagedorn R, Fuhr G, Fiedler S, Muller T (1993), Three-dimensional electric field traps for manipulation of cells - calculation and experimental verification, Biochem Biophys Acta 1157:127-140.

Segel GB, Simon W, Lichtman AH, Lichtman MA (1981), The activation of lymphocyte plasma membrane (Na, K)-ATPase by EGTA is explained better by zinc than calcium chelation, J Biol Chem 256:6629-6632.

Shack R, Baker R, Buchroeder R, Hilman D, Shoemaker R and Bartels PH (1979), Ultrafast laser scanner microscope, J Histochem Cytochem 27:153-159.

Shapiro HM (1983), Apparatus and method for killing unwanted cells, US Patent No. 4,395,397.

Shapiro HM (1995), Practical Flow Cytometry, Alan R. Liss, Inc., New York, 1995.

Shapiro HM (1985), Post-cardiac arrest therapy: calcium entry blockade and brain resuscitation, Anesthesiology 62:384-387.

Sloot PMA, Hoekstra AG and Fidgor CG (1988), Osmotic response of lymphocytes measured by means of forward light scattering: theoretical considerations, Cytometry 9:636-641.

Soini JT, Chernyshev AV, Hanninwn PE, Soini E and Maltsev VP (1998), A new design of the flow cuvette and optical set-up for the scanning flow cytometer, Cytometry 31:78-81.

Steven FS, Desai M, Sin J, Palcic B (1996), Fluorescent location of cells of cytological interest in cervical smears prestained with thionin, Anticancer Res 16(3A):1193-1196.

Sunray M, Deutsch M, Kaufman M, Tirosh R, Weinrab A and Rachmani H (1997), Cell activation influences cell staining kinetics, Spectrochimica Acta Part A53:1645-1653.

Sunray M, Kaufman M, Zurgil N and Deutsch M (1999), The trace and subgrouping of lymphocyte activation by dynamic fluorescence intensity and polarization measurements, Biochem Biophys Res Comm 261:712-719.

Tanaka N, Ikeda H, Ueno T, Mukawa A and Kamitsuma K (1979), Field test and experimental use of CYBEST Model 2 for practical gynecologic mass screening, Anal Quant Cytol J 1:122-126.

Tomei LD, Cornhill F, Jagadeesh J and Boninger M (1988), Method and apparatus for the measurement of low-level laser-induced fluorescence, US Patent 4,748,727.

Turck JJ and Robinson JP (1994), Leucine aminopeptidase activity by flow cytometry, Methods Cell Biol 41:461-468.

Wang XB, Huang Y, Burt JPH, Markx GM and Pethig R(1993), J Phys D Appl Phys 26:1278-1285.

Watson JV and Dive C (1994), Enzyme kinetics, Methods Cell Biol 41:469-508.

Watson JV, Dive C and Workman P (1994), A practical approach, in Flow Cytometry (Ormerod MG, ed.) pp. 141-162, IRL Press/Oxford University Press, Oxford, UK.

Weetall HH (1993), A method for the assay of hydrolytic enzymes using dynamic light scattering, Appl Biochem Biotechno 41:139-144.

Weetall HH and Vann WP (1976), Studies on immobilized trypsin in high concentrations of organic solvents. Biotechnol bioeng 18:105-118.

Wilson GS and Yibai HU (1999), In vivo measurements using implantable microsensors, 7th Intl Seminar on electroanalytical Chemistry, p. 20, Oct. 22-24, 1999, Changchuan, China.

Woodgate R and Sedgwick SG (1992), Mutagenesis induced by bacterial proteins and their plasmid homologues, Mol Microbiol 6:2213-2218.

Zurgil N, Schiffer Z, Shafran Y, Kaufman M and Deutsch M (2000), Fluorescein fluorescence hyperpolarization as an early kinetic measure of the apoptotic process, Biochem Biophys Res Comm 268:155-163.

**Claims**

1. A device for holding a plurality of living cells, comprising:

   a transparent element defining a multiplicity of optically transparent wells, wherein the size of said wells is configured to hold a single cell, any defined number of cells, or other defined particles; and

   **characterized by**

   a plurality of said wells including an integral biosensor component.

2. A device according to claim 1, wherein said biosensor component comprises at least one sensing electrode.

**3.** A device according to claim 1, wherein said biosensor component comprises at least one optical element.

**4.** A device according to claim 1, wherein said biosensor component comprises a bioactive material adhered to or impregnating said wells.

**5.** A device according to claim 4, wherein said bioactive material is associated with a bottom of said wells.

**6.** A device according to claim 4, wherein said bioactive material is associated with a sidewall of said wells.

**7.** A device according to any of claims 4-6, wherein said bioactive material is selected from the group consisting of enzymes, antibodies, immunogens, antigens, ligands, receptors, nucleic acids, proteins, peptides and receptors.

**8.** A device according to any of the preceding claims, wherein said wells are configured to contain said cells and contents thereof also after death thereof.

**9.** A device according to any of the preceding claims, wherein a multiplicity of said wells define fluid flow pores in one or both of their bottom and their sides.

**10.** A device according to any of the preceding claims, wherein said multiplicity of wells are positioned with a pitch of about the well diameter.

**11.** A device according to any of the preceding claims, wherein a multiplicity of said wells are pitched at 150 microns or less and arranged as an array of size at least 3x3 mm.

**12.** A device according to any of the preceding claims, wherein each of said multiplicity of wells includes at least one manipulator element.

**13.** A method of cell study, comprising:

  arranging a plurality of elements comprising cells, particles or cell conglomerates each into one of a plurality of adjacent transparent wells, sized for said arranged elements;
  studying said elements as individual elements in said wells using in-well sensing components.

**14.** A method according to claim 13, wherein arranging comprises allowing said elements to drop by gravity towards said wells such that each well can contain only one element and a wall between said wells is insufficient to support an element.

**15.** A method according to claim 13 or claim 14, comprising also studying said elements in said wells using destructive testing protocols, said wells maintaining a contents of said elements in said wells.

**16.** A method according to any of claims 13-15, wherein said in-well sensing components are separately provided to said wells after said arranging.

Fig 1

Fig 2

Fig 3

Fig 4

Fig 5

Fig 6

Fig 7

Fig 8

Fig 9

Fig. 10

Fig. 11

Fig 12

Fig 13

Fig 14

Fig 15

Fig 16

Fig 17

Fig 18

Fig. 19

Cell Nucleus

Cell Membrane

2

1

3

Fig. 20

Fig. 21

Fig. 22

Fig 23

Fig 24

Fig 25

Fig 26

Fig 27

Fig 28

Fig 29

Fig 30

Fig 31

Fig 32

Fig 33

Fig 34

Fig. 35

Fig 36

Fig.37

Fig.38

Fig 39a

Fig 39b

Fig 39c

Fig 39d

Fig 39e

Fig 39f

Fig.39

Fig.40

Fig.41

Fig.42

Fig.43

## Coin With Built -In Spacers

View From Above

60

60 a

Rinsing

3

1

a — — — — — — — — — — a

2

Cover Slip

Cross Section View (aa)

Rinsing

60

3   2   1

# Fig 43a

Fig.44

Fig.45

Fig.46

Fig.47

Fig.48

Fig.48a

Fig 48b

Fig 48c

A single
perforation

## Fig 48d

A single
perforation

## Fig 48e

Before Sonification

Fig 48f

After Sonification

Fig 48g

Optical
Transparency

Porous Region (1)

NonPorous Region (2)

## Fig 48h

Porous Region

NonPorous Region

## Fig 48i

Fig.49

Fig 50

Fig 51

Fig 52

Fig 53

Fig 54

Fig 55

Fig 56

Left To Right Stream

Fig 57

Fig 58

Fluorescence
Detection and IA

Dichroic
Mirror

Light
Source

Excitation
Objective

34

30

LWD
Observation
Objective

Image Analysis
Of Transparent
Excitation Light

Fig 59

Fig 60

4 directional optical fiber illumination

Fig 61

Fig.62

Fig.63

Cell per well per bundle - LCD selective illumination emission optical arrangement.

Fig.64

Integrated Microlense Array (Sandwich)

Fluorescence Detection and IA

Light

Optical Filter (F1)

Positive Microlense Array

Solution

Optical Filter (F2)

Cell

Negative Microlense Array

IA with exciting light

Fig.65

Integrated ITICBP Sandwich

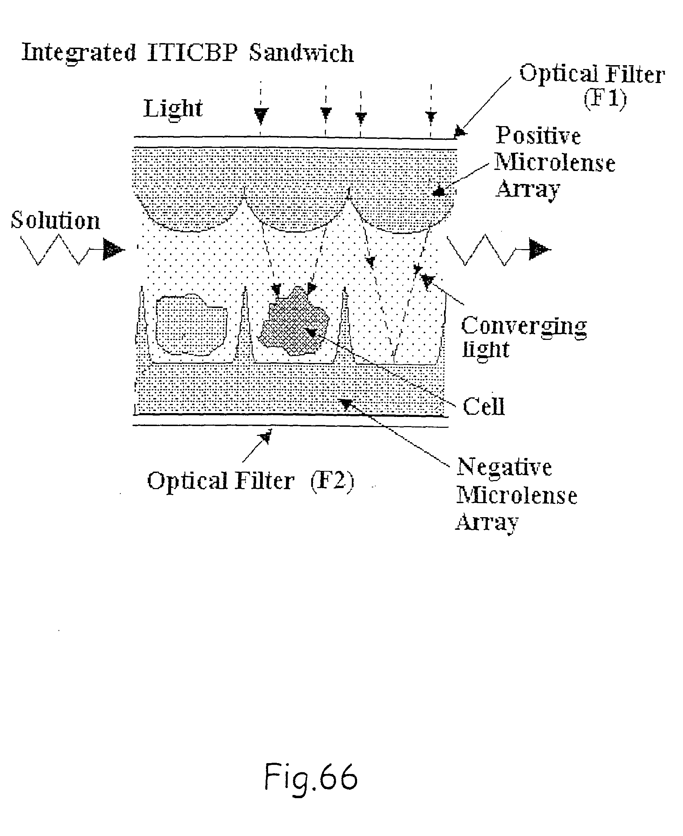

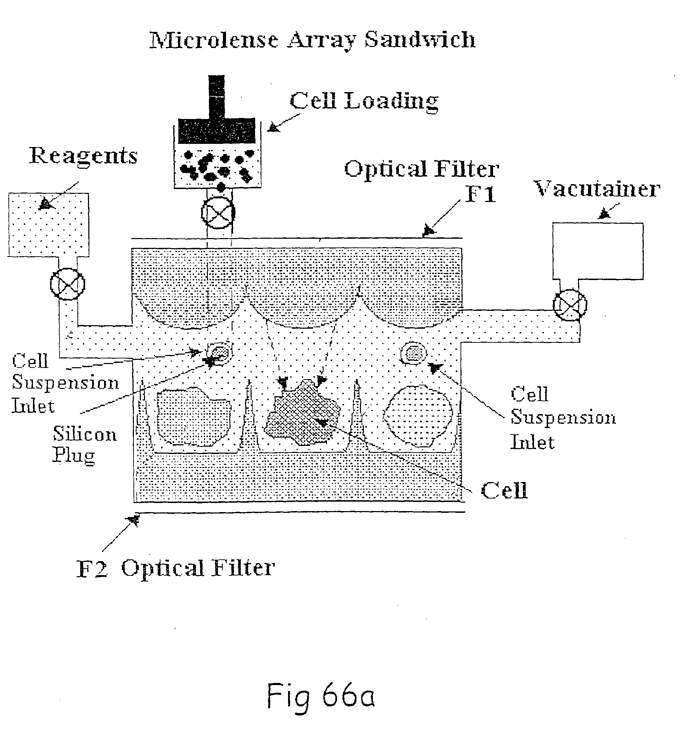

Fig.66

Fig 66a

## A Cell Per Single Well Per Single Detector Pixels Array

Fig 67

System Elementary Block Diagram

Fig 68

Fig 69

Fig 69a

**Theory**
**a. Continues single step cell staining**

$\alpha$

$[S]_0$  $[S]_i$  $E + S \underset{k_{-1}}{\overset{k_1}{\rightleftharpoons}} ES$  $[P]_i \rightarrow [P]_0$  $\beta$

$ES \overset{k_2}{\longrightarrow} P + E$

- [S] = fluorescein-di acetat (FDA)
  [E] = esterase
  [P] = fluorescein

The production rate of P:

$$\frac{d[P]_I}{dt} = \alpha \cdot [S]_0 - \beta \cdot [P]_I$$

Solving this equation when (P(t=0) = 0):

$\alpha$ - rate constant for formation of P

$\beta$ - rate constant for leakage of P

Fig. 70

Fig. 71

Fig. 72A

EP 2 332 651 A2

Fig. 72B

Fig. 72C

Fig. 72D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4729949 A
- US 5272081 A
- US 5310674 A
- US 5506141 A
- US 5506 A
- US 4395397 A
- US 4748727 A

**Non-patent literature cited in the description**

- **DOLBCARE F.** Fluorescent staining of enzymes for flow cytometry. *Methods Cell Biol,* 1990, vol. 33, 81-88
- **KLINGEL S ; ROTHE G ; KELLERMAN W ; VALET G.** Flow cytometric determination of serine proteinase activities in living cells with rhodamine 110 substrates. *Methods Cell Biol,* 1994, vol. 41, 449-460
- **MALIN-BERDEL J ; VALET G.** Flow cytometric determination of esterase and phosphatase activities and kinetics in hematopoietic cells with fluorogenic substrates. *Cytometry,* 1980, vol. 1, 222-228
- **NOOTER K ; HERWEIJER H ; JONKER RR ; VAN DEN ENGH GJ.** On-line flow cytometry. A versatile method for kinetic measurement. *Methods Cell Biol,* 1994, vol. 41, 509-526
- **TURCK JJ ; ROBINSON JP.** Leucine aminopeptidase activity by flow cytometry. *Methods Cell Biol,* 1994, vol. 41, 461-468
- **WATSON JV ; DIVE C.** Enzyme kinetics. *Methods Cell Biol,* 1994, vol. 41, 469-508
- **WATSON JV ; DIVE C.** Enzyme kinetics. *Methods Cell Biol,* 1994, vol. 41, 469-508
- **BEDNER E ; MELAMED MR ; DARZYNKIEWICZ Z.** Enzyme kinetic reactions and fluorochrome uptake rates measured in individual cells by laser scanning cytometry. *Cytometry,* 1998, vol. 33, 1-9
- **SUNRAY M ; DEUTSCH M ; KAUFMAN M ; TIROSH R ; WEINREB A ; RACHMANI H.** Cell Activation influences cell staining kinetics. *Spectrochimica Acta A,* 1997, vol. 53, 1645-1653
- **DEUTSCH M ; WEINREB A.** Apparatus for High Precision Repetitive Sequential Optical Measurement of Living Cells. *Cytometry,* 1994, vol. 16, 214-226
- **ALTMAN, JD ; MOSS PAH ; GOULDER PJR ; BAROUCH DH ; MCHEYZER-WILLIAMS MG ; BELL JI ; MCMICHAEL AJ ; DAVIS MM.** Direct visualization and phenotypic analysis of virus specific T-lymphocytes in HIF-infected individuals. *Science,* 1996, vol. 274, 94-96
- **AUBIN J.** Autofluorescence of viable cultured mammalian cells. *J. Histochem Cytochem,* 1979, vol. 27, 36
- **BARKER SL ; KOPELMAN R.** Development and cellular applications of fiber optic nitric oxide sensors based on a gold-adsorbed fluorophore. *Anal Chem,* 1998, vol. 70, 100-104
- **BAYACHOU M et al.** Electrochemical reduction of NO by myoglobin in surfactant film. *J. Am. Chem. Soc.,* 1998, vol. 120, 9888
- **BENSON HC ; MEYER RA ; ZARUBA ME ; MCK-HANN GM.** Cellular autofluorescence - is it due to flavins?. *J Histochem Cytochem,* 1979, vol. 27, 44-48
- **BIRAN I ; LEVKOV K ; HENGGE-ARONIS R ; RON EZ ; RISHPON J.** *App. Environ. Microb.,* 1998
- **BODDINGTON MM ; DIAMOND RA.** Apparatus for automatic preparation and scanning in cervical cytology. *Br Med J,* 1967, vol. 3, 160-161
- **BURGER D ; GERSHMAN R.** Acousto-optic laser scanning cytometer. *Cytometry,* 1988, vol. 9, 101-110
- **CALLAN MF ; TAN L ; ANNELS N ; OGG GS ; WILSON JD ; O'CALLAGHAN CA ; STEVEN N ; MCMICHAEL AJ ; RICKINSON AB.** Direct visualization of antigen-specific CD8+ T cells during primary immune response to EBV in vivo. *J Exp Med,* 1998, vol. 187, 1395-1402
- **CERCEK ; CERCEK.** Application of the phenomenon of changes in the structuredness of cytoplasmic matrix (SCM) in the diagnosis of malignant disorders: A review. *Eur J Cancer,* 1977, vol. 13, 903-915

- **CERCEK L ; CERCEK B ; OCKEY CH.** Fluoroscein excitation and emission polarization spectra in living cells: changes during the cell cycle. *Biophys.,* 1978, vol. 23, 395-405
- **CERCEK L ; CERCEK B.** Changes in SCM-responses of lymphocytes in mice after implantation with Ehrlich ascites cells. *Eur J Cancer,* 1981, vol. 17, 167-171
- **COULIE P.** Human tumor antigens recognized by T cells. *Mol Med Today,* 1997, vol. 3, 261-268
- **DAWSON IMP ; HEANLY CP ; HEBER-PERCY AC ; TYLKO JK.** Cervical smear analyzer and reader. *J Clin Pathol,* 1967, vol. 20, 724-730
- **DE GROOTH BG ; GEERKEN TH ; GREVE J.** The cytodisk: A cytometer based upon a new principle of cell alignment. *Cytometry,* 1985, vol. 6, 226-233
- **DEUTSCH M ; WEINREB A.** Apparatus for high precision repetitive sequential optical measurement of living cells. *Cytometry,* 1994, vol. 16, 214-226
- **DEUTSCH M ; WEINREB A.** Validation of the SCM-test for the Diagnosis of Cancer. *Eur J Cancer Clin Oncol,* 1983, vol. 119 (2), 187-193
- **DEUTSCH M ; RON I ; WEINREB A ; TIROSH R ; CHAITCHIK S.** Lymphocyte Fluorescence Polarization Measurements with the Cellscan System: Application to the SCM Cancer Test. *Cytometry,* 1996, vol. 23, 159-165
- **DIVE C ; WORKMAN P ; WATSON JV.** Inhibition of intracellular esterases by antitumour chloroethylnitrosoureas. *Biochem Pharmacol,* 1988, vol. 37, 3987-3993
- **DIVE C ; WORKMAN P ; WATSON JV.** Can flow cytoenzymology be applied to measure membrane-bound enzyme kinetics. *Biochem Pharmacol,* 1993, vol. 46, 643-650
- **EISENTHAL A ; MARDER O ; LIFSCHITZ-MERCER B ; SKORNICK Y ; TIROSH R ; WEINREB A ; DEUTSCH M.** Inhibition of mitogen-induced changes in intracellular fluoroscein fluorescence polarization of human peripheral blood lymphocytes by cholchicine, vinblastine and cytochalasin B. *Cell Struct Funct,* 1996, vol. 21, 159-166
- **FREED JJ ; ENGLE JL.** Development of the vibrating-mirror flying spot microscope for ultraviolet spectrophotometry. *Ann NY Acad Sci,* 1962, vol. 97, 412-430
- **FUHR G ; ARNOLD WM ; HAGEDORN R ; MULLER T ; BENECKE W ; WAGNER B ; ZIMMERMANN U.** Levitation, holding and rotation of cells within traps made by high-frequency fields. *Biochim Biophys Acta,* 1992, vol. 1108, 215-223
- **GREEN JE.** A practical application of computer pattern recognition research: The Abbott ADC-500 differential classifier. *J Histochem Cytochem,* 1979, vol. 27, 160-173
- **HART L ; CONOVAN RM ; GOLDSTEIN E ; BRADY FP.** Detection of human immunodeficiency virus in infected CEM cells using a laser-based computerized image cytofluorometry system. *Anal Quant Cytol Histol,* 1990, vol. 12, 127-134
- **ISRAEL DA ; BARRY WH ; EDELL DJ ; MARK RG.** An array of microelectrodes to stimulate and record from cardiac cells in culture. *Am J Physiol,* 1984, vol. 247, H669-H674
- **KAMENTSKY LA ; KAMENTSKY LD.** *Microscope-based multiparameter laser scanning cytometer yielding data comparable to flow cytometry,* 1991, vol. 12, 381-387
- **KAMENTSKY LA ; BURGER DE ; GERSHMAN RJ ; KAMENTSKY LD ; LUTHER E.** Slide-based laser scanning cytometry. *Acta Cytol,* 1997, vol. 41, 123-143
- **KLENERMAN P ; PHILLIPS RE ; RINALDO CR ; WAHL LM ; OGG G ; MAY RM ; MCMICHAEL AJ ; NOWAK MA.** Cytotoxic T lymphocytes and viral turnover in HIV type 1 infection. *Proc Natl Acad Sci USA,* 1996, vol. 93, 15323-15328
- **KLINGEL S ; ROTHE G ; KELLERMAN W ; VALET G.** Flow cytometric determination of serine proteinase activities in living cells with rhodamine 110 substrates. *Meth Cell Biol,* 1994, vol. 41, 449-460
- **KOOY NW ; ROYALL JA ; ISCHIROPOULOS H ; BECKMAN JS.** Peroxynitrite-mediated oxidation of dihydrorhodamine 123. *Free Radic Biol Med,* 1994, vol. 16, 149-156
- **LITTLE JW ; MOUNT DW.** The SOS regulatory systems of Escherichia coli. *Cell,* 1982, vol. 29, 11-22
- **MAHAWORASILPA TL ; COSTER HG ; GEORGE EP.** Forces on biological cells due to applied alternating (AC) electric fields, I. Dielectrophoresis. *Biochim Biophys Acta,* 1994, vol. 1193, 118-126
- **MANSBERG HP ; OHRINGER P.** Design consideration for electronic and electromechanical flying spot scanners. *Ann NY Acad Sci,* 1969, vol. 157, 5-37
- **MORUZZI JF ; WYROBEK AJ ; MAYALL BM ; GLEDHILL BL.** Quantification and classification of human sperm by computer assisted image analysis. *Fertil Steril,* 1988, vol. 50, 142-152
- **MOUBARAK AS ; MUHOBERAC BB.** The interaction of thioridazine with cardiac cytochrome oxidase; enzyme activity and drug binding studies. *Biochem Biophys Res Commun,* 1991, vol. 179, 1063-1069
- **NAFTALIN RJ ; SMITH PM ; ROSELAAR SE.** Evidence for non-uniform distribution of D-glucose within human red cells during net exit and counterflow. *Biochimica et Biophysica Acta,* 1985, vol. 820, 35-249

- **PASTEUR X ; MAUBON I ; COTTIER M ; AZEMA J ; GONTHIER AM ; LAURENT JL.** Automated image analysis of in vitro decondensation of human spermatozoa nuclei: III Variable decondensation with and without incubation in seminal fluid. *Anal Quant Cytol Histol,* 1988, vol. 10, 317-318
- **POHL MA.** Dielectrophoresis. Cambridge University Press, 1978
- Quantitative Fluorescence Cytometry: An Emerging Consensus. *Cytometry,* October 1998
- **RAHMANI H ; DEUTSCH M ; RON I ; GERBAT S ; TIROSH R ; WEINREB A ; CHAITCHIK S ; LALCHUK S.** Adaptation of the Cellscan technique for the SCM test in breast cancer. *Eur J Cancer,* 1996, vol. 32A, 1758-1765
- **RAJAN DP ; HUANG W ; DUTTA B ; DEVOE LD ; LEIBACH FH ; GANAPATHY V ; PRASAD PD.** Human placental sodium-dependent vitamin C transporter (SVCT2): molecular cloning and transport function. *Biochem Biophys Res Commun,* 1999, vol. 262, 762-768
- **RAVEH O ; PELEG N ; BETTLEHEIM A ; SILBEMAN I ; RISHPON J.** *Bioelectrochem Bioenerg,* 1997, vol. 43, 19-25
- **RAWAL N ; PANGBURN MK.** C5 Convertase of the alternative pathway of complement; kinetic analysis of the free and surface-bound forms of the enzyme. *J Biol Chem,* 1998, vol. 273, 16828-16835
- A fast processor for locating cell nuclei in uterine specimens. **READ JS ; BOROVEC RT ; BARTELS PH ; BIBBO M ; PULS JH ; REALE FR ; TAYLOR J ; WEID GL.** Proceedings of the Second International Conference on Automation of Cancer Cytology and Cell Image Analysis. International Academy of Cytology, 1979, 143-155
- **ROMERO P ; DUNBAR PR ; VALMORI D ; PITTET M ; OGG GS ; RIMOLDI D ; CHEN JL ; LIENARD D ; CEROTTINI JC ; CERUNDOLO V.** Ex-vivo staining of metastatic lymph nodes by cII MHC tetramers reveals high numbers of antigen-experienced tumor-specific CTL. *J Exp Med,* 1998, vol. 188, 1641-1650
- **ROMERO P et al.** Novel methods to monitor antigen-specific CTL responses in cancer immunotherapy. *Mol Med,* 1998, vol. 7, 305-312
- **ROZDZIAL MM ; PLEIMAN CM ; CAMBIER JC ; FINKEL TH.** pp56Lck mediates TCR zeta-chain binding to the microfilament cytoskeleton. *J Immunol,* 1998, vol. 161, 5491-5499
- **SCHIFFER Z ; ASHKENAZI Y ; TIROSH R ; DEUTSCH M.** Fourier analysis of light scattered by elongated scatterers. *Applied Optics,* 1999, vol. 38, 3626-3635
- **SCHIFFER Z ; KEREN-TAL I ; DEUTSCH M ; DANTES A ; AHARONI D ; WEINREB A ; TIROSH R ; AMSTERDAM A.** Fourier analysis of differential light scattering for the quantitation of FSH response associated with structural changes in immortalized granulosa cells. *Molecular Cellular Endocrin,* 1996, vol. 118, 145-153
- **SCHNELLE TH ; HAGEDORN R ; FUHR G ; FIEDLER S ; MULLER T.** Three-dimensional electric field traps for manipulation of cells - calculation and experimental verification. *Biochem Biophys Acta,* 1993, vol. 1157, 127-140
- **SEGEL GB ; SIMON W ; LICHTMAN AH ; LICHTMAN MA.** The activation of lymphocyte plasma membrane (Na,K)-ATPase by EGTA is explained better by zinc than calcium chelation. *J Biol Chem,* 1981, vol. 256, 6629-6632
- **SHACK R ; BAKER R ; BUCHROEDER R ; HILMAN D ; SHOEMAKER R ; BARTELS PH.** Ultrafast laser scanner microscope. *J Histochem Cytochem,* 1979, vol. 27, 153-159
- **SHAPIRO HM.** Practical Flow Cytometry. Alan R. Liss, Inc, 1995
- **SHAPIRO HM.** Post-cardiac arrest therapy: calcium entry blockade and brain resuscitation. *Anesthesiology,* 1985, vol. 62, 384-387
- **SLOOT PMA ; HOEKSTRA AG ; FIDGOR CG.** Osmotic response of lymphocytes measured by means of forward light scattering: theoretical considerations. *Cytometry,* 1988, vol. 9, 636-641
- **SOINI JT ; CHERNYSHEV AV ; HANNINWN PE ; SOINI E ; MALTSEV VP.** A new design of the flow cuvette and optical set-up for the scanning flow cytometer. *Cytometry,* 1998, vol. 31, 78-81
- **STEVEN FS ; DESAI M ; SIN J ; PALCIC B.** Fluorescent location of cells of cytological interest in cervical smears prestained with thionin. *Anticancer Res,* 1996, vol. 16 (3A), 1193-1196
- **SUNRAY M ; DEUTSCH M ; KAUFMAN M ; TIROSH R ; WEINRAB A ; RACHMANI H.** Cell activation influences cell staining kinetics. *Spectrochimica Acta Part,* 1997, vol. A53, 1645-1653
- **SUNRAY M ; KAUFMAN M ; ZURGIL N ; DEUTSCH M.** The trace and subgrouping of lymphocyte activation by dynamic fluorescence intensity and polarization measurements. *Biochem Biophys Res Comm,* 1999, vol. 261, 712-719
- **TANAKA N ; IKEDA H ; UENO T ; MUKAWA A ; KAMITSUMA K.** Field test and experimental use of CYBEST Model 2 for practical gynecologic mass screening. *Anal Quant Cytol J,* 1979, vol. 1, 122-126
- **WANG XB ; HUANG Y ; BURT JPH ; MARKX GM ; PETHIG R.** *J Phys D Appl Phys,* 1993, vol. 26, 1278-1285
- **WATSON JV ; DIVE C ; WORKMAN P.** A practical approach, in Flow Cytometry. IRL Press/Oxford University Press, 1994, 141-162

- **WEETALL HH.** A method for the assay of hydrolytic enzymes using dynamic light scattering. *Appl Biochem Biotechno,* 1993, vol. 41, 139-144
- **WEETALL HH ; VANN WP.** Studies on immobilized trypsin in high concentrations of organic solvents. *Biotechnol bioeng,* 1976, vol. 18, 105-118
- **WILSON GS ; YIBAI HU.** In vivo measurements using implantable microsensors. *7th Intl Seminar on electroanalytical Chemistry,* 22 October 1999, 20
- **WOODGATE R ; SEDGWICK SG.** Mutagenesis induced by bacterial proteins and their plasmid homologues. *Mol Microbiol,* 1992, vol. 6, 2213-2218
- **ZURGIL N ; SCHIFFER Z ; SHAFRAN Y ; KAUFMAN M ; DEUTSCH M.** Fluorescein fluorescence hyperpolarization as an early kinetic measure of the apoptotic process. *Biochem Biophys Res Comm,* 2000, vol. 268, 155-163